# EUROPEAN PATENT APPLICATION

(11) **EP 3 640 234 A1**
(43) Date of publication of application: **22.04.2020**
(21) Application number: 18200674.2
(22) Date of filing: 16.10.2018
(51) Int. Cl.: C07C 43/184, C07C 43/188, C07C 69/013, C07C 69/14, C07C 69/145, C07C 69/24, A61K 8/34, C11B 9/00

(54) **ETHERS AND ESTERS OF 1-SUBSTITUTED CYCLOALKANOLS FOR USE AS AROMA CHEMICALS**

(71) Applicant: BASF SE, 67056 Ludwigshafen am Rhein (DE)
(72) Inventor: STOCK, Christoph, 67056 Ludwigshafen am Rhein (DE); BRU ROIG, Miriam, 67056 Ludwigshafen am Rhein (DE); PELZER, Ralf, 68623 Lampertheim (DE); GARLICHS, Florian, 68623 Lampertheim (DE); DANZ, Manuel, 67056 Ludwigshafen am Rhein (DE)
(74) Representative: Reitstötter Kinzebach

(57) **Abstract**

The present invention relates to the use of an ether or an ester of a 1-substituted cycloalkanol or of mixtures of two or more ethers or esters of 1-substituted cycloalkanols or of a stereoisomer thereof or of a mixture of two or more stereoisomers thereof as aroma chemicals; to the use thereof for modifying the scent character of a fragranced composition; to an aroma chemical composition containing an ether or an ester of a 1-substituted cycloalkanol or of mixtures of two or more ethers or esters of 1-substituted cycloalkanols or of a stereoisomer thereof or of a mixture of two or more stereoisomers thereof; and to a method of preparing a fragranced composition or for modifying the scent character of a fragranced composition.

The invention further relates to specific ethers or esters of 1-substituted cycloalkanols.

## Description

The present invention relates to the use of an ether or an ester of a 1-substituted cycloalkanol or of mixtures of two or more ethers or esters of 1-substituted cycloalkanols or of a stereoisomer thereof or of a mixture of two or more stereoisomers thereof as aroma chemicals; to the use thereof for modifying the scent character of a fragranced composition; to an aroma chemical composition containing an ether or an ester of a 1-substituted cycloalkanol or of mixtures of two or more ethers or esters of 1-substituted cycloalkanols or of a stereoisomer thereof or of a mixture of two or more stereoisomers thereof; and to a method of preparing a fragranced composition or for modifying the scent character of a fragranced composition.

The invention further relates to specific ethers or esters of 1-substituted cycloalkanols.

### TECHNICAL BACKGROUND

Aroma chemicals, especially fragrances, are of great interest especially in the field of cosmetics and cleaning and laundry compositions. Fragrances of natural origin are mostly expensive, often limited in their available amount and, on account of fluctuations in environmental conditions, are also subject to variations in their content, purity etc. To circumvent these undesirable factors, it is therefore of great interest to create synthetic substances which have organoleptic properties that resemble more expensive natural fragrances or which have novel and interesting organoleptic profiles.

Despite a large number of already existing synthetic aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should also have additional positive secondary properties, such as e.g. an efficient preparation method, the possibility of providing better sensory profiles as a result of synergistic effects with other fragrances, a higher stability under certain application conditions, a higher extendability, a better higher substantivity, etc.

However, since even small changes in chemical structure bring about massive changes in the sensory properties such as odor and also taste, the targeted search for substances with certain sensory properties such as a certain odor is extremely difficult. The search for new fragrances and flavorings is therefore in most cases difficult and laborious without knowing whether a substance with the desired odor and/or taste will even actually be found.

GB 1344653 describes 1,2-substituted cycloalkanols and esters of 1,2-substituted cycloalkanols as well as perfume or scented compositions containing these compounds.

DE 2152016 describes 1-substituted cyclododecanols and methyl- and ethylethers of 1-substituted cyclododecanols as well as scent compositions comprising these compounds.

US 4,327,749 describes several 1-acyl-substituted cycloalkyl alkanoates as well as scent compositions comprising said compounds. Further described are 1-alkinylsubstituted cycloalkyl alkanoates, which are used as precursors for the production of the 1-acyl-substituted cycloalkyl alkanoates.

Furthermore, 1-ethynylcyclohexyl acetate (CAS registry number 5240-32-4) is known as an olfactory substance.

It was the object of the present invention to provide new aroma chemicals. These should have pleasant organoleptic properties. It was a further object of the present invention to provide substances which can be used as an aroma chemical in ready-to-use compositions. In particular, odor-intensive substances having a pleasant odor are sought. Furthermore, they should be combinable with other aroma chemicals, allowing the creation of novel advantageous sensory profiles. In addition, these aroma chemicals should be obtainable from readily available starting materials, allowing their fast and economic manufacturing, and should be free of toxicological concerns.

This object is achieved by the compound of formula (I) as shown below or mixtures thereof or stereoisomers thereof.

### SUMMARY OF THE INVENTION

The invention relates to the use of a compound of the general formula (I) 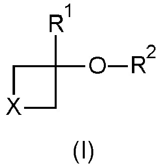 wherein
- X: is linear C₁-C₄-alkylene, which is unsubstituted or substituted by 1, 2, 3 or 4 radicals independently of each other selected from C₁-C₄-alkyl,
- R¹: is ethyl or ethenyl, and
- R²: is C₁-C₄-alkyl or C₁-C₅-alkanoyl,
or of a mixture of two or more compounds of the general formula (I),
or of a stereoisomer thereof or of a mixture of two or more stereoisomers thereof,
as an aroma chemical.

Another aspect of the invention is the use of a compound of formula (I) or a mixture of two or more compounds of formula (I) or a stereoisomer thereof or a mixture of two or more stereoisomers thereof for modifying the scent character of a fragranced composition, e.g. of a fragranced ready-to-use composition.

Yet another aspect of the invention is a composition comprising a compound of formula (I) or a mixture of two or more compounds of the general formula (I) or a stereoisomer thereof or a mixture of two or more stereoisomers thereof, and at least one further compound selected from the group consisting of further aroma chemicals different from compounds (I) and non-aroma chemical carriers, where the non-aroma chemical carrier is in particular selected from the group consisting of surfactants, oil components and solvents.

The invention also relates to a method for preparing a fragranced composition, e.g. a fragranced ready-to-use composition, or for modifying the scent character of a fragranced composition, e.g. of a fragranced ready-to-use composition, comprising incorporating a compound of formula (I) or a mixture of two or more compounds of the general formula (I) or a stereoisomer thereof or a mixture of two or more stereoisomers thereof into said composition.

Further, the invention relates to a compound of the general formula (I.a) or a stereoisomer thereof 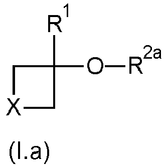 wherein
- X: is 1,2-ethanediyl or 1,3-propanediyl,
- R¹: is ethyl or ethenyl, and
- R^{2a}: is C₂-C₄-alkyl, in particular ethyl,
or a compound of the general formula (I.b) or a stereoisomer thereof 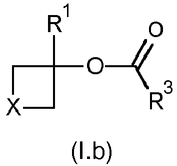 wherein
- X: is 1,2-ethanediyl or 1,3-propanediyl,
- R¹: is ethyl or ethenyl, and
- R³: is C₂-C₄-alkyl, in particular ethyl.

The invention also relates to a method for preparing the compounds of formula (I.a) or a mixture of two or more compounds of formula (I.a) or a stereoisomer thereof or a mixture of two or more stereoisomers thereof.

The invention also relates to a method for preparing the compounds of formula (I.b) or a mixture of two or more compounds of formula (I.b) or a stereoisomer thereof or a mixture of two or more stereoisomers thereof.

The compounds of formula (I) as well as mixtures of two or more compounds of formula (I), their stereoisomers and the mixtures of their stereoisomers possess advantageous organoleptic properties, in particular a pleasant odor. Therefore, they can be favorably used as an aroma chemical for example in perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions, crop protection compositions and other ready-to-use compositions.

By virtue of their physical properties, the compounds of formula (I) as well as mixtures of two or more compounds of formula (I), their stereoisomers and the mixtures of their stereoisomers have particularly good, virtually universal solvent properties for other fragrances and other customary ingredients in fragranced ready-to-use compositions such as, in particular, perfume compositions. Therefore, the compounds of formula (I) as well as mixtures of two or more compounds of formula (I), their stereoisomers and the mixtures of their stereoisomers are favorably combinable with other aroma chemicals, allowing, in particular, the creation of perfume compositions having novel advantageous sensory profiles.

Furthermore, the compounds of formula (I) as well as mixtures of two or more compounds of formula (I), their stereoisomers and the mixtures of their stereoisomers can be produced in good yields and purities by a simple synthesis which generally requires only one step, starting from readily available starting compounds. Thus, the compounds of formula (I) as well as mixtures of two or more compounds of formula (I), their stereoisomers and the mixtures of their stereoisomers can be produced in large scales and in a simple and cost-efficient manner.

### DETAILED DESCRIPTION OF THE INVENTION

In mixtures of two or more compounds of the formula (I) the two or more different compounds (I) differ in the definition of at least one of the radicals R¹ and R².

In the context of the present invention, the term "alkyl" as used herein refers to a linear or branched saturated hydrocarbon radicals having 1 to 3 ("C₁-C₃-alkyl"), 1 to 4 ("C₁-C₄-alkyl") or 2 to 4 ("C₂-C₄-alkyl") carbon atoms. C₁-C₃-Alkyl is methyl, ethyl, propyl and isopropyl. C₁-C₄-Alkyl is additionally n-butyl, 1-methylpropyl (sec-butyl), 2-methylpropyl (isobutyl) or 1,1-dimethylethyl (tert-butyl).

In the context of the present invention, the term linear "C₁-C₄-alkylene" as used herein refers to a linear divalent alkanediyl radical having 1 to 4 carbon atoms ("C₁-C₄-alkylene"). Linear C₁-C₄-alkylene are methylene, 1,2-ethylene, 1,3-propylene and 1,4-butylene.

In the context of the present invention, the term "C₁-C₅-alkanoyl" denotes a hydrogen atom or a C₁-C₄-alkyl group, as defined above, attached via a carbonyl [(C=O)] group to the remainder of the molecule. The term "C₂-C₅-alkanoyl" denotes a C₁-C₄-alkyl group, as defined above, attached via a carbonyl [(C=O)] group to the remainder of the molecule. C₁-C₅-alkanoyl is formyl, methylcarbonyl, ethylcarbonyl, n-propylcarbonyl, isopropylcarbonyl, n-butylcarbonyl, 1-methylpropylcarbonyl, 2-methylpropylcarbonyl or 1,1-dimethylethylcarbonyl.

The term "stereoisomers" encompasses optical isomers, such as enantiomers or diastereomers, the latter existing due to more than one stereogenic center in the molecule. The compounds of the formula (I) have one stereogenic center, namely the carbon atom in the 1-position of the cycloalkane ring. Furthermore, the radical R² may also have a stereogenic center, for example if R² is selected from 1-methylpropyl or 1-methylpropylcarbonyl. The invention provides both the pure enantiomers or diastereomers and mixtures thereof and the use according to the invention of the pure enantiomers or pure diastereomers of the compound (I) or mixtures thereof.

In the present context, the term "compound (I)" or "compound of formula (I)", when not defined as a specific stereoisomer or a specific mixture of stereoisomers, refers to the form of the compound as it is obtained in a non-stereoselective method used for its production. The term is however also used if it is not necessary or not possible to specify in more detail the stereochemistry of the compound (I).

Preferably, X is selected from linear C₁-C₄-alkylene, which is unsubstituted or substituted by 1, 2, 3 or 4 radicals independently of each other selected from C₁-C₄-alkyl,where X is chosen such that the total number of carbon atoms in the compound of the general formula (I) is 16 or less.

More preferably, X is selected from linear C₁-C₃-alkylene, which is unsubstituted or substituted by 1, 2 or 3 radicals independently of each other selected from C₁-C₃-alkyl, where X is chosen such that the total number of carbon atoms in the compound of the general formula (I) is 16 or less.

Even more preferably, X is selected from linear C₁-C₃-alkylene, which is unsubstituted or substituted by 1, 2 or 3 methyl groups.

In particular, X is selected from linear C₂-C₃-alkylene, which is unsubstituted or substituted by 1, 2 or 3 methyl groups.

Specifically, X is selected from linear C₁-C₄-alkylene, which is unsubstituted. More specifically, X is selected from the group consisting of 1,2-ethanediyl, 1,3-propanediyl and 1,4-butanediyl. Even more specifically, X is 1,2-ethanediyl or 1,3-propanediyl.

Preferably, R¹ is ethyl.

A first preferred embodiment of the present invention relates to the use of a compound of the general formula (I) 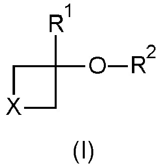 wherein
- X: is linear C₁-C₄-alkylene,
- R¹: is ethyl or ethenyl, and
- R²: is C₁-C₄-alkyl,
or of a mixture of two or more compounds of the general formula (I),
or of a stereoisomer thereof or of a mixture of two or more stereoisomers thereof, as an aroma chemical.

Preferably, in compounds (I) of this first preferred embodiment,
- X: is 1,2-ethanediyl, 1,3-propanediyl or 1,4-butanediyl, in particular 1,2-ethanediyl or 1,3-propanediyl,
- R¹: is ethyl or ethenyl, in particular ethyl, and
- R²: is C₁-C₄-alkyl.

More preferably, in compounds (I) of this first preferred embodiment,
- X: is 1,2-ethanediyl, 1,3-propanediyl or 1,4-butanediyl, in particular 1,2-ethanediyl or 1,3-propanediyl,
- R¹: is ethyl or ethenyl, in particular ethyl, and
- R²: is C₁-C₃-alkyl.

Even more preferably, in compounds (I) of this first preferred embodiment,
- X: is 1,2-ethanediyl, 1,3-propanediyl or 1,4-butanediyl, in particular 1,2-ethanediyl or 1,3-propanediyl,
- R¹: is ethyl, and
- R²: is C₁-C₃-alkyl, in particular methyl and ethyl.

In particular, in compounds (I) of this first preferred embodiment,
- X: is 1,2-ethanediyl or 1,3-propanediyl,
- R¹: is ethyl, and
- R²: is methyl or ethyl, especially ethyl.

A second preferred embodiment of the present invention relates to the use of a compound of the general formula (I), 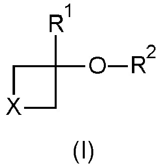 wherein
- X: is linear C₁-C₄-alkylene,
- R¹: is ethyl or ethenyl, and
- R²: is C₂-C₄-alkyl,
or of a mixture of two or more compounds of the general formula (I),
or of a stereoisomer thereof or of a mixture of two or more stereoisomers thereof, as an aroma chemical.

Preferably, in compounds (I) of this second preferred embodiment,
- X: is 1,2-ethanediyl, 1,3-propanediyl or 1,4-butanediyl, in particular 1,2-ethanediyl or 1,3-propanediyl,
- R¹: is ethyl or ethenyl, in particular ethyl, and
- R²: is C₂-C₄-alkyl.

More preferably, in compounds (I) of this second preferred embodiment,
- X: is 1,2-ethanediyl, 1,3-propanediyl or 1,4-butanediyl, in particular 1,2-ethanediyl or 1,3-propanediyl,
- R¹: is ethyl, and
- R²: is C₂-C₄-alkyl.

Even more preferably, in compounds (I) of this second preferred embodiment,
- X: is 1,2-ethanediyl or 1,3-propanediyl,
- R¹: is ethyl, and
- R²: is C₂-C₃-alkyl, in particular propyl.

A third preferred embodiment of the present invention relates to the use of a compound of the general formula (I) 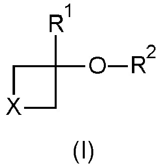 wherein
- X: is linear C₁-C₄-alkylene,
- R¹: is ethyl or ethenyl, and
- R²: is C₁-C₅-alkanoyl,
or of a mixture of two or more compounds of the general formula (I),
or of a stereoisomer thereof or of a mixture of two or more stereoisomers thereof, as an aroma chemical.

Preferably, in compounds (I) of this third preferred embodiment,
- X: is 1,2-ethanediyl, 1,3-propanediyl or 1,4-butanediyl, in particular 1,2-ethanediyl or 1,3-propanediyl,
- R¹: is ethyl or ethenyl, in particular ethyl, and
- R²: is C₁-C₅-alkanoyl.

More preferably, in compounds (I) of this third preferred embodiment,
- X: is 1,2-ethanediyl, 1,3-propanediyl or 1,4-butanediyl, in particular 1,2-ethanediyl or 1,3-propanediyl,
- R¹: is ethyl or ethenyl, in particular ethyl, and
- R²: is C₂-C₅-alkanoyl.

Even more preferably, in compounds (I) of this third preferred embodiment,
- X: is 1,2-ethanediyl, 1,3-propanediyl or 1,4-butanediyl, in particular 1,2-ethanediyl or 1,3-propanediyl,
- R¹: is ethyl, and
- R²: is C₂-C₄-alkanoyl.

In particular, in compounds (I) of this third preferred embodiment,
- X: is 1,2-ethanediyl or 1,3-propanediyl,
- R¹: is ethyl, and
- R²: is acetyl or propanoyl, especially acetyl.

A fourth preferred embodiment of the present invention relates to the use of a compound of the general formula (I) 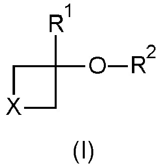 wherein
- X: is linear C₁-C₄-alkylene,
- R¹: is ethyl or ethenyl, and
- R²: is C₃-C₅-alkanoyl,
or of a mixture of two or more compounds of the general formula (I),
or of a stereoisomer thereof or of a mixture of two or more stereoisomers thereof, as an aroma chemical.

Preferably, in compounds (I) of this fourth preferred embodiment,
- X: is 1,2-ethanediyl, 1,3-propanediyl or 1,4-butanediyl, in particular 1,2-ethanediyl or 1,3-propanediyl,
- R¹: is ethyl or ethenyl, in particular ethenyl, and
- R²: is C₃-C₅-alkanoyl.

More preferably, in compounds (I) of this fourth preferred embodiment,
- X: is 1,2-ethanediyl, 1,3-propanediyl or 1,4-butanediyl, in particular 1,2-ethanediyl or 1,3-propanediyl,
- R¹: is ethyl, and
- R²: is C₃-C₄-alkanoyl.

Even more preferably, in compounds (I) of this fourth preferred embodiment,
- X: is 1,2-ethanediyl or 1,3-propanediyl,
- R¹: is ethyl, and
- R²: is C₃-C₄-alkanoyl.

In particular, in compounds (I) of this fourth preferred embodiment,
- X: is 1,2-ethanediyl or 1,3-propanediyl,
- R¹: is ethyl, and
- R²: is propanoyl.

Preferable compounds (I) that can be advantageously used as aroma chemicals are for example
1-methoxy-1-ethyl-cyclohexane,
1-ethoxy-1-ethyl-cyclohexane,
1-methoxy-1-vinyl-cyclohexane,
1-ethoxy-1-vinyl-cyclohexane,
1-methoxy-1-ethyl-cyclopentane,
1-ethoxy-1-ethyl-cyclopentane,
1-methoxy-1-vinyl-cyclopentane,
1-ethoxy-1-vinyl-cyclopentane,
(1-ethylcyclohexyl) acetate,
(1-vinylcyclohexyl) acetate,
(1-ethylcyclohexyl) propanoate,
(1-vinylcyclohexyl) propanoate,
(1-ethylcyclopentyl) acetate,
(1-vinylcyclopentyl) acetate,
(1-ethylcyclopentyl) propanoate, or
(1-ethylcyclopentyl) propanoate.

Particularly preferable compounds (I) that can be advantageously used as aroma chemicals are for example
1-methoxy-1-ethyl-cyclohexane,
1-ethoxy-1-ethyl-cyclohexane,
1-methoxy-1-vinyl-cyclohexane,
1-ethoxy-1-vinyl-cyclohexane,
1-methoxy-1-ethyl-cyclopentane,
(1-ethylcyclohexyl) acetate,
(1-vinylcyclohexyl) acetate,
(1-ethylcyclohexyl) propanoate, or
(1-ethylcyclopentyl) acetate.

The compounds of formula (I) as well as the mixtures of two or more compounds of formula (I), the stereoisomers thereof and the mixtures of stereoisomers thereof, as defined above, are useful as aroma chemicals.

The term "aroma chemical" denotes a substance which is used to obtain a sensory impression, to be more precise an olfactory or flavor impression, in particular a fragrance or flavor impression. The term "olfactory" denotes an odor impression without any positive or negative judgement, while the term "fragrance" (also termed "perfume" or "scent") is connected to an odor impression which is generally felt as pleasant. A flavor induces a taste impression.

"Pleasant odor", "pleasant odor impression", "pleasant odiferous properties" are hedonistic expressions which describe the niceness and conciseness of an odor impression conveyed by an aroma chemical. The more general hedonistic expressions "advantageous sensory properties" or "advantageous organoleptic properties" describe the niceness and conciseness of an organoleptic impression conveyed by an aroma chemical. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typically tart apple can also be concise. If both reactions arise upon smelling the substance, in the example thus a nice and concise apple odor, then this substance has particularly advantageous sensory properties.

The term "odor-intensive substances" refers to substances or aroma chemicals exhibiting intense odor impressions. Intense odor impressions are to be understood as meaning those properties of aroma chemicals which permit a striking perception even in very low gas space concentrations. The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

Preferably, the compounds of formula (I) as well as the mixtures of two or more compounds of formula (I), the stereoisomers thereof and the mixtures of stereoisomers thereof, as defined above, are used as a fragrance.

In particular, 1-methoxy-1-ethyl-cyclohexane is used to impart a eucalyptus leaf, ethereal, pine needle and green note; or is used to produce a scent with a eucalyptus leaf, ethereal, pine needle and green note.
In particular, 1-ethoxy-1-ethyl-cyclohexane is used to impart a camphor, rosemary, sweet and fruity note; or is used to produce a scent with a camphor, rosemary, sweet and fruity note.
In particular, 1-methoxy-1-vinyl-cyclohexane is used to impart a minty, cool, sweet and floral note; or is used to produce a scent with a minty, cool, sweet and floral note.
In particular, 1-ethoxy-1-vinyl-cyclohexane is used to impart an eucalyptus and leave note; or are used to produce a scent with an eucalyptus and leave note.
In particular, 1-methoxy-1-ethyl-cyclopentane is used to impart a sweet and grapefruit note; or is used to produce a scent with a sweet and grapefruit note.
In particular, (1-ethylcyclohexyl) acetate is used to impart a sweet, damascone, dry, hay and fresh note; or is used to produce a scent with a sweet, damascone, dry, hay and fresh note.
In particular, (1-vinylcyclohexyl) acetate is used to impart a fresh, raspberry, sweet, woody and etheral note; or is used to produce a scent with a fresh, raspberry, sweet, woody and etheral note.
In particular, (1-ethylcyclohexyl) propanoate is used to impart a plum, powdery, chamomile and raisin note; or is used to produce a scent with a plum, powdery, chamomile and raisin note.
In particular, (1-ethylcyclopentyl) acetate is used to impart a dried fruit, damascone, sweet and woody note; or is used to produce a scent with a dried fruit, damascone, sweet and woody note.

The compounds of formula (I) as well as the mixtures of two or more compounds of formula (I), the stereoisomers thereof and the mixtures of stereoisomers thereof, as defined above, are generally used in a ready-to-use composition, in particular in a fragranced ready-to-use composition. "Fragranced ready-to-use composition", as used herein, refers to a ready-to-use composition which predominately induces a pleasant odor impression.

Fragranced ready-to-use compositions are for example compositions used in personal care, in home care, in industrial applications as well as compositions used in other applications, such as pharmaceutical compositions or crop protection compositions.

Preferably, the compounds of formula (I) as well as the mixtures of two or more compounds of formula (I), the stereoisomers thereof and the mixtures of stereoisomers thereof are used in a composition selected from the group consisting of perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions and crop protection compositions. The compounds of formula (I) as well as the mixtures of two or more compounds of formula (I), the stereoisomers thereof and the mixtures of stereoisomers thereof are used as an aroma chemical, preferably as a fragrance, in the above compositions.

In particular, 1-methoxy-1-ethyl-cyclohexane is used to impart a eucalyptus leaf, ethereal, pine needle and green note to the above-listed compositions.
In particular, 1-ethoxy-1-ethyl-cyclohexane is used to impart a camphor, rosemary, sweet and fruity note to the above-listed compositions.
In particular, 1-methoxy-1-vinyl-cyclohexane is used to impart a minty, cool, sweet and floral note to the above-listed compositions.
In particular, 1-ethoxy-1-vinyl-cyclohexane is used to impart an eucalyptus and leave note to the above-listed compositions.
In particular, 1-methoxy-1-ethyl-cyclopentane is used to impart a sweet and grapefruit note to the above-listed compositions.
In particular, (1-ethylcyclohexyl) acetate is used to impart a sweet, damascone, dry, hay and fresh note to the above-listed compositions.
In particular, (1-vinylcyclohexyl) acetate is used to impart a fresh, raspberry, sweet, woody and etheral note to the above-listed compositions.
In particular, (1-ethylcyclohexyl) propanoate is used to impart a plum, powdery, chamomile and raisin note to the above-listed compositions.

In particular, (1-ethylcyclopentyl) acetate is used to impart a dried fruit, damascone, sweet and woody note to the above-listed compositions.

Details to the above-listed compositions are given below.

Accordingly, another aspect of the invention relates to the use of a compounds of formula (I) or mixtures of two or more compounds of formula (I), or a stereoisomer thereof or a mixtures of stereoisomers thereof for modifying the scent character of a fragranced composition.

In addition to the olfactory properties, the compounds of formula (I) as well as the mixtures of two or more compounds of formula (I), the stereoisomers thereof and the mixtures of stereoisomers thereof exhibit advantageous secondary properties.

For example, they can provide better sensory profiles as a result of synergistic effects with other fragrances, which means that they can provide a booster effect for other fragrances. They can therefore be used as boosters for other fragrances.

Accordingly, another aspect of the invention relates to the use of a compound of formula (I) or mixtures of two or more compounds of formula (I), or a stereoisomer thereof or a mixtures of stereoisomers thereof as a booster for other fragrances.

Booster effect means that the substances enhance and intensify in perfumery formulations the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione® (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the compounds of formula (I) or the mixture of two or more compounds of formula (I), or the stereoisomers thereof or the mixtures of stereoisomers thereof are generally used in an overall amount of 0.1 - 20% by weight, preferably in an amount of 0.5 to 5% by weight, in particular in an amount of from 0.6 to 3% by weight, based on the total weight of the fragrance mixture.

Furthermore, the compounds of formula (I) as well as the mixtures of two or more compounds of formula (I), the stereoisomers thereof and the mixtures of stereoisomers thereof can have further positive effects on the composition in which they are used. For example, they can enhance the overall performance of the composition into which they are incorporated, such as the stability, e.g. the formulation stability, the extendability or the staying power of the composition.

In another aspect, the present invention relates to an aroma chemical composition comprising the compounds of formula (I) or a mixture of two or more compounds of formula (I), or a stereoisomer thereof or a mixture of stereoisomers thereof. The term "aroma chemical composition", as used herein, refers to a composition which induces a pleasant odor impression.

Preferably, the aroma chemical composition comprises
- a compound of formula (I) or a mixture of two or more compounds of the general formula (I) or a stereoisomer thereof or a mixture of two or more stereoisomers thereof; and
- at least one further aroma chemical and/or a non-aroma chemical carrier, where the non-aroma chemical carrier is in particular selected from the group consisting of surfactants, oil components and solvents.

The further aroma chemical is of course different from the compounds of formula (I) or its stereoisomers or mixtures of its stereoisomers.

By virtue of their physical properties, the compounds of formula (I) as well as the mixtures of two or more compounds of formula (I), the stereoisomers thereof and the mixtures of stereoisomers thereof have particularly good, virtually universal solvent properties for other fragrances and other customary ingredients in fragranced ready to use compositions such as, in particular, perfume compositions. Therefore, they are well combinable with other aroma chemicals, allowing, in particular, the creation of perfume compositions having novel advantageous sensory profiles. Furthermore, as already explained above, they can provide a booster effect for other fragrances.

Accordingly, in one preferred embodiment, the aroma chemical composition comprises a compound of formula (I) or a mixture of two or more compounds of formula (I), a stereoisomer thereof or a mixture of stereoisomers thereof, as defined above; and at least one further aroma chemical.

The further aroma chemical can for example be one, preferably 2, 3, 4, 5, 6, 7, 8 or further aroma chemicals, selected from the group consisting of:
Geranyl acetate (3,7-Dimethyl-2,6 octadien-1yl acetate), alpha-hexylcinnamaldehyde, 2-phenoxyethyl isobutyrate (Phenirat¹), dihydromyrcenol (2,6-dimethyl-7-octen-2-ol), methyl dihydrojasmonate (preferably with a content of cis isomer of more than 60% by weight) (Hedione⁹, Hedione HC⁹), 4,6,6,7,8,8-hexamethyl-1,3,4,6,7,8-hexahydrocyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lysmeral⁴), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat¹), citronellol, citronellyl acetate, tetrahydrogeraniol, vanillin, linalyl acetate, styrolyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone¹), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, (E)- and/or (Z)-3-methylcyclopentadec-5-enone (Muscenon⁹), 15-pentadec-11-enolide and/or 15-pentadec-12-enolide (Globalide¹), 15-cyclopentadecanolide (Macrolide¹), 1-(5,6,7,8-tetrahydro-3,5,5,6,8,8-hexamethyl-2-naphthalenyl)ethanone (Tonalid¹⁰), 2-isobutyl-4-methyltetrahydro-2H-pyran-4-ol(Florol⁹), 2-ethyl-4-(2,2,3-trimethyl-3-cyclopenten-1-yl)-2-buten-1-ol (Sandolen¹), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetramethyloct-6-en-3-one (Claritone¹), 2,6-dimethyl-5-hepten-1-al (Melonal²), borneol, 3-(3-isopropylphenyl)butanal (Florhydral²), 2-methyl-3-(3,4-methylenedioxyphenyl)-propanal (Helional³), 3-(4-ethylphenyl)-2,2-dimethylpropanal (Florazon¹), 7-methyl-2H-1,5-benzodioxepin-3(4H)-one (Calone⁹), 3,3,5-trimethylcyclohexyl acetate (preferably with a content of cis isomers of 70% by weight) or more and 2,5,5-trimethyl-1,2,3,4,4a,5,6,7-octahydronaphthalen-2-ol (Ambrinol S¹). Within the context of the present invention, the aforementioned aroma chemical(s) are accordingly preferably combined with the compound of formula (I) or a mixture of two or more compounds of formula (I), or a stereoisomer thereof or a mixture of stereoisomers thereof, as defined above.

A further embodiment of the invention relates to a composition comprising the compound of formula (I) or a mixture of two or more compounds of formula (I), or a stereoisomer thereof or a mixture of stereoisomers thereof, as defined above, and at least one further aroma chemical selected from the group consisting of methyl benzoate, benzyl acetate, geranyl acetate, 2-isobutyl-4-methyltetrahydro-2H-pyran-4-ol and linalool.

A further embodiment of the invention relates to a composition comprising the compound of formula (I) or a mixture of two or more compounds of formula (I), or a stereoisomer thereof or a mixture of stereoisomers thereof, as defined above, and 2-isobutyl-4-methyltetrahydro-2H-pyran-4-ol.

A further embodiment of the invention relates to a composition comprising the compound of formula (I) or a mixture of two or more compounds of formula (I), or a stereoisomer thereof or a mixture of stereoisomers thereof, as defined above, and methyl benzoate.

Where trade names are given above, these refer to the following sources:
¹ trade name of Symrise GmbH, Germany;
² trade name of Givaudan AG, Switzerland;
³ trade name of International Flavors & Fragrances Inc., USA;
⁴ trade name of BASF SE;
⁹ trade name of Firmenich S.A., Switzerland;
¹⁰ trade name of PFW Aroma Chemicals B.V., the Netherlands.

Further aroma chemicals with which the compound of formula (I) or a mixture of two or more compounds of formula (I), or a stereoisomer thereof or a mixture of stereoisomers thereof, as defined above, can be combined, e.g. to give a composition according to the invention, can be found e.g. in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
the aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
the aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
the aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
the aliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
the esters of aliphatic carboxylic acids such as e.g. (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
the acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
the acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
the cyclic terpene aldehydes and ketones such as e.g. menthone; isomenthone; 8-mercaptomenthan-3-one; carvone; camphor; fenchone; alpha-ionone; beta-ionone;
alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
the cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
the cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
the cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
the cyclic and macrocyclic ketones such as e.g. 4-tert-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclohexadecen-1-one; 7-cyclohexadecen-1-one; (7/8)-cyclohexadecen-1-one; 9-cycloheptadecen-1-one; cyclopentadecanone; cyclohexadecanone;
the cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
the cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1 ,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
the esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
the esters of cycloaliphatic alcohols such as e.g. 1-cyclohexylethyl crotonate;
the esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;
the araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
the esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
the araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
the aromatic and araliphatic aldehydes such as e.g. benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 2-methyl-3-(4-isobutylphenyl)propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
the aromatic and araliphatic ketones such as e.g. acetophenone; 4-methylacetophenone; 4-methoxyacetophenone; 4-tert-butyl-2,6-dimethylacetophenone; 4-phenyl-2-butanone; 4-(4-hydroxyphenyl)-2-butanone; 1-(2-naphthalenyl)-ethanone; 2-benzofuranylethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
the aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenyl-glycidate; ethyl 3-methyl-3-phenylglycidate;
the nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile;
3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclo-hexenecarbaldehyde; 6-isopropylquinoline; 6-isobutylquinoline; 6-sec-butylquinoline; 2-(3-phenylpropyl)pyridine; indole; skatole; 2-methoxy-3-isopropylpyrazine; 2-isobutyl-3-methoxypyrazine;
the phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
the heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

The at least one non-aroma chemical carrier can be a compound, a mixture of compounds or other additives, which have no or no noteworthy sensory properties. Typically, the at least one non-aroma chemical carrier, if present in the aroma chemical compositions according to the present invention, is a compound, a mixture of compounds or other additives, which have no or no noteworthy sensory properties. The non-aroma chemical carrier serves for the dilution and/or the fixing of the aroma chemical(s), i.e. the compounds of formula (I) and optionally one or more further aroma chemical different from compounds (I), as defined above, comprised in the aroma chemical composition.

Suitable carrier materials can be liquid or oil-like carrier materials as well as wax-like or solid carrier materials.

In particular, the non-aroma chemical carrier, if present in the compositions according to the present invention, is selected from the group consisting of surfactants, oil components and solvents.

Accordingly, a further aspect of the invention is directed to a composition comprising the compound of formula (I) or a mixture of two or more compounds of formula (I), or a stereoisomer thereof or a mixture of stereoisomers thereof, as defined above, and at least one component selected from the group consisting of surfactants, emollients (oil component) and solvents.

One embodiment of the invention is directed to a composition comprising the compound of formula (I) or a mixture of two or more compounds of formula (I), or a stereoisomer thereof or a mixture of stereoisomers thereof, as defined above, and at least one solvent.

In the context of the present invention, a "solvent" serves for the dilution of the compound of formula (I) or a mixture of two or more compounds of formula (I), or a stereoisomer thereof or a mixture of stereoisomers thereof, as defined above, to be used according to the invention without having its own odiferous properties. Some solvents have fixing properties at the same time.

The one or more solvent(s) can be present in the composition from 0.01 to 99% by weight based on the composition. In a preferred embodiment of the invention, the composition comprise 0.1 to 90 weight %, preferably 0.5 to 80 weight% of solvent(s) based on the composition. The amount of solvent(s) can be chosen depending on the composition. In one embodiment of the invention, the composition comprises 0.05 to 10 weight%, preferably 0.1 to 5 weight%, more preferably 0.2 to 3 weight% based on the composition. In one embodiment of the invention, the composition comprises 20 to 70 weight%, preferably 25 to 50 weight% of solvent(s) based on the composition.

Preferred solvents are ethanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

According to a further aspect, the compounds of formula (I) as well as the mixtures of two or more compounds of formula (I), the stereoisomers thereof and the mixtures of stereoisomers thereof are suitable for use in surfactant-containing compositions. According to their characteristic scent profiles, they can especially be used for the perfuming of surfactant-containing compositions such as, for example, cleaners (in particular laundry care products and all-purpose cleaners).

One embodiment of the invention is therefore directed to a composition comprising the compound of formula (I) or a mixture of two or more compounds of formula (I), or a stereoisomer thereof or a mixture of stereoisomers thereof, as defined above, and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in a quantity of 0 to 40% by weight, preferably 0 to 20% by weight, more preferably 0.1 to 15% by weight, and particularly 0.1 to 10% by weight, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, co-coacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C₈ to C₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl amino-propionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C₁₂₋C₁₈ alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart® series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolyzates.

One embodiment of the invention is directed to a composition comprising the compound of formula (I) or a mixture of two or more compounds of formula (I), or a stereoisomer thereof or a mixture of stereoisomers thereof, as defined above, and at least one oil component.

The oil components are typically present in a total quantity of 0.1 to 80, preferably 0.5 to 70, more preferably 1 to 60, even more preferably 1 to 50% by weight, in particular 1 to 40% by weight, more particularly 5 to 25% by weight and specifically 5 to 15% by weight based on the composition.

The oil components may be selected, for example, from Guerbet alcohols based on fatty alcohols containing 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl ole- ate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈-alkyl-hydroxycarboxylic acids with linear or branched C₆-C₂₂-fatty alcohols, more especially dioctyl malate, esters of linear and/or branched fatty acids with polyhydric alcohols (for example propylene glycol, dimer dial or trimer triol), triglycerides based on C₆-C₁₀-fatty acids, liquid mono-, di- and triglyceride mixtures based on C₆-C₁₈-fatty acids, esters of C₆-C₂₂-fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂-fatty alcohol carbonates such as, for example, dicaprylyl carbonate (Cetiol® CC), Guerbet carbonates based on fatty alcohols containing 6 to 18 and preferably 8 to 10 carbon atoms, esters of benzoic acid with linear and/or branched C₆ to C₂₂-alcohols (for example Finsolv® TN), linear or branched, symmetrical or non-symmetrical dialkyl ethers containing 6 to 22 carbon atoms per alkyl group such as, for example, dicaprylyl ether (Cetiol® OE), ring opening products of epoxidized fatty acid esters with polyols and hydrocarbons or mixtures thereof.

The compounds of formula (I) as well as the mixtures of two or more compounds of formula (I), the stereoisomers thereof and the mixtures of stereoisomers thereof, as defined above, can be used in a wide range of aroma chemical compositions. The olfactory properties, the substance properties (such as solubility in customary solvents and compatibility with further customary constituents of such compositions), as well as the toxicological acceptability of the compounds of formula (I) as well as the mixtures of two or more compounds of formula (I), the stereoisomers thereof and the mixtures of stereoisomers thereof underline their particular suitability for the stated use purposes and compositions.

Suitable aroma chemical compositions are for example perfume compositions, body care compositions, hygiene articles, cleaning compositions, textile detergent compositions, foods, food supplements, pharmaceutical compositions and crop protection compositions.

Perfume compositions can be selected from fine fragrances, air fresheners in liquid form, gel-like form or a form applied to a solid carrier, aerosol sprays, scented cleaners, perfume candles and oils, such as lamp oils or oils for massage.

Examples for fine fragrances are perfume extracts, Eau de Parfums, Eau de Toilettes, Eau de Colognes, Eau de Solide and Extrait Parfum.

Body care compositions include cosmetic compositions and products for oral and dental hygiene, and can be selected from after-shaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, after-shave creams and lotions, tanning creams and lotions, hair care products such as e.g. hairsprays, hair gels, setting hair lotions, hair conditioners, hair shampoo, permanent and semi-permanent hair colorants, hair shaping compositions such as cold waves and hair smoothing compositions, hair tonics, hair creams and hair lotions, deodorants and antiperspirants such as e.g. underarm sprays, roll-ons, deodorant sticks and deodorant creams, products of decorative cosmetics such as e.g. eye-liners, eye-shadows, nail varnishes, make-ups, lipsticks and mascara, and products for oral and dental hygiene, such as toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces, can be selected from perfumed acidic, alkaline and neutral cleaners, such as e.g. floor cleaners, window cleaners, dishwashing detergents both for handwashing and machine washing use, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, softgels, gelcaps, liquids, or powders.

Pharmaceutical compositions comprise compositions which are intended for use in the diagnosis, cure, mitigation, treatment, or prevention of disease as well as articles (other than food) intended to affect the structure or any function of the body of man or other animals.

Crop protection compositions comprise compositions which are intended for the managing of plant diseases, weeds and other pests (both vertebrate and invertebrate) that damage agricultural crops and forestry.

The compositions according to the invention can further comprise one or more substances, such as, for example: preservatives, abrasives, anti-acne agents, agents to combat skin aging, antibacterial agents, anti-cellulite agents, antidandruff agents, antiinflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, oils, waxes, fats, phospholipids, saturated fatty acids, mono- or polyunsaturated fatty acids, α-hydroxy acids, polyhydroxy fatty acids, liquefiers, dyes, color-protection agents, pigments, anticorrosives, polyols, surfactants, electrolytes, organic solvents or silicone derivatives.

The compounds of formula (I) as well as the mixtures of two or more compounds of formula (I), the stereoisomers thereof and the mixtures of stereoisomers thereof, as defined above, as well as the aroma chemical compositions according to the invention comprising them can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the compounds of formula (I) or the mixtures of two or more compounds of formula (I), or the stereoisomers thereof or mixtures of stereoisomers thereof, as defined above, and composition obtainable by the above method of the invention, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting the compounds of formula (I) or the mixtures of two or more compounds of formula (I), or the stereoisomers thereof or mixtures of stereoisomers thereof, as defined above, or the composition obtainable by the above method of the invention with a suitable wax-like substance and by extrusion with subsequent solidification, optionally in a suitable solvent, e.g. isopropanol.

Generally, the total amount of the compounds of formula (I) or the mixtures of two or more compounds of formula (I), or the stereoisomers thereof or mixtures of stereoisomers thereof, in the aroma chemical compositions according to the present invention is typically adapted to the particular intended use or the intended application and can, thus, vary over a wide range. As a rule, the customary standard commercial amounts for scents are used.

The compositions according to the invention can comprise the compounds of formula (I) or the mixtures of two or more compounds of formula (I), or the stereoisomers thereof or mixtures of stereoisomers thereof, as defined above, in an overall amount of from 0.001 to 99.9% by weight, preferably from 0.01 to 90% by weight, more preferably from 0.05 to 80%, in particular from 0.1 to 60% by weight, more particularly from 0.1 to 40% by weight, e.g. from 0.1 to 10% by weight or 0.1 to 15% by weight, based on the total weight of the composition.

In one embodiment of the invention, the compositions comprise the compounds of formula (I) or the mixtures of two or more compounds of formula (I), or the stereoisomers thereof or mixtures of stereoisomers thereof, as defined above, in an overall amount of from 0.001 to 5 weight%, preferably from 0.01 to 2 weight% based on the total weight of the composition.

A further aspect of the invention is directed to a method of preparing an aroma chemical composition, in particular a fragranced composition, especially a fragranced ready-to-use composition, or for modifying the scent character of an aroma chemical composition, in particular of a fragranced composition, especially of a fragranced ready-to-use composition, comprising incorporating a compound of formula (I) or a mixture of two or more compounds of the general formula (I) or a stereoisomer thereof or a mixture of two or more stereoisomers thereof, as defined above, into said composition.

In particular, the invention is directed to a method of preparing a perfume composition, body care composition, hygiene article, cleaning composition, textile detergent composition, food, food supplement, pharmaceutical composition or crop protection composition, comprising including a compound of formula (I) or a mixture of two or more compounds of the general formula (I) or a stereoisomer thereof or a mixture of two or more stereoisomers thereof, as defined above, into said perfume composition, body care composition, hygiene article, cleaning composition, textile detergent composition, food, food supplement, pharmaceutical composition or crop protection composition.

In one embodiment the invention is directed to a method for imparting a eucalyptus leaf, ethereal, pine needle and green note to a perfume composition, body care composition, hygiene article, cleaning composition, textile detergent composition, food, food supplement, pharmaceutical composition or crop protection composition, which comprises including 1-methoxy-1-ethyl-cyclohexane in a perfume composition, body care composition, hygiene article, cleaning composition, textile detergent composition, food, food supplement, pharmaceutical composition or crop protection composition.

In another embodiment the invention is directed to a method for imparting a camphor, rosemary, sweet and fruity note to a perfume composition, body care composition, hygiene article, cleaning composition, textile detergent composition, food, food supplement, pharmaceutical composition or crop protection composition, which comprises including 1-ethoxy-1-ethyl-cyclohexane in a perfume composition, body care composition, hygiene article, cleaning composition, textile detergent composition, food, food supplement, pharmaceutical composition or crop protection composition.

In another embodiment the invention is directed to a method for imparting a minty, cool, sweet and floral note to a perfume composition, body care composition, hygiene article, cleaning composition, textile detergent composition, food, food supplement, pharmaceutical composition or crop protection composition, which comprises including 1-methoxy-1-vinyl-cyclohexane in a perfume composition, body care composition, hygiene article, cleaning composition, textile detergent composition, food, food supplement, pharmaceutical composition or crop protection composition.

In another embodiment the invention is directed to a method for imparting an eucalyptus and leave note to a perfume composition, body care composition, hygiene article, cleaning composition, textile detergent composition, food, food supplement, pharmaceutical composition or crop protection composition, which comprises including 1-ethoxy-1-vinyl-cyclohexane in a perfume composition, body care composition, hygiene article, cleaning composition, textile detergent composition, food, food supplement, pharmaceutical composition or crop protection composition.

In another embodiment the invention is directed to a method for imparting a sweet and grapefruit note to a perfume composition, body care composition, hygiene article, cleaning composition, textile detergent composition, food, food supplement, pharmaceutical composition or crop protection composition, which comprises including 1-methoxy-1-ethyl-cyclopentane in a perfume composition, body care composition, hygiene article, cleaning composition, textile detergent composition, food, food supplement, pharmaceutical composition or crop protection composition.

In another embodiment the invention is directed to a method for imparting a sweet, damascone, dry, hay and fresh note to a perfume composition, body care composition, hygiene article, cleaning composition, textile detergent composition, food, food supplement, pharmaceutical composition or crop protection composition, which comprises including (1-ethylcyclohexyl) acetate in a perfume composition, body care composition, hygiene article, cleaning composition, textile detergent composition, food, food supplement, pharmaceutical composition or crop protection composition.

In another embodiment the invention is directed to a method for imparting a fresh, raspberry, sweet, woody and etheral note to a perfume composition, body care composition, hygiene article, cleaning composition, textile detergent composition, food, food supplement, pharmaceutical composition or crop protection composition, which comprises including (1-vinylcyclohexyl) acetate in a perfume composition, body care composition, hygiene article, cleaning composition, textile detergent composition, food, food supplement, pharmaceutical composition or crop protection composition.

In another embodiment the invention is directed to a method for imparting a plum, powdery, chamomile and raisin note to a perfume composition, body care composition, hygiene article, cleaning composition, textile detergent composition, food, food supplement, pharmaceutical composition or crop protection composition, which comprises including (1-ethylcyclohexyl) propanoate in a perfume composition, body care composition, hygiene article, cleaning composition, textile detergent composition, food, food supplement, pharmaceutical composition or crop protection composition.

In another embodiment the invention is directed to a method for imparting a dried fruit, damascone, sweet and woody note to a perfume composition, body care composition, hygiene article, cleaning composition, textile detergent composition, food, food supplement, pharmaceutical composition or crop protection composition, which comprises including (1-ethylcyclopentyl) acetate in a perfume composition, body care composition, hygiene article, cleaning composition, textile detergent composition, food, food supplement, pharmaceutical composition or crop protection composition.

The compounds of the formula (I) can be prepared by standard methods of organic chemistry.

To be more precise, the compounds (I), where R² is selected from C₁-C₅-alkanoyl, can efficiently be prepared for example by reacting the alcohol of formula (II) 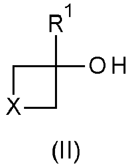 wherein X and R¹ have one of the meanings given above,
with a C₁-C₅-carboxylic acid or a C₁-C₅-carboxylic acid anhydride in the presence of an esterification catalyst, or
with a C₁-C₅-carboxylic acid chloride in the presence of a base.

Suitable esterification catalysts that can be applied in this reaction are well known to the skilled person. Suitable esterification catalysts are for example metal based catalysts, e.g. iron, cadmium, cobalt, lead, zinc, antimony, magnesium, titanium and tin catalysts in the form of metals, metal oxides or metal salts, such as metal alcoxylates; mineral acids, such as sulfuric acid, hydrochloric acid or phosphoric acid; or organic sulfonic acids, such as methane sulfonic acid or para-toluene sulfonic acid.

Suitable bases are preferably selected from organic bases. Suitable organic bases that can be used are for example tertiary amines, e.g. trimethylamine, triethylamine, tripropylamine, ethyldiisopropylamine and the like, or basic N-heterocycles, such as morpholine, pyridine, lutidine, DMAP, DABCO, DBU or DBN.

The individual reaction conditions for the preparation of the ester compounds of the general formula (I) are well known to the skilled person.

The compounds (I), where R² is selected from C₁-C₄-alkyl,can efficiently be prepared for example by reacting the alcohol of formula (II) 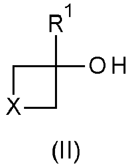 wherein X and R¹ have one of the meanings given above,
with an alkylation reagent R²-Y, wherein R² is selected from C₁-C₄-alkyl and Y represents a leaving group, selected from halogen, such as Cl, Br or I, sulfonates, such as tosylate, mesylate, triflate or nonaflate, sulfates, such as methylsulfate or ethylsulfate and carbonates, such as methylcarbonate or ethylcarbonatet, in the presence of a base.

Suitable bases are typically selected from inorganic bases and organic bases.

Suitable inorganic bases that can be used in this alkylation reaction are for example alkali metal carbonates, e.g. Li₂CO₃, Na₂CO₃, K₂CO₃ or CS₂CO₃, alkali metal hydroxides, e.g. LiOH, NaOH or KOH, and hydride donors, e.g. NaH, LiAlH₄ or NaBH₄.

Suitable organic bases that can be used in this alkylation reaction are for example tertiary amines, e.g. trimethylamine, triethylamine, tripropylamine, ethyldiisopropylamine and the like, or basic N-heterocycles, such as morpholine, pyridine, lutidine, DMAP, DABCO, DBU or DBN.

The alkylation reaction is performed under conventional alkylation reaction conditions that are well known to the skilled person.

The alcohol compounds (II) are either commercially available or they can be prepared from readily available precursors by using processes that are well described in the art.

For example the alcohols of the general formula (II.a) 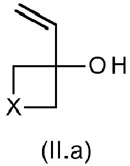 wherein X has one of the meanings given above,
can be prepared by ethynylation of the corresponding cycloketones of formula (III), as depicted in scheme 1 (step i)), followed by partial (selective) hydrogenation of the thus obtained acetylenically unsaturated alcohols (IV) (step ii)).

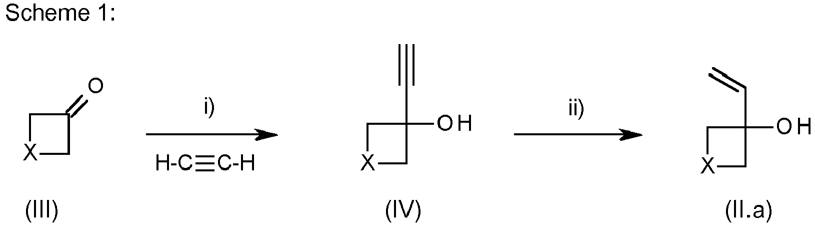

Typically, step i) is carried out in the presence of a strong base, e.g. an alkali metal hydroxide, such as sodium hydroxide or potassium hydroxide, or an alkali metal alkoxide, such as potassium methoxide, sodium methoxide, potassium ethoxide, sodium ethoxide, potassium isopropoxide or sodium isopropoxide. The mineral base is usually applied in amounts ranging from 0.1 to 5 equivalents, based on the amount of the carbonyl compound (III). The ethynylation is typically carried out in the presence of an excess of acetylene (ethyne), i.e. at an acetylene pressure in the range of from 1 to 50 bar. Usually, the acetylene pressure is maintained at an appropriate value over the course of the ethynylation reaction.

The ethynylation is usually carried out in an inert solvent, i.e. a solvent that does not react with the starting materials, intermediates and reagents applied in the ethynylation or with the obtained products. Suitable solvents are for example alcohols, such as methanol, ethanol, propanol and isopropanol; aromatic and substituted aromatic hydrocarbons, such as benzene, chlorobenzene, dichlorobenzenes, toluene, xylene; and aliphatic hydrocarbons, such as pentane, hexanes, cyclohexane, heptanes, octanes, nonanes, decanes, ligroin and petrol ether, halogenated aliphatic hydrocarbons, such as dichloromethane, trichloromethane and tetrachloromethane, ethers, such as dibutyl ether, THF, 1,4-dioxane, 1,2-dimethoxyethane; as well as mixtures thereof.

A similar process, where the alkynylation reaction is performed in ammonia as the solvent while the strong base is only applied in catalytic amounts, is for example described in EP1532092 B1 and US2015/0246868 A1.

The partial (selective) hydrogenation in step ii) is typically carried out in liquid phase with hydrogen in the presence of a hydrogenation catalyst but can also be carried out in gas phase. Preferably, the partial (selective) hydrogenation in step ii) is carried out in liquid phase. The hydrogenation catalyst and the reaction conditions are chosen such that the carbon-carbon triple bond is only partially hydrogenated to the corresponding carbon-carbon double bond.

Suitable hydrogenation catalysts that can be applied in the partial hydrogenation of the triple bond of compounds (IV) are those customarily used in the hydrogenation of carbon-carbon triple bonds to carbon-carbon double bonds. The hydrogenation catalyst can be selected from homogeneous hydrogenation catalysts and heterogeneous hydrogenation catalysts. Preferably, the hydrogenation catalyst is selected from heterogeneous hydrogenation catalysts. If a heterogeneous hydrogenation catalyst is used in the partial (selective) hydrogenation in step ii), the heterogeneous hydrogenation catalyst is typically applied in the form of a granulate or a finely divided powder, or in the form of larger molded bodies, for example molded bodies having a defined particle size of one to several millimeters, e.g. 1, 2, 5 or 10 millimeters. If a heterogeneous hydrogenation catalyst is used, the partial (selective) hydrogenation in step ii) is preferably carried out in suspension mode or in fixed bed mode.

The heterogeneous hydrogenation catalysts preferably comprise at least one metal of group VIII. Suitable metals of group VIII are selected from the group consisting of ruthenium, cobalt, rhodium, nickel, palladium and platinum, preferably form the group consisting of ruthenium, nickel, palladium and platinum. The heterogeneous hydrogenation catalysts especially comprise palladium as the catalytically active species.

Furthermore, the heterogeneous hydrogenation catalysts that can be applied in the partial hydrogenation of the triple bond of compounds (IV) typically contain, in addition to the catalytically active metal, a catalyst poison, i.e. a substance, which reduces the activity/effectiveness of the catalyst, in order to prevent the further hydrogenation of the obtained olefinic species. Suitable catalyst poisons are for example metals other than the catalytically active metals, such as for example Sn, Pb, Zn, Cd, Sb or Bi; metal salts of inorganic anions, such as for example barium sulfate or calcium carbonate; inorganic compounds, such as CO, CO₂, H₂O, H₂S, CS₂, SO₂ or sulfur; or organic compounds, such as nitrogen containing heterocycles, e.g. quinoline and pyridine, or thiols, e.g. ethane thiol.

Preferably, the heterogeneous hydrogenation catalysts that can be applied in the partial hydrogenation of the triple bond of compounds (IV) are supported heterogeneous hydrogenation catalyst. The support may be any of a variety of materials on which a catalytically active material may be coated. Typically support materials are preferred, which have a rather high surface area and which are stable under the applied reaction and, if required, the applied regeneration conditions. Suitable materials are, for example, mineral materials, for example natural and synthetic minerals, metal oxides, glasses or ceramics, carbon, for example activated carbon or carbon black, plastics, for example synthetic or natural polymers, or combinations thereof. Preferred support materials are for example activated carbon, silicon dioxide, in particular amorphous silicon dioxide, alumina, titanium dioxide, chromium dioxide, and also the sulfates and carbonates of the alkaline earth metals, such as calcium carbonate, calcium sulfate, magnesium carbonate, magnesium sulfate, barium carbonate and barium sulfate. The supported heterogeneous hydrogenation catalyst may be used in the form of powders, particles, pellets, monoliths, honeycombs, packed beds, foams, aerogels, granules, beads, pills, cylinders, trilobes, extrudates, spheres or other rounded shapes, or in the form of other manufactured configurations.

The reaction temperature applied in the partial hydrogenation in step ii) is generally from 10 to 100°C, preferably from 15 to 70°C, in particular from 20 to 50°C. The hydrogen pressure is generally from 1 to 20 bar absolute (0.1 to 2 MPa), preferably from 1 to 10 bar absolute (0.1 to 1 MPa). The hydrogenation can be carried out in a variety of reactors known for this purpose, such as for example stirred tank reactors, fixed bed reactors and trickle bed reactors. Preference is given to fixed bed reactors or stirred tank reactors.

The hydrogenation reaction can be carried out in the presence or the absence of an inert solvent. Preferably, the hydrogenation reaction is carried carried out in the presence of an inert solvent. Suitable inert solvent are as defined above.

Similarly, the alcohols of the general formula (II.b) 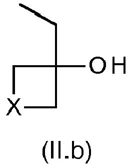 wherein X has one of the meanings given above,
can for example be prepared by ethynylation of the corresponding cycloketones of formula (III), as depicted in scheme 2 (step i)), followed by complete hydrogenation of the thus obtained acetylenically unsaturated alcohols (IV) (step iii)).

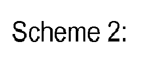

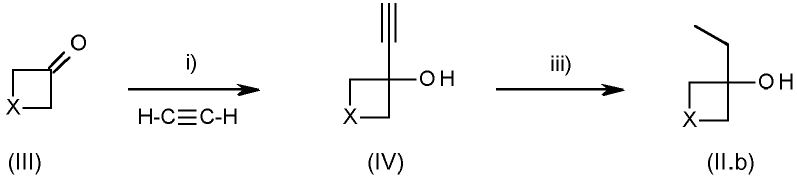

The catalytic hydrogenation in step iii) is typically carried out in liquid phase with hydrogen in the presence of a hydrogenation catalyst, but can also be carried out in gas phase. Preferably, the catalytic hydrogenation in step iii) is carried out in liquid phase.

Suitable hydrogenation catalysts are those customarily used in the hydrogenation of carbon-carbon triple bonds to carbon-carbon single bonds (complete hydrogenation). The hydrogenation catalyst can be selected from homogeneous hydrogenation catalysts and heterogeneous hydrogenation catalysts. Preferably, the hydrogenation catalyst is selected from heterogeneous hydrogenation catalysts. If a heterogeneous hydrogenation catalyst is used in the catalytic hydrogenation in step iii), the heterogeneous hydrogenation catalyst is typically applied in the form of a granulate or a finely divided powder, or in the form of larger molded bodies, as defined above. If a heterogeneous hydrogenation catalyst is used, the hydrogenation is preferably carried out in suspension mode or in fixed bed mode.

The hydrogenation catalysts preferably comprise at least one metal of group VIII and also VIIa. Suitable metals of group VIII are selected from the group consisting of iron, ruthenium, cobalt, rhodium, nickel, palladium and platinum. Particularly suitable metals of group VIII are iron, ruthenium, nickel, palladium and platinum. A suitable metal of group VIIa is rhenium. The metals may also be used in the form of mixtures, for example two or more metals of the group VIII. Metals of group VIII may also comprise further metals, for example metals of group VIIa, in particular rhenium, or metals of group Ib, i.e. copper, silver or gold. The catalyst especially comprises palladium as the catalytically active species.

The heterogeneous hydrogenation catalysts applied in the catalytic hydrogenation in step iii) can be supported or unsupported. Preferably, the heterogeneous hydrogenation catalysts applied in the catalytic hydrogenation in step iii) is a supported heterogeneous hydrogenation catalyst. Suitable supports are as defined above.

When a heterogeneous catalyst is used, it is preferably present in finely divided form, i.e. in the form of a fine granulate or a finely divided powder. The finely divided form is achieved, for example, as follows:
a) Black catalyst: shortly before use as a catalyst, the metal is deposited reductively from the solution of one of its salts.
b) Adams catalyst: the metal oxides, in particular the oxides of platinum and palladium, are reduced in situ by the hydrogen used for the hydrogenation.
c) Skeletal or Raney catalyst: the catalyst is prepared as a "metal sponge" from a binary alloy of the metal (in particular nickel or cobalt) with aluminum or silicon by leaching out one partner with acid or alkali. Residues of the original alloy partner often act synergistically.
d) Supported catalyst: black catalysts can also be precipitated on the surface of a support substance. Suitable supports and support materials are described below.

The reaction temperature is generally from 10 to 100°C, preferably from 15 to 70°C, in particular from 20 to 50°C. The hydrogen pressure is generally from 2 to 100 bar absolute (0.2 to 10 MPa), preferably from 5 to 50 bar absolute (0.5 to 5 MPa). The hydrogenation can be carried out in a variety of reactors known for this purpose. Preference is given to fixed bed reactors, in particular to trickle bed reactors.

The catalytic hydrogenation reaction in step iii) can be carried carried out in the presence or the absence of an inert solvent. Preferably, the hydrogenation reaction is carried carried out in the presence of an inert solvent. Suitable inert solvent are as defined above.

Generally, the reaction mixtures are worked up in a customary manner, for example by mixing with water, neutralizing the reaction mixture, if acids and bases were applied in the reaction, separating the phases, isolating the product from the organic phases and, if appropriate, purifying the crude products by usual methods, e.g. by distillative, extractive or chromatographic methods. If the reaction is not run in fixed bed mode and a heterogeneous catalyst, e.g. a heterogeneous hydrogenation catalyst, is applied in the reactions the catalyst is filtered off prior to work up.

In another aspect, the invention relates to a compound of the general formula (I.a) or a stereoisomer thereof 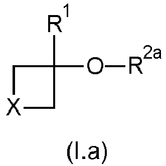 wherein
- X: is 1,2-ethanediyl or 1,3-propanediyl,
- R¹: is ethyl or ethenyl, and
- R^{2a}: is C₂-C₄-alkyl.

Preferably, in these compounds (I.a) R^{2a} is C₂-C₃-alkyl, in particular ethyl.

In particular, the invention relates to compounds (I.a) selected from the group consisting of
1-ethoxy-1-ethyl-cyclohexane,
1-ethoxy-1-ethyl-cyclopentane, and
1-ethoxy-1-vinyl-cyclohexane.

The compounds of the general formula (I.a) can be prepared as described above for the ether compounds of the general formula (I).

Accordingly, the present invention also relates to a method for preparing the compound of formula (I.a), a stereoisomer thereof or a mixture of stereoisomers thereof, as defined above, comprising the following steps:
(i) providing a cycloketone of formula (III), wherein X is 1,2-ethanediyl or 1,3-propanediyl,
(ii) reacting the cycloketone provided in step (i) with acetylene in the presence of a base to obtain an acetylenically unsaturated alcohol of the general formula (IV), wherein X is 1,2-ethanediyl or 1,3-propanediyl,
(iii) partially or completely hydrogenating the acetylenically unsaturated alcohol (IV) obtained in step (ii) with hydrogen in the presence of an hydrogenation catalyst to obtain the saturated alcohol of the general formula (II.a), wherein X is 1,2-ethanediyl or 1,3-propanediyl (complete hydrogenation), or the olefinic alcohol of the general formula (II.b), wherein X is 1,2-ethanediyl or 1,3-propanediyl (partial hydrogenation),
(iv) reacting the alcohol (II.a) or (II.b) obtained in step (iii) with an alkylation reagent R^{2a}-Y, wherein R^{2a} is C₂-C₄-alkyl and Y represents a leaving group, selected from halogen, such as Cl, Br or I, sulfonates, such as tosylate, mesylate, triflate or nonaflate, sulfates, such as methylsulfate or ethylsulfate and carbonates, such as methylcarbonate or ethylcarbonatet, in the presence of a base, to obtain a raw-product mixture, and
(v) subjecting the raw-product mixture obtained in step (iv) to a purification step.

Suitable and preferred reagents and reaction conditions are as described above.

In yet another aspect, the invention relates to a compound of the general formula (I.b) or a stereoisomer thereof 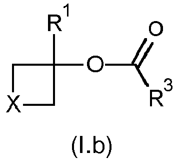 wherein
- X: is 1,2-ethanediyl or 1,3-propanediyl,
- R¹: is ethyl or ethenyl, and
- R³: is C₂-C₄-alkyl.

Preferably, in these compounds (I.b) R³ is C₂-C₃-alkyl, in particular ethyl.

In particular, the invention relates to (1-vinylcyclohexyl) propanoate.

The compounds of the general formula (I.b) can be prepared as described above for the ester compounds of the general formula (I).

Accordingly, the present invention also relates to a method for preparing the compound of formula (I.b), a stereoisomer thereof or a mixture of stereoisomers thereof, as defined above, comprising the following steps:
(i) providing a cycloketone of formula (III), wherein X is 1,2-ethanediyl or 1,3-propanediyl,
(ii) reacting the cycloketone provided in step (i) with acetylene in the presence of a base to obtain an acetylenically unsaturated alcohol of the general formula (IV), wherein X is 1,2-ethanediyl or 1,3-propanediyl,
(iii) partially or completely hydrogenating the acetylenically unsaturated alcohol (IV) obtained in step (ii) with hydrogen in the presence of an hydrogenation catalyst to obtain the saturated alcohol of the general formula (II.a), wherein X is 1,2-ethanediyl or 1,3-propanediyl (complete hydrogenation), or the olefinic alcohol of the general formula (II.b), wherein X is 1,2-ethanediyl or 1,3-propanediyl (partial hydrogenation),
(iv) reacting the alcohol (II.a) or (II.b) obtained in step (iii) with a C₃-C₅-carboxylic acid or a C₃-C₅-carboxylic acid anhydride in the presence of an esterification catalyst, or with a C₃-C₅-carboxylic acid chloride in the presence of a base, to obtain a raw-product mixture, and
(v) subjecting the raw-product mixture obtained in step (iv) to a purification step.

Suitable and preferred reagents and reaction conditions are as described above.

The invention is illustrated by the following examples.

### EXAMPLES

### Abbreviations:

- GC:: Gas Chromatography
- RT:: retention time
- DMAP:: 4-dimethylaminopyridine
- MTBE:: methyl tert.-butyl ether

### Analytics:

The purity of the products was determined by gas chromatography on the basis of area-%:
GC-system: Agilent 7890 B;
GC-Column: DB-WAX (30 m (Length), 0.32 mm (ID), 0,25 micrometer (film));
Temperature program: 85 °C to 230 °C at 5°/min

The products were identified by ¹³C NMR.

### 1. Preparation examples

### 1.1 Preparation of 1-ethinyl-cyclohexane-1-ol

In a 5 I autoclave, 308.5 g (5.5 mol) sodium methoxide is suspended in 1500 ml tetra-hydrofurane and purged with nitrogen. The autoclave is pressurized with 2 bar nitrogen and 18 bar acetylene at 10°C. 5 mol of cyclohexanone is continuously added over 16 h at 10°C, while keeping the pressure at 20 bar. The mixture is stirred for additional 5 h at 0°C before releasing the pressure. 500 ml water is carefully added while keeping the temperature below 30°C. The organic phase is separated, the pH adjusted to pH7 with phosphoric acid and the so obtained crude product is fractionated distilled to obtain the pure 1-ethinyl-cyclohexan-1-ol.

### 1.2 Preparation of 1-vinyl-cyclohexane-1-ol

A Steel-Autoklave was charged with 15 g of 1-ethinyl-cyclohexane-1-ol, prepared according to example 1.1, 135 g methanol and 0.15 g Lindlar-Catalyst (Pd on Calcium Carbonat, Poisoned with lead, commercially available e.g. from Sigma-Aldrich). The hydrogenation was performed at a hydrogen pressure of 2-3 bar and a temperature of 30 °C. After a reaction time of 1 h complete conversion of the starting material was observed. The partial hydrogenation was observed with a selectivity of 80% (GC area-%). The reaction was stopped. The catalyst was filtered off and the solvent was evaporated at reduced pressure. The reaction product, i.e. 1-vinyl-cyclohexane-1-ol, was directly applied in the next step without further purification.

### 1.3 Preparation of 1-ethyl-cyclohexane-1-ol

1-Ethynyl-1-cyclohexane-1-ol (50 g, 0.4 mol), prepared according to example 1.1, 1,4-dioxane (50 g) and Pd/C (6 g, 12 weight-%) were mixed in an autoclave. The autoclave was cooled in an ice bath and hydrogen (20 bar) was applied. The reaction mixture was stirred for 4.5 hours at 600 rounds/min with cooling. When the pressure dropped below 10 bar, hydrogen was pressed onto the autoclave again (20 bar; 7 times within 4.5 hours). The reaction was stirred overnight at room temperature with 5 bar hydrogen pressure. After a total of 23 hours reaction time, GC analysis of an aliquot sample indicated complete conversion of the starting material and the intermediary alkene. The crude product was removed from the autoclave with addition of dioxane (10 g) and filtered over celite. The residue was washed with dioxane (30 g). The first filtrate was 55.7 g of dioxane solution containing 44.75 GC area-% of the desired product; the second filtrate was 35.9 g of dioxane solution containing 39.76 GC area-% of the desired product. Assuming similar response factors in GC, the yield of the desired 1-ethyl-cyclohexane-1-ol is approximately 76 % (39.2 g in dioxane solution; 0.305 mol).

### 1.4 Preparation of 1-ethyl-cyclohexyl-acetate:

| **Compound** | **MW** | **Mass/Volume** | **Moles** |
|---|---|---|---|
| 1-ethyl-cyclohexane-1-ol | 128.21 | 15g | 0.117 |
| acetic anhydride | 102.09 | 14.33g | 0.140 |
| N,N'-dimethylaminopyridine | 122.17 | 0.429g | 0.004 |
| Tetrahydrofuran | | 50mL | |

DMAP was added to the THF solution of the alcohol at RT. The mixture was set to reflux (53°C) and acetic anhydride (1,2eq) was slowly added at this temperature. After 142h, 94% alcohol conversion was observed by GC. The reaction was cooled down to room temperature and slowly quenched with 50mL of water. 50mL of ethyl acetate were then added. The organic phase was separated and washed with NaHCO₃ and brine solution. The organic extracts were combined and dried with sodium sulfate. After evaporating the solvent at reduced pressure, 18.2g of crude product that contained ca 93% of the acetate according to the GC (area %) were obtained. The product was purified by distillation. The corresponding acetate was obtained with a purity of 98% (GC area %). The identity of the product was confirmed by NMR. 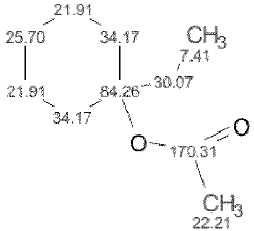 1-Ethyl-cyclohexyl-acetate: ¹³C NMR (125 MHz, CDCl₃): δ = 7.41, 21.91, 22.21, 25.70, 30.07, 34.17, 84.26, 170.31

### 1.5 Preparation of 1-vinyl-cyclohexyl-acetate

| **Compound** | **MW** | **Mass/Volume** | **Moles** |
|---|---|---|---|
| 1-vinyl-cyclohexane-1-ol | 126.20 | 7g | 0.055 |
| acetic anhydride | 102.09 | 6.79g | 0.067 |
| N,N'-dimethylaminopyridine | 122.17 | 0.203g | 0.002 |
| Tetrahydrofuran | | 50mL | |

DMAP was added to the THF solution of the alcohol at RT. The mixture was set to reflux (53°C) and acetic anhydride (1.2eq) was slowly added at this temperature. After 96h, 75% alcohol conversion was observed by GC. The reaction was cooled down to room temperature and slowly quenched with 50mL of water. 50mL of ethyl acetate were then added. The organic phase was separated and washed with NaHCO₃ and brine solution. The organic extracts were combined and dried with sodium sulfate. After evaporating the solvent at reduced pressure, 6.2g of crude product that contained ca 73% of the acetate according to the GC (area %) were obtained. The product was purified by column chromatography. The corresponding acetate was obtained with a purity of 99% (GC area %). The identity of the product was confirmed by NMR. 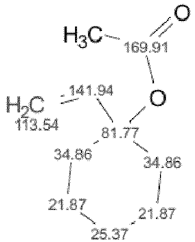 1-Vinyl-cyclohexyl-acetate: ¹³C NMR (125 MHz, CDCl₃): δ = 21.87, 22.12 25.37, 34.86, 81.77, 113.54, 141.94, 169.91

### 1.6 Preparation of 1-vinyl-cyclohexyl-propanoate:

| **Compound** | **MW** | **Mass/Volume** | **Moles** |
|---|---|---|---|
| 1-vinyl-cyclohexanol | 126.20 | 15g | 0.119 |
| propionic anhydride | 130.14 | 20.11 g | 0.155 |
| N,N'-dimethylaminopyridine | 122.17 | 0.436g | 0.004 |
| Tetrahydrofuran | | 70mL | |

DMAP was added to the THF solution of the alcohol at RT. The mixture was set to reflux (53°C) and propionic anhydride (1.3eq) was slowly added at this temperature. After 23h, 80% alcohol conversion was observed by GC. The reaction was cooled down to room temperature and slowly quenched with 50mL of water. 50mL of ethyl acetate were then added. The organic phase was separated and washed with NaHCO₃ and brine solution. The organic extracts were combined and dried with sodium sulfate. After evaporating the solvent at reduced pressure, 25.3g of crude product that contained ca 60% of the propionate according to the GC (area %) were obtained. The product was purified by column chromatography. The corresponding propionate was obtained with a purity > 95% (GC area %). The identity of the product was confirmed by NMR. 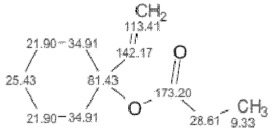 1-Vinyl-cyclohexyl-propanoate: ¹³C NMR (125 MHz, CDCl₃): δ = 9.33, 21.90, 25.43, 28.61, 34.91, 81.43, 113.41, 142.17, 173.20

### 1.7 Preparation of 1-ethynyl-cyclopentane-1-ol

In a 5 I autoclave, 308.5 g (5.5 mol) sodium methoxide is suspended in 1500 ml tetra-hydrofurane and purged with nitrogen. The autoclave is pressurized with 2 bar nitrogen and 18 bar acetylene at 10°C. 5 mol of cyclopentanone is continuously added over 16 h at 10°C, while keeping the pressure at 20 bar. The mixture is stirred for additional 5 h at 0°C before releasing the pressure. 500 ml water is carefully added while keeping the temperature below 30°C. The organic phase is separated, the pH adjusted to pH7 with phosphoric acid and the so obtained crude product is fractionated distilled to obtain the pure 1-ethynyl-cyclopentane-1-ol.

### 1.8 Preparation of 1-ethyl-cyclopentane-1-ol

A Steel-Autoclave was charged with 25 g of the 1-ethynyl-cyclopentane-1-ol, prepared according to example 1.7, and 50 ml of methanol. 2 g of catalyst (5% Pd/C) were added and the hydrogenation was performed at 30 bar hydrogen pressure and 80 °C. After complete conversion (check by GC), the reaction was stopped, the catalyst was filtered over celite and the solvent was evaporated. The crude product was directly used in the following step. The crude product, i.e. 1-ethyl-cyclopentane-1-ol, was directly applied in the next step without further purification.

### 1.9 Preparation of 1-ethyl-cyclopentyl-acetate

| **Compound** | **MW** | **Mass/Volume** | **Moles** |
|---|---|---|---|
| 1-Ethyl-cyclopentane-1-ol | 114.19 | 15g | 0.131 |
| Acetic anhydride | 102.09 | 16.93g | 0.158 |
| N,N'-dimethylaminopyridine | 122.17 | 0.481g | 0.004 |
| Tetrahydrofuran | | 50mL | |

DMAP was added to the THF solution of the alcohol at RT. The mixture was set to reflux (53°C) and acetic anhydride (1.2eq) was slowly added at this temperature. After 71h, 99% alcohol conversion was observed by GC. The reaction was cooled down to room temperature and slowly quenched with 50mL of water. 50mL of ethyl acetate were then added. The organic phase was separated and washed with NaHCO₃ and brine solution. The organic extracts were combined and dried with sodium sulfate. After evaporating the solvent at reduced pressure, 19.6g of crude product that contained ca. 97% of the acetate according to the GC (area %) were obtained. The product was purified by distillation. The corresponding acetate was obtained with a purity of 99% (GC area %). The identity of the product was confirmed by NMR. 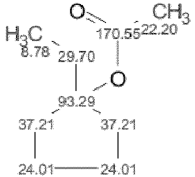 1-Ethyl-cyclopentyl-acetate: ¹³C NMR (125 MHz, CDCl₃): δ = 8.78, 22.20, 24.01, 29.70, 37.21, 93.29, 170.55

### 1.10 Preparation of 1-methoxy-1-ethyl-cyclohexane

| **Compound** | **MW** | **Mass/Volume** | **Moles** |
|---|---|---|---|
| 1-Ethyl-cyclohexane-1-ol | 128.21 | 10g | 0.078 |
| Methyl Iodide | 141.94 | 14.4g | 0.101 |
| Sodium hydride | 23.99 | 2.4g | 0.101 |
| THF | | 105mL | |

To a dispersion of sodium hydride (1.3eq) in 75mL of THF a solution of 1-ethyl-cyclohexane-1-ol in 30mL of THF was slowly added at 0°C. The mixture was stirred 30min at 0°C. After this time 1.3 eq of methyl iodide were slowly added at RT. After the addition, the mixture was stirred at 40°C for 4h. After this time the reaction mixture was cooled down to 0°C and the addition of 0.5eq of NaH followed by 0.5eq of methyl iodide was repeated. The mixture was then stirred for 18h at 40°C. At this point, the reaction was slowly quenched with 50mL of water. The organic phase was extracted with 3 times 50mL MTBE. The organic extracts were combined and washed with 50mL of NH₃ solution and with 50mL brine solution. The organic extracts were combined and dried with sodium sulfate. After evaporating the solvent at reduced pressure 9.4g of crude product were obtained with 99% of the methyl ether derivative according to the GC (area-%). The product was purified by distillation. The corresponding ether was obtained with a purity > 99%. The identity of the product was confirmed by NMR. 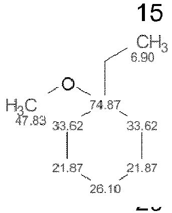 1-methoxy-1-ethyl-cyclohexane: ¹³C NMR (125 MHz, CDCl₃): δ = 6.90, 21.87, 26.10, 28.47, 33.62, 47.83, 74.87

### 1.11 Preparation of 1-ethoxy-1-ethyl-cyclohexane

| **Compound** | **MW** | **Mass/Volume** | **Moles** |
|---|---|---|---|
| 1-Ethyl-cyclohexane-1-ol | 128.21 | 7.5g | 0.058 |
| Diethyl sulfate | 154.19 | 6.3g | 0.041 |
| Sodium hydride | 23.99 | 1.8g | 0.076 |
| Toluene | | 70mL | |

To a dispersion of sodium hydride (1,3eq) in 50mL of toluene a solution of ethyl cyclohexanol in 20mL of toluene was slowly added at 0°C. The mixture was stirred 30min at 0°C. After this time the reaction vessel was heated to 87°C and stirred for 2h. 0,7eq of diethylsulfate were then slowly added at this temperature. The reaction was stirred for 3h at 87°C, 16h at RT and 5h more at 87°C. After this time the reaction mixture was cooled down and slowly quenched with 50mL of water. The organic phase extracts were combined and dried with sodium sulfate. After evaporating the solvent at reduced pressure 6g of crude product were obtained with 77% of the ethyl ether derivative according to the GC (area %). The product was purified by column chromatography. The corresponding ether was obtained with a purity of 99%. The identity of the product was confirmed by NMR. 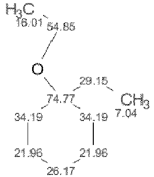 1-Ethoxy-1-ethyl-cyclohexane: ¹³C NMR (125 MHz, CDCl₃): δ = 7.04, 16.01, 21.96, 26.17, 29.15, 34.19, 54.85, 74.77

### 1.12 Preparation of 1-methoxy-1-vinyl-cyclohexane

| **Compound** | **MW** | **Mass/Volume** | **Moles** |
|---|---|---|---|
| 1-Vinyl-cyclohexane-1-ol | 126.20 | 10g | 0.079 |
| Methyl iodide | 141.94 | 14.6g | 0.103 |
| Sodium hydride | 23.99 | 2.5g | 0.103 |
| Tetrahydrofuran | | 105mL | |

To a dispersion of sodium hydride (1.3eq) in 75mL of THF a solution of the alcohol in 30mL of THF was slowly added at 0°C. The mixture was stirred 30min at 0°C. After this time 1.3 eq of methyl iodide were slowly added at RT. After the addition, the mixture was stirred at 40°C for 22h. After this time the reaction mixture was cooled down to 0°C and the addition of 0.5eq of NaH followed by 0.5eq of methyl iodide was repeated. The mixture was then stirred for 5h at 40°C. At this point, the reaction was slowly quenched with 50mL of water. The organic phase was extracted with 3 times 50mL MTBE. The organic extracts were combined and washed with 50mL of NH₃ solution and with 50mL brine solution. The organic extracts were combined and dried with sodium sulfate. After evaporating the solvent at reduced pressure 13.4g of crude product were obtained with 99% of the methyl ether according to the GC (area %). The product was purified by distillation. The corresponding methyl ethers was obtained with a purity > 99% (GC area %). The identity of the product was confirmed by NMR. 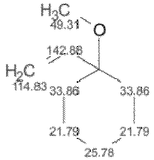 1-methoxy-1-vinyl-cyclohexane: ¹³C NMR (125 MHz, CDCl₃): δ = 21.79, 25.78, 33.86, 49.31, 75.70, 114.83, 142.88

### 1.13 Preparation of 1-ethoxy-1-vinyl-cyclohexane

| **Compound** | **MW** | **Mass/Volume** | **Moles** |
|---|---|---|---|
| 1-Vinyl-cyclohexane-1-ol | 126.20 | 7g | 0.055 |
| Ethyl iodide | 155,.97 | 11.2g | 0.072 |
| Sodium hydride | 23.99 | 1.7g | 0.072 |
| Tetrahydrofuran | | 105mL | |

To a dispersion of sodium hydride (1.3eq) in 75mL of THF a solution of the alcohol in 30mL of THF was slowly added at 0°C. The mixture was stirred 30min at 0°C. After this time 1.3 eq of ethyl iodide were slowly added at RT. After the addition, the mixture was stirred at 50°C for 70h. After this time the reaction mixture was cooled down to 0°C and the addition of 0.5eq of NaH followed by 0,5eq of ethyl iodide was repeated. The mixture was then stirred for 23h at 50°C. At this point, the reaction was slowly quenched with 50mL of water. The organic phase was extracted with 3 times 50mL MTBE. The organic extracts were combined and washed with 50mL of NH₃ solution and with 50mL brine solution. The organic extracts were combined and dried with sodium sulfate. After evaporating the solvent at reduced pressure 6.1g of crude product were obtained with 32% of the ethyl ether according to the GC (area %). The product was purified by column chromatography. The corresponding ethyl ether was obtained with a purity > 95% (GC area %). The identity of the product was confirmed by NMR. 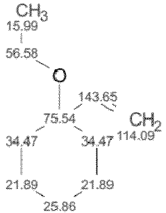 1-ethoxy-1-vinyl-cyclohexane: ¹³C NMR (125 MHz, CDCl₃): δ = 15.99, 21.89, 25.86, 34.47, 56.58, 75.54, 114.09, 143.65

### 1.14 Preparation of 1-ethyl-1-methoxy-cyclopentane

| **Compound** | **MW** | **Mass/Volume** | **Moles** |
|---|---|---|---|
| Ethyl-cyclopentane-1-ol | 114.19 | 10g | 0.088 |
| Methyl Iodide | 141.94 | 16.2g | 0.114 |
| Sodium hydride | 23.99 | 2.7g | 0.114 |
| THF | | 105mL | |

To a dispersion of sodium hydride (1.3eq) in 75mL of THF a solution of ethyl-cyclopentanol in 30mL of THF was slowly added at 0°C. The mixture was stirred 30min at 0°C. After this time 1.3 eq of methyl iodide were slowly added at RT. After the addition, the mixture was stirred at 40°C for 4h. After this time the reaction mixture was cooled down to 0°C and the addition of 0.25eq of NaH followed by 0.25eq of methyl iodide was repeated. The mixture was then stirred for 18h at 40°C. At this point, the reaction was slowly quenched with 50mL of water. The organic phase was extracted with 3 times 50mL MTBE. The organic extracts were combined and washed with 50mL of NH₃ solution and with 50mL brine solution. The organic extracts were combined and dried with sodium sulfate. After evaporating the solvent at reduced pressure 8.9g of crude product were obtained with 99% of the methyl ether derivative according to the GC (area %). The product was purified by distillation. The corresponding ether was obtained with a purity >99%. The identity of the product was confirmed by NMR. 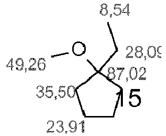 1-Ethyl-1-methoxy-cyclopentane: ¹³C NMR (125 MHz, CDCl₃): δ = 8.54, 23.91, 28.10, 35.50, 49.26, 87.02.

### 2. Olfactory assessment:

In order to test the quality and intensity of the odor of the compounds (I) of the present invention, scent strip tests were performed.

For this purpose, strips of absorbent paper were dipped into solution containing 1 to 10 % by weight solution of the compound (I) to be tested in ethanol. After evaporation of the solvent (about 30 sec.) the scent impression was olfactively evaluated by a trained perfumer.

The results of the scent test are summarized in table 1.

**Table 1: Results of the scent tests.**

| Example no. | Compound | Description |
|---|---|---|
| 1.4 | 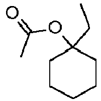 | Sweet, Damascone, Dry, Hay, Fresh |
| 1.5 | 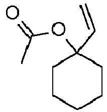 | Fresh, Raspberry, Sweet, Woody, Etheral |
| 1.6 | 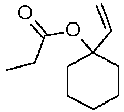 | Plum, Powdery, Chamomile, Raisin |
| 1.9 | 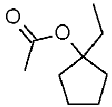 | Dried fruit, Damascone, Sweet, Woody |
| 1.10 | 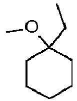 | Eucalyptus leaf, Ethereal, Pine needle, Green |
| 1.11 | 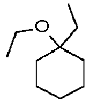 | Campher, Rosemary, Sweet, Fruity |
| 1.12 | 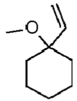 | Minty, Cool, Sweet, Floral; slightly cooling in the mouth, provides a clean mouth feeling |
| 1.13 | 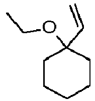 | Eukalyptus Leave |
| 1.14 | 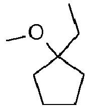 | Sweet, grapefruit |

### Advantageous perfume components

The compounds of examples 1.4, 1.5, 1.6, 1.9, 1.10, 1.11, 1.12, 1.13 and 1.14 were formulated in the perfume compositions according to tables 3 and 4.

**Table 3: Perfume oil compositions 1A and 1B**

| | **1A** | **1B** |
|---|---|---|
| Benzoe Siam 20% | 711 | 711 |
| Rosewood Oil brasilian | 85 | 85 |
| Copaivabalm rect. | 9 | 9 |
| Linalyl-benzoate | 31 | 31 |
| 3-cis-Hexenyl-salicylate | 21 | 21 |
| Geranyl-acetate | 47 | 47 |
| Ethyl-benzoate | 12 | 12 |
| Cinnamyl-acetate | 2 | 2 |
| Benzyl-acetate | 71 | 71 |
| Methyl-anthranilate 10% | 5 | 5 |
| Bayoil St. Thomas 10% | 5 | 5 |
| Compound mixture of example 1.4 | 0 | 20 |
| | 1000 | 1020 |

**Table 4: Perfume oil compositions 2A and 2B**

| | **2A** | **2B** |
|---|---|---|
| Ethyl Caproate | 1 | 1 |
| Ethyl Acetate | 1 | 1 |
| Iso Amyl Butyrate | 1 | 1 |
| Maltol or Veltol | 1 | 1 |
| Geranyl Butyrate | 2 | 2 |
| Ethyl Vanilline 10% DPG | 2 | 2 |
| Cis 3 Hexenyl Acetate | 3 | 3 |
| Allyl Caproate | 3 | 3 |
| Verdural B 10% DPG | 3 | 3 |
| Oxyphenylon | 3 | 3 |
| Hexyl Butyrate | 4 | 4 |
| Ethyl Decadienoate 10% DPG | 4 | 4 |
| DM.B.C. Butyrate | 4 | 4 |
| Ethyl Maltol or Veltol Plus | 4 | 4 |
| Cyclaprop | 5 | 5 |
| Iso Amyl Acetate | 5 | 5 |
| Cis 3 Hexenol 10% DPG | 6 | 6 |
| D.M.B.C. Acetate | 7 | 7 |
| Aldehyde C 16 100% | 8 | 8 |
| Geranyl Propionate | 8 | 8 |
| Ethyl 2 Methyl Butyrate | 8 | 8 |
| Decalactone Gamma | 10 | 10 |
| Orange Bresil Oil | 10 | 10 |
| Ethyl Aceto Acetate | 10 | 10 |
| Linalool | 15 | 15 |
| Benzyl Acetate | 15 | 15 |
| Aldehyde C 14 100% | 20 | 20 |
| Citronellol | 25 | 25 |
| Linalyl Acetate | 30 | 30 |
| Geranyl Acetate | 35 | 35 |
| Vertenex | 45 | 45 |
| Citronellyl Acetate | 50 | 50 |
| Verdox | 54 | 54 |
| Galaxolide 50 DEP | 100 | 100 |
| Hexyl Acetate | 190 | 190 |
| Mono Propylene Glycol | 300 | 300 |
| Compound mixture of example 1.4 | 0 | 200 |
| | 1000 | 1200 |

Perfume oil composition 3 corresponds to perfume oil composition 1B, where the compound of example 1.4 is replaced by the same amount of the compound of example 1.5.
Perfume oil composition 4 corresponds to perfume oil composition 1B, where the compound of example 1.4 is replaced by the same amount of the compound of example 1.6.
Perfume oil composition 5 corresponds to perfume oil composition 1B, where the compound of example 1.4 is replaced by the same amount of the compound of example 1.9.
Perfume oil composition 6 corresponds to perfume oil composition 1B, where the compound of example 1.4 is replaced by the same amount of the compound of example 1.10.
Perfume oil composition 7 corresponds to perfume oil composition 1B, where the compound of example 1.4 is replaced by the same amount of the compound of example 1.11.
Perfume oil composition 8 corresponds to perfume oil composition 1B, where the compound of example 1.4 is replaced by the same amount of the compound of example 1.12.
Perfume oil composition 9 corresponds to perfume oil composition 1B, where the compound of example 1.4 is replaced by the same amount of the compound of example 1.13.
Perfume oil composition 10 corresponds to perfume oil composition 1B, where the compound of example 1.4 is replaced by the same amount of the compound of example 1.14.
Perfume oil composition 11 corresponds to perfume oil composition 2B, where the compound of example 1.4 is replaced by the same amount of the compound of example 1.5.
Perfume oil composition 12 corresponds to perfume oil composition 2B, where the compound of example 1.4 is replaced by the same amount of the compound of example 1.6.
Perfume oil composition 13 corresponds to perfume oil composition 2B, where the compound of example 1.4 is replaced by the same amount of the compound of example 1.9.
Perfume oil composition 14 corresponds to perfume oil composition 2B, where the compound of example 1.4 is replaced by the same amount of the compound of example 1.10.
Perfume oil composition 15 corresponds to perfume oil composition 2B, where the compound of example 1.4 is replaced by the same amount of the compound of example 1.11.
Perfume oil composition 16 corresponds to perfume oil composition 2B, where the compound of example 1.4 is replaced by the same amount of the compound of example 1.12.
Perfume oil composition 17 corresponds to perfume oil composition 2B, where the compound of example 1.4 is replaced by the same amount of the compound of example 1.13.
Perfume oil composition 18 corresponds to perfume oil composition 2B, where the compound of example 1.4 is replaced by the same amount of the compound of example 1.14.

The compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14 may be used in a broad range of fragrance applications, e.g. in any field of fine and functional perfumery, such as perfumes, household products, laundry products, body care products and cosmetics. The compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, can be employed in widely varying amounts, depending upon the specific application and on the nature and quantity of other odorant ingredients. The proportion is typically from 0.001 to 20 weight per cent of the application. In one embodiment, the compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, are employed in a fabric softener in an amount of from 0.001 to 0.05 weight per cent. In another embodiment, the compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, are used in fine perfumery in amounts of from 0.1 to 20 weight per cent, more preferably between 0.1 and 5 weight per cent. However, these values are given only by way of example, since the experienced perfumer may also achieve effects or may create novel accords with lower or higher concentrations.

The compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, may be employed into the fragrance application simply by directly mixing the fragrance composition with the fragrance application, or they may, in an earlier step be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the incorporation of the compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, as a fragrance ingredient, either by directly admixing the compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, to the application or by admixing a fragrance composition comprising the compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, which may then be mixed to a fragrance application, using conventional techniques and methods.

As used herein, "fragrance application" means any product, such as fine perfumery, e.g. perfume and Eau de Toilette; household products, e.g. detergents for dishwasher, surface cleaner; laundry products, e.g. softener, bleach, detergent; body care products, e.g. shampoo, shower gel; and cosmetics, e.g. deodorant, vanishing creme, comprising an odorant. This list of products is given by way of illustration and is not to be regarded as being in any way limiting.

The compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, may be used as part of the perfume in the above mentioned applications. The compound of formula (I) or the mixtures of two or more compounds of the general formula (I), or of a stereoisomer thereof or of a mixture of two or more stereoisomers thereof, in particular the compounds of examples 1.4, 1.5, 1.6, 1.9, 1.10, 1.11, 1.12, 1.13 or 1.14, may be used alone or as part of a perfume. The term "perfume" is used synonymously to "perfume oil" or "perfume (oil) composition".

In the following tables all amounts are given in weight-% (% b.w.). Conc. means concentration.

**Table 5: Deo pump spray 1**

| **Deo Pump Spray; PIT** | | |
|---|---|---|
| **Component** | **INCI** | **amount %** |
| EMULGADE® SE-PF | Glyceryl Stearate (and) Ceteareth-20 (and) Ceteareth-12 (and) Cetearyl Alcohol (and) Cetyl Palmitate | 3.9 |
| EUMULGIN® B 2 | Ceteareth-20 | 1.6 |
| CETIOL® OE | Dicaprylyl Ether | 5 |
| CETIOL® PGL | Hexyldecanol (and) Hexyldecyl Laurate | 2 |
| Irgasan DP 300 | Triclosan | 0.25 |
| HYDAGEN® DEO | Triethyl Citrate (and) BHT | 2.5 |
| Eumulgin® HRE 60 | PEG-60 Hydrogenated Castor Oil | 1.5 |
| Perfume oil composition 1B | Perfume | 0.5 |
| Water, de ionized | Aqua | ad 100 |
| | | |
| Preservative | | q.s. |
| Viscosity mPas | < 100 | |

Deo pump spray 2 corresponds to deo pump spray 1, where the perfume oil composition 1 B is replaced by the same amount of the perfume oil composition 2B.
Deo pump spray 3 corresponds to deo pump spray 1, where the perfume oil composition 1B is replaced by the same amount of the perfume oil composition 3.
Deo pump spray 4 corresponds to deo pump spray 1, where the perfume oil composition 1B is replaced by the same amount of the perfume oil composition 4.
Deo pump spray 5 corresponds to deo pump spray 1, where the perfume oil composition 1B is replaced by the same amount of the perfume oil composition 5.
Deo pump spray 6 corresponds to deo pump spray 1, where the perfume oil composition 1B is replaced by the same amount of the perfume oil composition 6.
Deo pump spray 7 corresponds to deo pump spray 1, where the perfume oil composition 1B is replaced by the same amount of the perfume oil composition 7.
Deo pump spray 8 corresponds to deo pump spray 1, where the perfume oil composition 1B is replaced by the same amount of the perfume oil composition 8.
Deo pump spray 9 corresponds to deo pump spray 1, where the perfume oil composition 1B is replaced by the same amount of the perfume oil composition 9.
Deo pump spray 10 corresponds to deo pump spray 1, where the perfume oil composition 1B is replaced by the same amount of the perfume oil composition 10.
Deo pump spray 11 corresponds to deo pump spray 1, where the perfume oil composition 1B is replaced by the same amount of the perfume oil composition 11.
Deo pump spray 12 corresponds to deo pump spray 1, where the perfume oil composition 1B is replaced by the same amount of the perfume oil composition 12.
Deo pump spray 13 corresponds to deo pump spray 1, where the perfume oil composition 1B is replaced by the same amount of the perfume oil composition 13.
Deo pump spray 14 corresponds to deo pump spray 1, where the perfume oil composition 1B is replaced by the same amount of the perfume oil composition 14.
Deo pump spray 15 corresponds to deo pump spray 1, where the perfume oil composition 1B is replaced by the same amount of the perfume oil composition 15.
Deo pump spray 16 corresponds to deo pump spray 1, where the perfume oil composition 1B is replaced by the same amount of the perfume oil composition 16.
Deo pump spray 17 corresponds to deo pump spray 1, where the perfume oil composition 1B is replaced by the same amount of the perfume oil composition 17.
Deo pump spray 18 corresponds to deo pump spray 1, where the perfume oil composition 1B is replaced by the same amount of the perfume oil composition 18.

**Table 6: Deo pump spray 19**

| **Deo Pump Spray** | | |
|---|---|---|
| **Component** | **INCI** | **amount %** |
| Eumulgin® HRE 60 | PEG-60 Hydrogenated Castor Oil | 2 |
| Farnesol | Deo ingredient | 0.2 |
| HYDAGEN® DCMF | Chitosan | 0.1 |
| glycolic acid (Fa. Merck) | glycolic acid | 0.04 |
| Water, deionized | | ad 100 |
| Perfume oil composition 1B | Perfume | 0.5 |
| pH value | 4 | |

Deo pump spray 20 corresponds to deo pump spray 17, where the perfume oil composition 1B is replaced by the same amount of the perfume oil composition 2B.
Deo pump spray 21 corresponds to deo pump spray 17, where the perfume oil composition 1B is replaced by the same amount of the perfume oil composition 3.
Deo pump spray 22 corresponds to deo pump spray 17, where the perfume oil composition 1B is replaced by the same amount of the perfume oil composition 4.
Deo pump spray 23 corresponds to deo pump spray 17, where the perfume oil composition 1B is replaced by the same amount of the perfume oil composition 5.
Deo pump spray 24 corresponds to deo pump spray 17, where the perfume oil composition 1B is replaced by the same amount of the perfume oil composition 6.
Deo pump spray 25 corresponds to deo pump spray 17, where the perfume oil composition 1B is replaced by the same amount of the perfume oil composition 7.
Deo pump spray 26 corresponds to deo pump spray 17, where the perfume oil composition 1B is replaced by the same amount of the perfume oil composition 8.
Deo pump spray 27 corresponds to deo pump spray 17, where the perfume oil composition 1B is replaced by the same amount of the perfume oil composition 9.
Deo pump spray 28 corresponds to deo pump spray 17, where the perfume oil composition 1B is replaced by the same amount of the perfume oil composition 10.
Deo pump spray 29 corresponds to deo pump spray 17, where the perfume oil composition 1B is replaced by the same amount of the perfume oil composition 11.
Deo pump spray 30 corresponds to deo pump spray 17, where the perfume oil composition 1B is replaced by the same amount of the perfume oil composition 12.
Deo pump spray 31 corresponds to deo pump spray 17, where the perfume oil composition 1B is replaced by the same amount of the perfume oil composition 13.
Deo pump spray 32 corresponds to deo pump spray 17, where the perfume oil composition 1B is replaced by the same amount of the perfume oil composition 14.
Deo pump spray 33 corresponds to deo pump spray 17, where the perfume oil composition 1B is replaced by the same amount of the perfume oil composition 15.
Deo pump spray 34 corresponds to deo pump spray 17, where the perfume oil composition 1B is replaced by the same amount of the perfume oil composition 16.
Deo pump spray 35 corresponds to deo pump spray 17, where the perfume oil composition 1B is replaced by the same amount of the perfume oil composition 17.
Deo pump spray 36 corresponds to deo pump spray 17, where the perfume oil composition 1B is replaced by the same amount of the perfume oil composition 18.

**Table 7: Clean hair conditioner 1**

| **Clear Hair Conditioner** | | |
|---|---|---|
| **Component** | **INCI** | **amount %** |
| DEHYQUART® L 80 | Dicocoylethyl Hydroxyethylmonium Methosulfate (and) Propylene Glycol | 1.0 |
| Propylene Glycol | solvent | 10.0 |
| Eumulgin® HRE 60 | PEG-60 Hydrogenated Castor Oil | 1.0 |
| LAMESOFT® PO 65 | Coco-Glucoside (and) Glyceryl Oleate | 1.0 |
| GLUADIN® W 40 | Hydrolyzed Wheat Gluten Hydrolyzed Wheat Protein | 0.5 |
| Perfume oil composition 1B | Perfume | 0.02 |
| Preservative | | q.s. |
| HYDAGEN® HCMF | Chitosan | 10.0 (sol.1%) |
| Water, deionized | | up to 100.0 |
| | | |
| pH-value | 3.5 - 4.0 | |

Clean hair conditioner 2 corresponds to clean hair conditioner 1, where the perfume oil composition 1B is replaced by the same amount of the perfume oil composition 2B. Clean hair conditioner 3 corresponds to clean hair conditioner 1, where the perfume oil composition 1B is replaced by the same amount of the perfume oil composition 3. Clean hair conditioner 4 corresponds to clean hair conditioner 1, where the perfume oil composition 1B is replaced by the same amount of the perfume oil composition 4. Clean hair conditioner 5 corresponds to clean hair conditioner 1, where the perfume oil composition 1B is replaced by the same amount of the perfume oil composition 5. Clean hair conditioner 6 corresponds to clean hair conditioner 1, where the perfume oil composition 1B is replaced by the same amount of the perfume oil composition 6. Clean hair conditioner 7 corresponds to clean hair conditioner 1, where the perfume oil composition 1B is replaced by the same amount of the perfume oil composition 7. Clean hair conditioner 8 corresponds to clean hair conditioner 1, where the perfume oil composition 1B is replaced by the same amount of the perfume oil composition 8. Clean hair conditioner 9 corresponds to clean hair conditioner 1, where the perfume oil composition 1B is replaced by the same amount of the perfume oil composition 9. Clean hair conditioner 10 corresponds to clean hair conditioner 1, where the perfume oil composition 1B is replaced by the same amount of the perfume oil composition 10. Clean hair conditioner 11 corresponds to clean hair conditioner 1, where the perfume oil composition 1B is replaced by the same amount of the perfume oil composition 11. Clean hair conditioner 12 corresponds to clean hair conditioner 1, where the perfume oil composition 1B is replaced by the same amount of the perfume oil composition 12. Clean hair conditioner 13 corresponds to clean hair conditioner 1, where the perfume oil composition 1B is replaced by the same amount of the perfume oil composition 13. Clean hair conditioner 14 corresponds to clean hair conditioner 1, where the perfume oil composition 1B is replaced by the same amount of the perfume oil composition 14. Clean hair conditioner 15 corresponds to clean hair conditioner 1, where the perfume oil composition 1B is replaced by the same amount of the perfume oil composition 15. Clean hair conditioner 16 corresponds to clean hair conditioner 1, where the perfume oil composition 1B is replaced by the same amount of the perfume oil composition 16. Clean hair conditioner 17 corresponds to clean hair conditioner 1, where the perfume oil composition 1B is replaced by the same amount of the perfume oil composition 17. Clean hair conditioner 18 corresponds to clean hair conditioner 1, where the perfume oil composition 1B is replaced by the same amount of the perfume oil composition 18.

**Table 8: Face wash gel 1**

| **Face Wash Gel** | | |
|---|---|---|
| **Component** | **INCI** | **amount %** |
| PLANTAPON® 611 L | Sodium Laureth Sulfate (and) Lauryl Glucoside (and) Cocamidopropyl Betaine | 20 |
| PLANTAPON® ACG 35 | Disodium Cocoyl Glutamate | 1 |
| LAMESOFT® PO 65 | Coco-Glucoside (and) Glyceryl Oleate | 2 |
| NaCl | Sodium Chloride | 1.7 |
| PLANTACARE® PS 10 | Sodium Laureth Sulfate (and) Lauryl Polyglucose | 0.3 |
| Perfume oil composition 1B | Perfume | 0.1 |
| Water | Aqua | QS |
| Preservative | | QS |
| Dye | | QS |
| pH 6 to 7 | | |

Face wash gel 2 corresponds to face wash gel 1, where the perfume oil composition 1B is replaced by the same amount of the perfume oil composition 2B.
Face wash gel 3 corresponds to face wash gel 1, where the perfume oil composition 1B is replaced by the same amount of the perfume oil composition 3.
Face wash gel 4 corresponds to face wash gel 1, where the perfume oil composition 1B is replaced by the same amount of the perfume oil composition 4.
Face wash gel 5 corresponds to face wash gel 1, where the perfume oil composition 1B is replaced by the same amount of the perfume oil composition 5.
Face wash gel 6 corresponds to face wash gel 1, where the perfume oil composition 1B is replaced by the same amount of the perfume oil composition 6.
Face wash gel 7 corresponds to face wash gel 1, where the perfume oil composition 1B is replaced by the same amount of the perfume oil composition 7.
Face wash gel 8 corresponds to face wash gel 1, where the perfume oil composition 1B is replaced by the same amount of the perfume oil composition 8.
Face wash gel 9 corresponds to face wash gel 1, where the perfume oil composition 1B is replaced by the same amount of the perfume oil composition 9.
Face wash gel 10 corresponds to face wash gel 1, where the perfume oil composition 1B is replaced by the same amount of the perfume oil composition 10.
Face wash gel 11 corresponds to face wash gel 1, where the perfume oil composition 1B is replaced by the same amount of the perfume oil composition 11.
Face wash gel 12 corresponds to face wash gel 1, where the perfume oil composition 1B is replaced by the same amount of the perfume oil composition 12.
Face wash gel 13 corresponds to face wash gel 1, where the perfume oil composition 1B is replaced by the same amount of the perfume oil composition 13.
Face wash gel 14 corresponds to face wash gel 1, where the perfume oil composition 1B is replaced by the same amount of the perfume oil composition 14.
Face wash gel 15 corresponds to face wash gel 1, where the perfume oil composition 1B is replaced by the same amount of the perfume oil composition 15.
Face wash gel 16 corresponds to face wash gel 1, where the perfume oil composition 1B is replaced by the same amount of the perfume oil composition 16.
Face wash gel 17 corresponds to face wash gel 1, where the perfume oil composition 1B is replaced by the same amount of the perfume oil composition 17.
Face wash gel 18 corresponds to face wash gel 1, where the perfume oil composition 1B is replaced by the same amount of the perfume oil composition 18.

**Table 9: Foam bath concentrate 1**

| **Foam Bath Concentrate** | | |
|---|---|---|
| **Component** | **INCI** | **amount %** |
| TEXAPON® K 14 S 70spec. | Sodium Myreth Sulfate | 25 |
| PLANTACARE® 2000 UP | Decyl Glucoside | 20 |
| DEHYTON® K | Cocamidopropyl Betaine | 20 |
| GLUADIN® WP | Hydrolyzed Wheat Gluten Hydrolyzed Wheat Protein | 1 |
| PLANTACARE® PS 10 | Sodium Laureth Sulfate (and) Lauryl Polyglucose | 5 |
| Water, deionized | | ad 100 |
| Citric Acid, 50 % | | 0.5 |
| Perfume oil composition 1B | perfume | 2.0 |
| pH value | 5.5 | |

Foam bath concentrate 2 corresponds to foam bath concentrate 1, where the perfume oil composition 1B is replaced by the same amount of the perfume oil composition 2B. Foam bath concentrate 3 corresponds to foam bath concentrate 1, where the perfume oil composition 1B is replaced by the same amount of the perfume oil composition 3. Foam bath concentrate 4 corresponds to foam bath concentrate 1, where the perfume oil composition 1B is replaced by the same amount of the perfume oil composition 4. Foam bath concentrate 5 corresponds to foam bath concentrate 1, where the perfume oil composition 1B is replaced by the same amount of the perfume oil composition 5. Foam bath concentrate 6 corresponds to foam bath concentrate 1, where the perfume oil composition 1B is replaced by the same amount of the perfume oil composition 6. Foam bath concentrate 7 corresponds to foam bath concentrate 1, where the perfume oil composition 1B is replaced by the same amount of the perfume oil composition 7. Foam bath concentrate 8 corresponds to foam bath concentrate 1, where the perfume oil composition 1B is replaced by the same amount of the perfume oil composition 8. Foam bath concentrate 9 corresponds to foam bath concentrate 1, where the perfume oil composition 1B is replaced by the same amount of the perfume oil composition 9. Foam bath concentrate 10 corresponds to foam bath concentrate 1, where the perfume oil composition 1B is replaced by the same amount of the perfume oil composition 10. Foam bath concentrate 11 corresponds to foam bath concentrate 1, where the perfume oil composition 1B is replaced by the same amount of the perfume oil composition 11. Foam bath concentrate 12 corresponds to foam bath concentrate 1, where the perfume oil composition 1B is replaced by the same amount of the perfume oil composition 12. Foam bath concentrate 13 corresponds to foam bath concentrate 1, where the perfume oil composition 1B is replaced by the same amount of the perfume oil composition 13. Foam bath concentrate 14 corresponds to foam bath concentrate 1, where the perfume oil composition 1B is replaced by the same amount of the perfume oil composition 14. Foam bath concentrate 15 corresponds to foam bath concentrate 1, where the perfume oil composition 1B is replaced by the same amount of the perfume oil composition 15. Foam bath concentrate 16 corresponds to foam bath concentrate 1, where the perfume oil composition 1B is replaced by the same amount of the perfume oil composition 16. Foam bath concentrate 17 corresponds to foam bath concentrate 1, where the perfume oil composition 1B is replaced by the same amount of the perfume oil composition 17. Foam bath concentrate 18 corresponds to foam bath concentrate 1, where the perfume oil composition 1B is replaced by the same amount of the perfume oil composition 18.

**Table 10: Hair gel 1**

| **Hair Gel** | | |
|---|---|---|
| **Component** | **INCI** | **amount %** |
| HYDAGEN® HCMF | Chitosan | 0.5 |
| Glycolic Acid (Fa. Merck) | Solvent | 0.2 |
| Water | | ad 100 |
| Jaguar HP 105 (2%swelling) | Thickener | 65 |
| Eumulgin® HRE 60 | PEG-60 Hydrogenated Castor Oil | 1.5 |
| Perfume oil composition 1B | Perfume | 0.1 |
| Ethanol | Evaporation agent | 7 |
| pH-value | 5.8 | |
| Viscosity (mPas), Brook.RVF, 23°C, sp.7, 10rpm = 20.000 | | |

Hair gel 2 corresponds to hair gel 1, where the perfume oil composition 1B is replaced by the same amount of the perfume oil composition 2B.
Hair gel 3 corresponds to hair gel 1, where the perfume oil composition 1B is replaced by the same amount of the perfume oil composition 3.
Hair gel 4 corresponds to hair gel 1, where the perfume oil composition 1B is replaced by the same amount of the perfume oil composition 4.
Hair gel 5 corresponds to hair gel 1, where the perfume oil composition 1B is replaced by the same amount of the perfume oil composition 5.
Hair gel 6 corresponds to hair gel 1, where the perfume oil composition 1B is replaced by the same amount of the perfume oil composition 6.
Hair gel 7 corresponds to hair gel 1, where the perfume oil composition 1B is replaced by the same amount of the perfume oil composition 7.
Hair gel 8 corresponds to hair gel 1, where the perfume oil composition 1B is replaced by the same amount of the perfume oil composition 8.
Hair gel 9 corresponds to hair gel 1, where the perfume oil composition 1B is replaced by the same amount of the perfume oil composition 9.
Hair gel 10 corresponds to hair gel 1, where the perfume oil composition 1B is replaced by the same amount of the perfume oil composition 10.
Hair gel 11 corresponds to hair gel 1, where the perfume oil composition 1B is replaced by the same amount of the perfume oil composition 11.
Hair gel 12 corresponds to hair gel 1, where the perfume oil composition 1B is replaced by the same amount of the perfume oil composition 12.
Hair gel 13 corresponds to hair gel 1, where the perfume oil composition 1B is replaced by the same amount of the perfume oil composition 13.
Hair gel 14 corresponds to hair gel 1, where the perfume oil composition 1B is replaced by the same amount of the perfume oil composition 14.
Hair gel 15 corresponds to hair gel 1, where the perfume oil composition 1B is replaced by the same amount of the perfume oil composition 15.
Hair gel 16 corresponds to hair gel 1, where the perfume oil composition 1B is replaced by the same amount of the perfume oil composition 16.
Hair gel 17 corresponds to hair gel 1, where the perfume oil composition 1B is replaced by the same amount of the perfume oil composition 17.
Hair gel 18 corresponds to hair gel 1, where the perfume oil composition 1B is replaced by the same amount of the perfume oil composition 18.

**Table 11: Self foaming bodywash 1**

| **Self foaming bodywash** | | |
|---|---|---|
| **Component** | **INCI** | **amount %** |
| TEXAPON® SB 3 KC | Disodium Laureth Sulfosuccinate | 16.5 |
| DEHYTON® K | Cocamidopropyl Betaine | 6.5 |
| PLANTACARE® 818 UP | Coco Glucoside | 7.5 |
| TEXAPON® NSO | Sodium laureth sulfate | 14.2 |
| LAMESOFT® PO 65 | Coco-Glucoside (and) Glyceryl Oleate | 5 |
| Dow Corning 193 | Dimethicole Copolyol | 1 |
| PLANTACARE® PS 10 | Sodium Laureth Sulfate (and) Lauryl Polyglucose | 0.5 |
| Perfume oil composition 1B | Perfume | 0.5 |
| Kathon CG | Methylchloroisothiazolinone (and) Methyl-isothiazolinone | 0.05 |
| Water, de-ionized | Aqua | ad 100 |
| Hexylen Glycol (Elf Atochem) | Humectant | 3 |
| UCARE Polymer JR 400 | Polyquaternium-10 | 0.5 |
| pH: 5.5 | | |
| Viscosity: > 100 mPas | | |

Self foaming bodywash 2 corresponds to self foaming bodywash 1, where the perfume oil composition 1B is replaced by the same amount of the perfume oil composition 2B.
Self foaming bodywash 3 corresponds to self foaming bodywash 1, where the perfume oil composition 1B is replaced by the same amount of the perfume oil composition 3.
Self foaming bodywash 4 corresponds to self foaming bodywash 1, where the perfume oil composition 1B is replaced by the same amount of the perfume oil composition 4.
Self foaming bodywash 5 corresponds to self foaming bodywash 1, where the perfume oil composition 1B is replaced by the same amount of the perfume oil composition 5.
Self foaming bodywash 6 corresponds to self foaming bodywash 1, where the perfume oil composition 1B is replaced by the same amount of the perfume oil composition 6.
Self foaming bodywash 7 corresponds to self foaming bodywash 1, where the perfume oil composition 1B is replaced by the same amount of the perfume oil composition 7.
Self foaming bodywash 8 corresponds to self foaming bodywash 1, where the perfume oil composition 1B is replaced by the same amount of the perfume oil composition 8.
Self foaming bodywash 9 corresponds to self foaming bodywash 1, where the perfume oil composition 1B is replaced by the same amount of the perfume oil composition 9.
Self foaming bodywash 10 corresponds to self foaming bodywash 1, where the perfume oil composition 1B is replaced by the same amount of the perfume oil composition 10.
Self foaming bodywash 11 corresponds to self foaming bodywash 1, where the perfume oil composition 1B is replaced by the same amount of the perfume oil composition 11.
Self foaming bodywash 12 corresponds to self foaming bodywash 1, where the perfume oil composition 1B is replaced by the same amount of the perfume oil composition 12.
Self foaming bodywash 13 corresponds to self foaming bodywash 1, where the perfume oil composition 1B is replaced by the same amount of the perfume oil composition 13.
Self foaming bodywash 14 corresponds to self foaming bodywash 1, where the perfume oil composition 1B is replaced by the same amount of the perfume oil composition 14.
Self foaming bodywash 15 corresponds to self foaming bodywash 1, where the perfume oil composition 1B is replaced by the same amount of the perfume oil composition 15.
Self foaming bodywash 16 corresponds to self foaming bodywash 1, where the perfume oil composition 1B is replaced by the same amount of the perfume oil composition 16.
Self foaming bodywash 17 corresponds to self foaming bodywash 1, where the perfume oil composition 1B is replaced by the same amount of the perfume oil composition 17.
Self foaming bodywash 18 corresponds to self foaming bodywash 1, where the perfume oil composition 1B is replaced by the same amount of the perfume oil composition 18.

**Table 12: Sprayable sun care emulsion 1**

| **Sprayable Sun Care Emulsion** | | |
|---|---|---|
| **Component** | **INCI** | **amount %** |
| EMULGADE® SE-PF | Glyceryl Stearate (and) Ceteareth-20 (and) Ceteareth-12 (and) Cetearyl Alcohol (and) Cetyl Palmitate | 7.8 |
| EUMULGIN® B 3 | Ceteareth-30 | 4.2 |
| CETIOL® CC | Dicaprylyl Carbonate | 5.0 |
| CETIOL® OE | Dicaprylyl Ether | 5.0 |
| Neo Heliopan BB (Haarmann&Reimer) | | 4.5 |
| Neo Heliopan AV (Haarmann&Reimer) | | 7.5 |
| Neo Heliopan 357 (Haarmann&Reimer) | | 2.0 |
| Water, de-ionized | | ad 100 |
| Perfume oil composition 1B | Perfume | 0.05 |
| Eumulgin® HRE 60 | PEG-60 Hydrogenated Castor Oil | 1.2 |
| pH | 5.5 | |
| Viscosity (mPas), RVF spindle 2, 20°C, 50 rpm < 100 | | |

Sprayable sun care emulsion 2 corresponds to sprayable sun care emulsion 1, where the perfume oil composition 1B is replaced by the same amount of the perfume oil composition 2B.
Sprayable sun care emulsion 3 corresponds to sprayable sun care emulsion 1, where the perfume oil composition 1B is replaced by the same amount of the perfume oil composition 3.
Sprayable sun care emulsion 4 corresponds to sprayable sun care emulsion 1, where the perfume oil composition 1B is replaced by the same amount of the perfume oil composition 4.
Sprayable sun care emulsion 5 corresponds to sprayable sun care emulsion 1, where the perfume oil composition 1B is replaced by the same amount of the perfume oil composition 5.
Sprayable sun care emulsion 6 corresponds to sprayable sun care emulsion 1, where the perfume oil composition 1B is replaced by the same amount of the perfume oil composition 6.
Sprayable sun care emulsion 7 corresponds to sprayable sun care emulsion 1, where the perfume oil composition 1B is replaced by the same amount of the perfume oil composition 7.
Sprayable sun care emulsion 8 corresponds to sprayable sun care emulsion 1, where the perfume oil composition 1B is replaced by the same amount of the perfume oil composition 8.
Sprayable sun care emulsion 9 corresponds to sprayable sun care emulsion 1, where the perfume oil composition 1B is replaced by the same amount of the perfume oil composition 9.
Sprayable sun care emulsion 10 corresponds to sprayable sun care emulsion 1, where the perfume oil composition 1B is replaced by the same amount of the perfume oil composition 10.
Sprayable sun care emulsion 11 corresponds to sprayable sun care emulsion 1, where the perfume oil composition 1B is replaced by the same amount of the perfume oil composition 11.
Sprayable sun care emulsion 12 corresponds to sprayable sun care emulsion 1, where the perfume oil composition 1B is replaced by the same amount of the perfume oil composition 12.
Sprayable sun care emulsion 13 corresponds to sprayable sun care emulsion 1, where the perfume oil composition 1B is replaced by the same amount of the perfume oil composition 13.
Sprayable sun care emulsion 14 corresponds to sprayable sun care emulsion 1, where the perfume oil composition 1B is replaced by the same amount of the perfume oil composition 14.
Sprayable sun care emulsion 15 corresponds to sprayable sun care emulsion 1, where the perfume oil composition 1B is replaced by the same amount of the perfume oil composition 15.
Sprayable sun care emulsion 16 corresponds to sprayable sun care emulsion 1, where the perfume oil composition 1B is replaced by the same amount of the perfume oil composition 16.
Sprayable sun care emulsion 17 corresponds to sprayable sun care emulsion 1, where the perfume oil composition 1B is replaced by the same amount of the perfume oil composition 17.
Sprayable sun care emulsion 18 corresponds to sprayable sun care emulsion 1, where the perfume oil composition 1B is replaced by the same amount of the perfume oil composition 18.

**Table 13: Sprayable sun protection emulsion 1**

| **Sprayable Sun Protection Emulsion** | | |
|---|---|---|
| **Component** | **INCI** | **amount %** |
| EMULGADE® SE-PF | Glyceryl Stearate (and) Ceteareth-20 (and) | 9.0 |
| | Ceteareth-12 (and) Cetearyl Alcohol (and) Cetyl Palmitate Ceteareth-33 | |
| Eumulgin B3 | | 6.0 |
| CETIOL® CC | Dicaprylyl Carbonate | 4.0 |
| Eusolex HMS | Homosalate | 7.0 |
| Parsol MCX (Givaudan Roure) | Ethylhexyl Methoxycinnamate | 7.5 |
| Parsol 1789 | Butyl Methoxydibenzoylmethane | 3.0 |
| Neo Heliopan OS | Ethylhexyl Salicylate | 5.0 |
| Preservative | | q.s. |
| Water, de-ionized | | ad 100 |
| Perfume oil composition 1B | Perfume | 0.2 |
| Eumulgin® HRE 60 | PEG-60 Hydrogenated Castor Oil | 1.2 |
| pH-value | 6.2 | |

Sprayable sun protection emulsion 2 corresponds to sprayable sun protection emulsion 1, where the perfume oil composition 1B is replaced by the same amount of the perfume oil composition 2B.
Sprayable sun protection emulsion 3 corresponds to sprayable sun protection emulsion 1, where the perfume oil composition 1B is replaced by the same amount of the perfume oil composition 3.
Sprayable sun protection emulsion 4 corresponds to sprayable sun protection emulsion 1, where the perfume oil composition 1B is replaced by the same amount of the perfume oil composition 4.
Sprayable sun protection emulsion 5 corresponds to sprayable sun protection emulsion 1, where the perfume oil composition 1B is replaced by the same amount of the perfume oil composition 5.
Sprayable sun protection emulsion 6 corresponds to sprayable sun protection emulsion 1, where the perfume oil composition 1B is replaced by the same amount of the perfume oil composition 6.
Sprayable sun protection emulsion 7 corresponds to sprayable sun protection emulsion 1, where the perfume oil composition 1B is replaced by the same amount of the perfume oil composition 7.
Sprayable sun protection emulsion 8 corresponds to sprayable sun protection emulsion 1, where the perfume oil composition 1B is replaced by the same amount of the perfume oil composition 8.
Sprayable sun protection emulsion 9 corresponds to sprayable sun protection emulsion 1, where the perfume oil composition 1B is replaced by the same amount of the perfume oil composition 9.
Sprayable sun protection emulsion 10 corresponds to sprayable sun protection emulsion 1, where the perfume oil composition 1B is replaced by the same amount of the perfume oil composition 10.
Sprayable sun protection emulsion 11 corresponds to sprayable sun protection emulsion 1, where the perfume oil composition 1B is replaced by the same amount of the perfume oil composition 11.
Sprayable sun protection emulsion 12 corresponds to sprayable sun protection emulsion 1, where the perfume oil composition 1B is replaced by the same amount of the perfume oil composition 12.
Sprayable sun protection emulsion 13 corresponds to sprayable sun protection emulsion 1, where the perfume oil composition 1B is replaced by the same amount of the perfume oil composition 13.
Sprayable sun protection emulsion 14 corresponds to sprayable sun protection emulsion 1, where the perfume oil composition 1 B is replaced by the same amount of the perfume oil composition 14.
Sprayable sun protection emulsion 15 corresponds to sprayable sun protection emulsion 1, where the perfume oil composition 1B is replaced by the same amount of the perfume oil composition 15.
Sprayable sun protection emulsion 16 corresponds to sprayable sun protection emulsion 1, where the perfume oil composition 1B is replaced by the same amount of the perfume oil composition 16.
Sprayable sun protection emulsion 17 corresponds to sprayable sun protection emulsion 1, where the perfume oil composition 1B is replaced by the same amount of the perfume oil composition 17.
Sprayable sun protection emulsion 18 corresponds to sprayable sun protection emulsion 1, where the perfume oil composition 1 B is replaced by the same amount of the perfume oil composition 18.

**Table 14: Emollient facial gel 1**

| **Emollient facial Gel** | | |
|---|---|---|
| **Component** | **INCI** | **amount %** |
| Water | | Ad 100 |
| HISPAGEL® 200 | Glycerin (and) Glyceryl Polyacrylate | 10 |
| CET-OE-Primasponge®BLUE | | 0.5 |
| PLANTACARE® PS 10 | Sodium Laureth Sulfate (and) Lauryl Polyglucose | 0.6 |
| Perfume oil composition 1B | | 0.1 |

Emollient facial gel 2 corresponds to emollient facial gel 1, where the perfume oil composition 1 B is replaced by the same amount of the perfume oil composition 2B. Emollient facial gel 3 corresponds to emollient facial gel 1, where the perfume oil composition 1 B is replaced by the same amount of the perfume oil composition 3. Emollient facial gel 4 corresponds to emollient facial gel 1, where the perfume oil composition 1 B is replaced by the same amount of the perfume oil composition 4. Emollient facial gel 5 corresponds to emollient facial gel 1, where the perfume oil composition 1B is replaced by the same amount of the perfume oil composition 5. Emollient facial gel 6 corresponds to emollient facial gel 1, where the perfume oil composition 1B is replaced by the same amount of the perfume oil composition 6. Emollient facial gel 7 corresponds to emollient facial gel 1, where the perfume oil composition 1B is replaced by the same amount of the perfume oil composition 7. Emollient facial gel 8 corresponds to emollient facial gel 1, where the perfume oil composition 1B is replaced by the same amount of the perfume oil composition 8. Emollient facial gel 9 corresponds to emollient facial gel 1, where the perfume oil composition 1B is replaced by the same amount of the perfume oil composition 9. Emollient facial gel 10 corresponds to emollient facial gel 1, where the perfume oil composition 1B is replaced by the same amount of the perfume oil composition 10. Emollient facial gel 11 corresponds to emollient facial gel 1, where the perfume oil composition 1B is replaced by the same amount of the perfume oil composition 11. Emollient facial gel 12 corresponds to emollient facial gel 1, where the perfume oil composition 1B is replaced by the same amount of the perfume oil composition 12. Emollient facial gel 13 corresponds to emollient facial gel 1, where the perfume oil composition 1B is replaced by the same amount of the perfume oil composition 13. Emollient facial gel 14 corresponds to emollient facial gel 1, where the perfume oil composition 1B is replaced by the same amount of the perfume oil composition 14. Emollient facial gel 15 corresponds to emollient facial gel 1, where the perfume oil composition 1B is replaced by the same amount of the perfume oil composition 15. Emollient facial gel 16 corresponds to emollient facial gel 1, where the perfume oil composition 1B is replaced by the same amount of the perfume oil composition 16. Emollient facial gel 17 corresponds to emollient facial gel 1, where the perfume oil composition 1B is replaced by the same amount of the perfume oil composition 17. Emollient facial gel 18 corresponds to emollient facial gel 1, where the perfume oil composition 1B is replaced by the same amount of the perfume oil composition 18.

**Table 15: 2-phases oil foam bath 1**

| **2-Phases-Oilfoambath** | | |
|---|---|---|
| **Component** | **INCI** | **amount % b.w.** |
| PLANTACARE® PS 10 | Sodium Laureth Sulfate (and) Lauryl Polyglucose | 10 |
| Paraffin oil | (low viscosity) | 20 |
| TEXAPON® N 70 | Sodium Laureth sulfate | 13 |
| DEHYTON® PK 45 | Cocamidopropyl Betaine | 8 |
| Perfume oil composition 1B | Perfume | 2 |
| Glycerin (86%) | | 5 |
| Preservative | | q.s. |
| Water, de-ionized | | ad 100.0 |
| pH-value | 5.5 | |

2-phases oil foam bath 2 corresponds to 2-phases oil foam bath 1, where the perfume oil composition 1B is replaced by the same amount of the perfume oil composition 2B. 2-phases oil foam bath 3 corresponds to 2-phases oil foam bath 1, where the perfume oil composition 1B is replaced by the same amount of the perfume oil composition 3. 2-phases oil foam bath 4 corresponds to 2-phases oil foam bath 1, where the perfume oil composition 1B is replaced by the same amount of the perfume oil composition 4. 2-phases oil foam bath 5 corresponds to 2-phases oil foam bath 1, where the perfume oil composition 1B is replaced by the same amount of the perfume oil composition 5. 2-phases oil foam bath 6 corresponds to 2-phases oil foam bath 1, where the perfume oil composition 1B is replaced by the same amount of the perfume oil composition 6. 2-phases oil foam bath 7 corresponds to 2-phases oil foam bath 1, where the perfume oil composition 1B is replaced by the same amount of the perfume oil composition 7. 2-phases oil foam bath 8 corresponds to 2-phases oil foam bath 1, where the perfume oil composition 1B is replaced by the same amount of the perfume oil composition 8. 2-phases oil foam bath 9 corresponds to 2-phases oil foam bath 1, where the perfume oil composition 1B is replaced by the same amount of the perfume oil composition 9. 2-phases oil foam bath 10 corresponds to 2-phases oil foam bath 1, where the perfume oil composition 1B is replaced by the same amount of the perfume oil composition 10. 2-phases oil foam bath 11 corresponds to 2-phases oil foam bath 1, where the perfume oil composition 1B is replaced by the same amount of the perfume oil composition 11. 2-phases oil foam bath 12 corresponds to 2-phases oil foam bath 1, where the perfume oil composition 1B is replaced by the same amount of the perfume oil composition 12. 2-phases oil foam bath 13 corresponds to 2-phases oil foam bath 1, where the perfume oil composition 1B is replaced by the same amount of the perfume oil composition 13. 2-phases oil foam bath 14 corresponds to 2-phases oil foam bath 1, where the perfume oil composition 1B is replaced by the same amount of the perfume oil composition 14. 2-phases oil foam bath 15 corresponds to 2-phases oil foam bath 1, where the perfume oil composition 1B is replaced by the same amount of the perfume oil composition 15. 2-phases oil foam bath 16 corresponds to 2-phases oil foam bath 1, where the perfume oil composition 1B is replaced by the same amount of the perfume oil composition 16. 2-phases oil foam bath 17 corresponds to 2-phases oil foam bath 1, where the perfume oil composition 1B is replaced by the same amount of the perfume oil composition 17. 2-phases oil foam bath 18 corresponds to 2-phases oil foam bath 1, where the perfume oil composition 1B is replaced by the same amount of the perfume oil composition 18.

**Table 16: Shampoos 1, 2, 3 and 4**

| **Shampoo** | | | **1** | **2** | **3** | **4** |
|---|---|---|---|---|---|---|
| **Component** | **INCI** | **Conc.** | **% b.w**. | | | |
| Texapon N70 | Sodium Laureth Sulfate | 70% | 12.9 | 12.9 | 14.3 | 14.3 |
| Dehyton PK45 | Cocamidopropyl Betaine | 44 - 46% | 5.4 | 5.4 | 5.4 | 5.4 |
| Plantacare 818UP | Coco-Glucoside | 51 - 53% | 2.0 | 2.0 | --- | --- |
| PLANTACARE® PS 10 | Sodium Laureth Sulfate (and) Lauryl Polyglucose | 33% | 6.0 | 18.0 | 21.0 | 30.0 |
| Copherol 1250 C | Tocopheryl Acetate | 100% | 1.0 | --- | --- | --- |
| Cetiol J 600 | Oleyl Erucate | 100% | --- | 0.7 | --- | 1.0 |
| Almond Oil | Prunus Amygdalus Dulcis (Sweet Almond) Oil | 100% | --- | --- | 0.3 | --- |
| Jaguar C162 | Hydroxypropyl Guar Hydroxypropy-Itrimonium Chloride | 100% | 0.2 | 0.2 | --- | --- |
| Jaguar Excel | Guar Hydroxypropyltrimonium Chloride | 100% | --- | --- | 0.25 | 0.25 |
| | PEG/PPG-120/10 Trimethylolpropane | | | | | |
| | Trioleate (and) | 40 - 50% | | | | |
| Arlypon TT | Laureth-2 | 40 - 50% | 1.0 | 2.0 | 1.0 | 2.0 |
| Perfume oil composition 1B | Fragrance | 100% | 0.3 | 0.3 | 0.3 | 0.3 |
| Na-Benzoate | Sodium Benzoate | 100% | 0.5 | 0.5 | 0.5 | 0.5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 70.7 | 49.0 | 56.95 | 46.25 |
| pH value | | | 5.0 | 4.9 | 5.2 | 5.1 |
| Viscosity [mPas] | | | 5600 | 3100 | 4600 | 2500 |

**Table 17: Shampoos 5, 6, 7 and 8**

| **Shampoo** | | | | **5** | **6** | **7** | **8** |
|---|---|---|---|---|---|---|---|
| **Component** | **INCI** | | **Conc.** | **% b.w.** | | | |
| Sulfopon 1216G | Sodium Coco-Sulfate | | 90 - 95% | --- | --- | 12.8 | --- |
| Texapon ALS Benz | Ammonium Lauryl Sulfate | | 27 - 28% | --- | --- | --- | 32.0 |
| Plantacare 1200UP | Lauryl Glucoside | | 51 - 53% | 15.0 | --- | --- | --- |
| Plantacare 2000UP | Decyl Glucoside | | 51 - 55% | --- | --- | --- | 6.0 |
| Plantacare 818UP | Coco-Glucoside | | 51 - 53% | --- | 30.0 | 20.4 | --- |
| Plantacare 810UP | Caprylyl/Capryl Glucoside | | 62 - 65% | 12.0 | --- | --- | --- |
| Jaguar Excel | Guar Hydroxypropyltrimonium Chloride | | 100% | 0.2 | 0.25 | 0.2 | 0.3 |
| Dehyton PK45 | Cocamidopropyl Betaine | | 44 - 46% | | | | 11.0 |
| | | 15 - 25 | | | | | |
| | Coco-Glucoside | 10 - 20 | 28.5 - 34% | | | | |
| | (and) Glyceryl | 62 - 67 | | 2.0 | 2.0 | 2.0 | 3.0 |
| | Oleate | | | | | | |
| Lamesoft PO65 | water | | | | | | |
| | Lauryl Glucoside | 15 - 25 | | | | | |
| | (and) Stearyl | 15 - 25 | 38-44% | | | | 2.0 |
| | Citrate | 55 - 65 | | | | | |
| Euperlan Green | water | | | --- | --- | --- | |
| Gluadin WLM Benz | Hydrolyzed Wheat Protein | | 21 - 24% | --- | 0.5 | 1.0 | --- |
| PLANT ACARE ® PS 10 | Sodium Laureth Sulfate (and) Lauryl Polyglucose | | 33% | 6.0 | 15.0 | 7.5 | 10.5 |
| Copherol 1250C | Tocopheryl Acetate | | 100% | 0.25 | --- | --- | --- |
| Cetiol J 600 | Oleyl Erucate | | 100% | --- | 0.3 | --- | --- |
| Almond Oil | Prunus Amygdalus Dulcis (Sweet Almond) Oil | | 100% | --- | --- | 0.2 | 0.25 |
| Keltrol CG-SFT | Xanthan Gum | | 100% | 1.0 | 1.0 | | |
| Perfume oil composition 1B | Fragrance | | 100% | 0.25 | 0.25 | 0.25 | 0.25 |
| Na-Benzoate | Sodium Benzoate | | 100% | 0.5 | 0.5 | 0.5 | 0.5 |
| Citric Acid | Citric Acid | | 50% | q.s. | q.s. | q.s. | q.s. |
| NaCl | Sodium Chloride | | 100% | q.s. | q.s. | q.s. | q.s. |
| Floral water | Floral water | | 100% | --- | --- | --- | 10.0 |
| Water | Water | | 100% | 62.8 | 50.2 | 55.15 | 24.2 |
| | | | | | | | |
| pH value | | | | 5.1 | 5.0 | 5.3 | 4.8 |
| Viscosity [mPas] | | | | 6200 | 4600 | 6800 | 5800 |
| Density | g/cm³ | | | | | | 1.028 |

Shampoo 9 corresponds to shampoo 1, where the perfume oil composition 1B is replaced by the same amount of the perfume oil composition 2B.
Shampoo 10 corresponds to shampoo 2, where the perfume oil composition 1B is replaced by the same amount of the perfume oil composition 2B.
Shampoo 11 corresponds to shampoo 3, where the perfume oil composition 1B is replaced by the same amount of the perfume oil composition 2B.
Shampoo 12 corresponds to shampoo 4, where the perfume oil composition 1B is replaced by the same amount of the perfume oil composition 2B.
Shampoo 13 corresponds to shampoo 5, where the perfume oil composition 1B is replaced by the same amount of the perfume oil composition 2B.
Shampoo 14 corresponds to shampoo 6, where the perfume oil composition 1B is replaced by the same amount of the perfume oil composition 2B.
Shampoo 15 corresponds to shampoo 7, where the perfume oil composition 1B is replaced by the same amount of the perfume oil composition 2B.
Shampoo 16 corresponds to shampoo 8, where the perfume oil composition 1B is replaced by the same amount of the perfume oil composition 2B.
Shampoo 17 corresponds to shampoo 1, where the perfume oil composition 1B is replaced by the same amount of the perfume oil composition 3.
Shampoo 18 corresponds to shampoo 2, where the perfume oil composition 1B is replaced by the same amount of the perfume oil composition 3.
Shampoo 19 corresponds to shampoo 3, where the perfume oil composition 1B is replaced by the same amount of the perfume oil composition 3.
Shampoo 20 corresponds to shampoo 4, where the perfume oil composition 1B is replaced by the same amount of the perfume oil composition 3.
Shampoo 21 corresponds to shampoo 5, where the perfume oil composition 1B is replaced by the same amount of the perfume oil composition 3.
Shampoo 22 corresponds to shampoo 6, where the perfume oil composition 1B is replaced by the same amount of the perfume oil composition 3.
Shampoo 23 corresponds to shampoo 7, where the perfume oil composition 1B is replaced by the same amount of the perfume oil composition 3.
Shampoo 24 corresponds to shampoo 8, where the perfume oil composition 1B is replaced by the same amount of the perfume oil composition 3.
Shampoo 25 corresponds to shampoo 1, where the perfume oil composition 1B is replaced by the same amount of the perfume oil composition 4.
Shampoo 26 corresponds to shampoo 2, where the perfume oil composition 1B is replaced by the same amount of the perfume oil composition 4.
Shampoo 27 corresponds to shampoo 3, where the perfume oil composition 1B is replaced by the same amount of the perfume oil composition 4.
Shampoo 28 corresponds to shampoo 4, where the perfume oil composition 1B is replaced by the same amount of the perfume oil composition 4.
Shampoo 29 corresponds to shampoo 5, where the perfume oil composition 1B is replaced by the same amount of the perfume oil composition 4.
Shampoo 30 corresponds to shampoo 6, where the perfume oil composition 1B is replaced by the same amount of the perfume oil composition 4.
Shampoo 31 corresponds to shampoo 7, where the perfume oil composition 1B is replaced by the same amount of the perfume oil composition 4.
Shampoo 32 corresponds to shampoo 8, where the perfume oil composition 1B is replaced by the same amount of the perfume oil composition 4.
Shampoo 33 corresponds to shampoo 1, where the perfume oil composition 1B is replaced by the same amount of the perfume oil composition 5.
Shampoo 34 corresponds to shampoo 2, where the perfume oil composition 1B is replaced by the same amount of the perfume oil composition 5.
Shampoo 35 corresponds to shampoo 3, where the perfume oil composition 1B is replaced by the same amount of the perfume oil composition 5.
Shampoo 36 corresponds to shampoo 4, where the perfume oil composition 1B is replaced by the same amount of the perfume oil composition 5.
Shampoo 37 corresponds to shampoo 5, where the perfume oil composition 1B is replaced by the same amount of the perfume oil composition 5.
Shampoo 38 corresponds to shampoo 6, where the perfume oil composition 1B is replaced by the same amount of the perfume oil composition 5.
Shampoo 39 corresponds to shampoo 7, where the perfume oil composition 1B is replaced by the same amount of the perfume oil composition 5.
Shampoo 40 corresponds to shampoo 8, where the perfume oil composition 1B is replaced by the same amount of the perfume oil composition 5.
Shampoo 41 corresponds to shampoo 1, where the perfume oil composition 1B is replaced by the same amount of the perfume oil composition 6.
Shampoo 42 corresponds to shampoo 2, where the perfume oil composition 1B is replaced by the same amount of the perfume oil composition 6.
Shampoo 43 corresponds to shampoo 3, where the perfume oil composition 1B is replaced by the same amount of the perfume oil composition 6.
Shampoo 44 corresponds to shampoo 4, where the perfume oil composition 1B is replaced by the same amount of the perfume oil composition 6.
Shampoo 45 corresponds to shampoo 5, where the perfume oil composition 1B is replaced by the same amount of the perfume oil composition 6.
Shampoo 46 corresponds to shampoo 6, where the perfume oil composition 1B is replaced by the same amount of the perfume oil composition 6.
Shampoo 47 corresponds to shampoo 7, where the perfume oil composition 1B is replaced by the same amount of the perfume oil composition 6.
Shampoo 48 corresponds to shampoo 8, where the perfume oil composition 1B is replaced by the same amount of the perfume oil composition 6.
Shampoo 49 corresponds to shampoo 1, where the perfume oil composition 1B is replaced by the same amount of the perfume oil composition 7.
Shampoo 50 corresponds to shampoo 2, where the perfume oil composition 1B is replaced by the same amount of the perfume oil composition 7.
Shampoo 51 corresponds to shampoo 3, where the perfume oil composition 1B is replaced by the same amount of the perfume oil composition 7.
Shampoo 52 corresponds to shampoo 4, where the perfume oil composition 1B is replaced by the same amount of the perfume oil composition 7.
Shampoo 53 corresponds to shampoo 5, where the perfume oil composition 1B is replaced by the same amount of the perfume oil composition 7.
Shampoo 54 corresponds to shampoo 6, where the perfume oil composition 1B is replaced by the same amount of the perfume oil composition 7.
Shampoo 55 corresponds to shampoo 7, where the perfume oil composition 1B is replaced by the same amount of the perfume oil composition 7.
Shampoo 56 corresponds to shampoo 8, where the perfume oil composition 1B is replaced by the same amount of the perfume oil composition 7.
Shampoo 57 corresponds to shampoo 1, where the perfume oil composition 1B is replaced by the same amount of the perfume oil composition 8.
Shampoo 58 corresponds to shampoo 2, where the perfume oil composition 1B is replaced by the same amount of the perfume oil composition 8.
Shampoo 59 corresponds to shampoo 3, where the perfume oil composition 1B is replaced by the same amount of the perfume oil composition 8.
Shampoo 60 corresponds to shampoo 4, where the perfume oil composition 1B is replaced by the same amount of the perfume oil composition 8.
Shampoo 61 corresponds to shampoo 5, where the perfume oil composition 1B is replaced by the same amount of the perfume oil composition 8.
Shampoo 62 corresponds to shampoo 6, where the perfume oil composition 1B is replaced by the same amount of the perfume oil composition 8.
Shampoo 63 corresponds to shampoo 7, where the perfume oil composition 1B is replaced by the same amount of the perfume oil composition 8.
Shampoo 64 corresponds to shampoo 8, where the perfume oil composition 1B is replaced by the same amount of the perfume oil composition 8.
Shampoo 65 corresponds to shampoo 1, where the perfume oil composition 1B is replaced by the same amount of the perfume oil composition 9.
Shampoo 66 corresponds to shampoo 2, where the perfume oil composition 1B is replaced by the same amount of the perfume oil composition 9.
Shampoo 67 corresponds to shampoo 3, where the perfume oil composition 1B is replaced by the same amount of the perfume oil composition 9.
Shampoo 68 corresponds to shampoo 4, where the perfume oil composition 1B is replaced by the same amount of the perfume oil composition 9.
Shampoo 69 corresponds to shampoo 5, where the perfume oil composition 1B is replaced by the same amount of the perfume oil composition 9.
Shampoo 70 corresponds to shampoo 6, where the perfume oil composition 1B is replaced by the same amount of the perfume oil composition 9.
Shampoo 71 corresponds to shampoo 7, where the perfume oil composition 1B is replaced by the same amount of the perfume oil composition 9.
Shampoo 72 corresponds to shampoo 8, where the perfume oil composition 1B is replaced by the same amount of the perfume oil composition 9.
Shampoo 73 corresponds to shampoo 1, where the perfume oil composition 1B is replaced by the same amount of the perfume oil composition 10.
Shampoo 74 corresponds to shampoo 2, where the perfume oil composition 1B is replaced by the same amount of the perfume oil composition 10.
Shampoo 75 corresponds to shampoo 3, where the perfume oil composition 1B is replaced by the same amount of the perfume oil composition 10.
Shampoo 76 corresponds to shampoo 4, where the perfume oil composition 1B is replaced by the same amount of the perfume oil composition 10.
Shampoo 77 corresponds to shampoo 5, where the perfume oil composition 1B is replaced by the same amount of the perfume oil composition 10.
Shampoo 78 corresponds to shampoo 6, where the perfume oil composition 1B is replaced by the same amount of the perfume oil composition 10.
Shampoo 79 corresponds to shampoo 7, where the perfume oil composition 1B is replaced by the same amount of the perfume oil composition 10.
Shampoo 80 corresponds to shampoo 8, where the perfume oil composition 1B is replaced by the same amount of the perfume oil composition 10.
Shampoo 81 corresponds to shampoo 1, where the perfume oil composition 1B is replaced by the same amount of the perfume oil composition 11.
Shampoo 82 corresponds to shampoo 2, where the perfume oil composition 1B is replaced by the same amount of the perfume oil composition 11.
Shampoo 83 corresponds to shampoo 3, where the perfume oil composition 1B is replaced by the same amount of the perfume oil composition 11.
Shampoo 84 corresponds to shampoo 4, where the perfume oil composition 1B is replaced by the same amount of the perfume oil composition 11.
Shampoo 85 corresponds to shampoo 5, where the perfume oil composition 1B is replaced by the same amount of the perfume oil composition 11.
Shampoo 86 corresponds to shampoo 6, where the perfume oil composition 1B is replaced by the same amount of the perfume oil composition 11.
Shampoo 87 corresponds to shampoo 7, where the perfume oil composition 1B is replaced by the same amount of the perfume oil composition 11.
Shampoo 88 corresponds to shampoo 8, where the perfume oil composition 1B is replaced by the same amount of the perfume oil composition 11.
Shampoo 89 corresponds to shampoo 1, where the perfume oil composition 1B is replaced by the same amount of the perfume oil composition 12.
Shampoo 90 corresponds to shampoo 2, where the perfume oil composition 1B is replaced by the same amount of the perfume oil composition 12.
Shampoo 91 corresponds to shampoo 3, where the perfume oil composition 1B is replaced by the same amount of the perfume oil composition 12.
Shampoo 92 corresponds to shampoo 4, where the perfume oil composition 1B is replaced by the same amount of the perfume oil composition 12.
Shampoo 93 corresponds to shampoo 5, where the perfume oil composition 1B is replaced by the same amount of the perfume oil composition 12.
Shampoo 94 corresponds to shampoo 6, where the perfume oil composition 1B is replaced by the same amount of the perfume oil composition 12.
Shampoo 95 corresponds to shampoo 7, where the perfume oil composition 1B is replaced by the same amount of the perfume oil composition 12.
Shampoo 96 corresponds to shampoo 8, where the perfume oil composition 1B is replaced by the same amount of the perfume oil composition 12.
Shampoo 97 corresponds to shampoo 1, where the perfume oil composition 1B is replaced by the same amount of the perfume oil composition 13.
Shampoo 98 corresponds to shampoo 2, where the perfume oil composition 1B is replaced by the same amount of the perfume oil composition 13.
Shampoo 99 corresponds to shampoo 3, where the perfume oil composition 1B is replaced by the same amount of the perfume oil composition 13.
Shampoo 100 corresponds to shampoo 4, where the perfume oil composition 1B is replaced by the same amount of the perfume oil composition 13.
Shampoo 101 corresponds to shampoo 5, where the perfume oil composition 1B is replaced by the same amount of the perfume oil composition 13.
Shampoo 102 corresponds to shampoo 6, where the perfume oil composition 1B is replaced by the same amount of the perfume oil composition 13.
Shampoo 103 corresponds to shampoo 7, where the perfume oil composition 1B is replaced by the same amount of the perfume oil composition 13.
Shampoo 104 corresponds to shampoo 8, where the perfume oil composition 1B is replaced by the same amount of the perfume oil composition 13.
Shampoo 105 corresponds to shampoo 1, where the perfume oil composition 1B is replaced by the same amount of the perfume oil composition 14.
Shampoo 106 corresponds to shampoo 2, where the perfume oil composition 1B is replaced by the same amount of the perfume oil composition 14.
Shampoo 107 corresponds to shampoo 3, where the perfume oil composition 1B is replaced by the same amount of the perfume oil composition 14.
Shampoo 108 corresponds to shampoo 4, where the perfume oil composition 1B is replaced by the same amount of the perfume oil composition 14.
Shampoo 109 corresponds to shampoo 5, where the perfume oil composition 1B is replaced by the same amount of the perfume oil composition 14.
Shampoo 110 corresponds to shampoo 6, where the perfume oil composition 1B is replaced by the same amount of the perfume oil composition 14.
Shampoo 111 corresponds to shampoo 7, where the perfume oil composition 1B is replaced by the same amount of the perfume oil composition 14.
Shampoo 112 corresponds to shampoo 8, where the perfume oil composition 1B is replaced by the same amount of the perfume oil composition 14.
Shampoo 113 corresponds to shampoo 1, where the perfume oil composition 1B is replaced by the same amount of the perfume oil composition 15.
Shampoo 114 corresponds to shampoo 2, where the perfume oil composition 1B is replaced by the same amount of the perfume oil composition 15.
Shampoo 115 corresponds to shampoo 3, where the perfume oil composition 1B is replaced by the same amount of the perfume oil composition 15.
Shampoo 116 corresponds to shampoo 4, where the perfume oil composition 1B is replaced by the same amount of the perfume oil composition 15.
Shampoo 117 corresponds to shampoo 5, where the perfume oil composition 1B is replaced by the same amount of the perfume oil composition 15.
Shampoo 118 corresponds to shampoo 6, where the perfume oil composition 1B is replaced by the same amount of the perfume oil composition 15.
Shampoo 119 corresponds to shampoo 7, where the perfume oil composition 1B is replaced by the same amount of the perfume oil composition 15.
Shampoo 120 corresponds to shampoo 8, where the perfume oil composition 1B is replaced by the same amount of the perfume oil composition 15.
Shampoo 121 corresponds to shampoo 1, where the perfume oil composition 1B is replaced by the same amount of the perfume oil composition 16.
Shampoo 122 corresponds to shampoo 2, where the perfume oil composition 1B is replaced by the same amount of the perfume oil composition 16.
Shampoo 123 corresponds to shampoo 3, where the perfume oil composition 1B is replaced by the same amount of the perfume oil composition 16.
Shampoo 124 corresponds to shampoo 4, where the perfume oil composition 1B is replaced by the same amount of the perfume oil composition 16.
Shampoo 125 corresponds to shampoo 5, where the perfume oil composition 1B is replaced by the same amount of the perfume oil composition 16.
Shampoo 126 corresponds to shampoo 6, where the perfume oil composition 1B is replaced by the same amount of the perfume oil composition 16.
Shampoo 127 corresponds to shampoo 7, where the perfume oil composition 1B is replaced by the same amount of the perfume oil composition 16.
Shampoo 128 corresponds to shampoo 1, where the perfume oil composition 1B is replaced by the same amount of the perfume oil composition 17.
Shampoo 129 corresponds to shampoo 2, where the perfume oil composition 1B is replaced by the same amount of the perfume oil composition 17.
Shampoo 130 corresponds to shampoo 3, where the perfume oil composition 1B is replaced by the same amount of the perfume oil composition 17.
Shampoo 131 corresponds to shampoo 4, where the perfume oil composition 1B is replaced by the same amount of the perfume oil composition 17.
Shampoo 132 corresponds to shampoo 5, where the perfume oil composition 1B is replaced by the same amount of the perfume oil composition 17.
Shampoo 133 corresponds to shampoo 6, where the perfume oil composition 1B is replaced by the same amount of the perfume oil composition 17.
Shampoo 134 corresponds to shampoo 7, where the perfume oil composition 1B is replaced by the same amount of the perfume oil composition 17.
Shampoo 135 corresponds to shampoo 1, where the perfume oil composition 1B is replaced by the same amount of the perfume oil composition 18.
Shampoo 136 corresponds to shampoo 2, where the perfume oil composition 1B is replaced by the same amount of the perfume oil composition 18.
Shampoo 137 corresponds to shampoo 3, where the perfume oil composition 1B is replaced by the same amount of the perfume oil composition 18.
Shampoo 138 corresponds to shampoo 4, where the perfume oil composition 1B is replaced by the same amount of the perfume oil composition 18.
Shampoo 139 corresponds to shampoo 5, where the perfume oil composition 1B is replaced by the same amount of the perfume oil composition 18.
Shampoo 140 corresponds to shampoo 6, where the perfume oil composition 1B is replaced by the same amount of the perfume oil composition 18.
Shampoo 141 corresponds to shampoo 7, where the perfume oil composition 1B is replaced by the same amount of the perfume oil composition 18.
Shampoo 142 corresponds to shampoo 8, where the perfume oil composition 1B is replaced by the same amount of the perfume oil composition 18.

**Table 18: Shower bath 1, 2, 3 and 4**

| **Shower bath** | | | | **1** | **2** | **3** | **4** |
|---|---|---|---|---|---|---|---|
| **Component** | **INCI** | | **Conc.** | **% b.w.** | | | |
| Plantapon SF | Sodium Cocoamphoacetate (and) | 10 - 20 | | | | | |
| | Glycerin (and) | 5 - 15 | 42 - 52% | | | | |
| | Lauryl Glucoside (and) | 5 - 15 | | | | | |
| | Sodium Cocoyl Glutamate (and) | 5 | | 45.0 | 50,0 | 48.0 | 44.0 |
| | Sodium Lauryl Glu-cose Carboxylate water | 5 | | | | | |
| | | 48 - 58 | | | | | |
| | Coco-Glucoside (and) Glyceryl Oleate water | 15 - 25 | 28.5-34% | 2.0 | 2.0 | 2.0 | 3.0 |
| | | 10 - 20 | | | | | |
| Lamesoft PO65 | | 62 - 67 | | | | | |
| | Lauryl Glucoside (and) Stearyl Citrate water | 15 - 25 | 38-44% | | | | |
| | | 15 - 25 | | | | | 2.0 |
| Euperlan Green | | 55 - 65 | | --- | --- | --- | |
| Gluadin WLM | Hydrolyzed Wheat | | 21 - | | | | |
| Benz | Protein | | 24% | --- | --- | 1.0 | 0.5 |
| Keltrol CG-SFT | Xanthan Gum | | 100% | 1.0 | 0.5 | --- | --- |
| PLANTACARE® PS 10 | Sodium Laureth Sulfate (and) Lauryl Polyglucose | | 33% | 4.5 | 6.0 | 10.5 | 7.5 |
| Copherol1250 C | Tocopheryl Acetate | | 100% | 0.1 | --- | --- | --- |
| Cetiol J 600 | Oleyl Erucate | | 100% | --- | 0.2 | --- | --- |
| Almond Oil | Prunus Amygdalus Dulcis (Sweet Almond) Oil | | 100% | --- | --- | 0.15 | 0.1 |
| Perfume oil composition 1B | Fragrance | | 100% | 0.25 | 0.25 | 0.25 | 0.25 |
| Na-Benzoate | Sodium Benzoate | | 100% | 0.5 | 0.5 | 0.5 | 0.5 |
| Citric Acid | Citric Acid | | 50% | q.s. | q.s. | q.s. | q.s. |
| Water | Water | | 100% | 46.65 | 40.55 | 37.6 | 42.15 |
| pH value | | | | 5.1 | 4.7 | 4.9 | 4.9 |
| Viscosity [mPas] | | | | 4200 | 4600 | 2900 | 2800 |
| Density | g/cm³ | | | | | | 1.039 |

**Table 19: Shower bath 5, 6, 7 and 8**

| **Shower bath** | | | **5** | **6** | **7** | **8** |
|---|---|---|---|---|---|---|
| **Component** | **INCI** | **Conc.** | % b.w. | | | |
| Texapon N70 | Sodium Laureth Sulfate | 70% | 12.9 | 12.9 | 14.3 | 14.3 |
| Dehyton PK45 | Cocamidopropyl Betaine | 44 - 46% | 5.4 | 5.4 | 5.4 | 5.4 |
| Plantacare 818UP | Coco-Glucoside | 51 - 53% | 2.0 | 2.0 | --- | --- |
| PLANTACARE® PS 10 | Sodium Laureth Sulfate (and) Lauryl Polyglucose | 33% | 9.0 | 9.0 | 10.5 | 10.5 |
| DC 245 | Cyclopentasiloxane | 100% | 1.0 | --- | 1.1 | --- |
| Synfluid PAO2 | Hydrogenated Didecene | 100% | --- | 0.8 | --- | 1.0 |
| Jaguar C162 | Hydroxypropyl Guar Hydroxypropyltrimonium Chloride | 100% | 0.2 | 0.2 | --- | --- |
| AquaCAT CG518 | Guar Hydroxypropy-Itrimonium Chloride | 10% | --- | --- | 2.5 | 2.5 |
| | PEG/PPG-120/10 Trimethylolpropane | 40 - 50% | | | | |
| Arlypon TT | Trioleate (and) Laureth-2 | 40 - 50% | 1.0 | 1.5 | 1.0 | 1.5 |
| Perfume oil composition 1B | Fragrance | 100% | 0.3 | 0.3 | 0.3 | 0.3 |
| Na-Benzoate | Sodium Benzoate | 100% | 0.5 | 0.5 | 0.5 | 0.5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 67.7 | 67.4 | 64.4 | 64.0 |
| pH value | | | 5.0 | 4.9 | 5.2 | 5.1 |
| Viscosity [mPas] | | | 6600 | 8100 | 6900 | 9300 |

Shower bath 9 corresponds to shower bath 1, where the perfume oil composition 1B is replaced by the same amount of the perfume oil composition 2B.
Shower bath 10 corresponds to shower bath 2, where the perfume oil composition 1B is replaced by the same amount of the perfume oil composition 2B.
Shower bath 11 corresponds to shower bath 3, where the perfume oil composition 1B is replaced by the same amount of the perfume oil composition 2B.
Shower bath 12 corresponds to shower bath 4, where the perfume oil composition 1B is replaced by the same amount of the perfume oil composition 2B.
Shower bath 13 corresponds to shower bath 5, where the perfume oil composition 1B is replaced by the same amount of the perfume oil composition 2B.
Shower bath 14 corresponds to shower bath 6, where the perfume oil composition 1B is replaced by the same amount of the perfume oil composition 2B.
Shower bath 15 corresponds to shower bath 7, where the perfume oil composition 1B is replaced by the same amount of the perfume oil composition 2B.
Shower bath 16 corresponds to shower bath 8, where the perfume oil composition 1B is replaced by the same amount of the perfume oil composition 2B.
Shower bath 17 corresponds to shower bath 1, where the perfume oil composition 1B is replaced by the same amount of the perfume oil composition 3.
Shower bath 18 corresponds to shower bath 2, where the perfume oil composition 1B is replaced by the same amount of the perfume oil composition 3.
Shower bath 19 corresponds to shower bath 3, where the perfume oil composition 1B is replaced by the same amount of the perfume oil composition 3.
Shower bath 20 corresponds to shower bath 4, where the perfume oil composition 1B is replaced by the same amount of the perfume oil composition 3.
Shower bath 21 corresponds to shower bath 5, where the perfume oil composition 1B is replaced by the same amount of the perfume oil composition 3.
Shower bath 22 corresponds to shower bath 6, where the perfume oil composition 1B is replaced by the same amount of the perfume oil composition 3.
Shower bath 23 corresponds to shower bath 7, where the perfume oil composition 1B is replaced by the same amount of the perfume oil composition 3.
Shower bath 24 corresponds to shower bath 8, where the perfume oil composition 1B is replaced by the same amount of the perfume oil composition 3.
Shower bath 25 corresponds to shower bath 1, where the perfume oil composition 1B is replaced by the same amount of the perfume oil composition 4.
Shower bath 26 corresponds to shower bath 2, where the perfume oil composition 1B is replaced by the same amount of the perfume oil composition 4.
Shower bath 27 corresponds to shower bath 3, where the perfume oil composition 1B is replaced by the same amount of the perfume oil composition 4.
Shower bath 28 corresponds to shower bath 4, where the perfume oil composition 1B is replaced by the same amount of the perfume oil composition 4.
Shower bath 29 corresponds to shower bath 5, where the perfume oil composition 1B is replaced by the same amount of the perfume oil composition 4.
Shower bath 30 corresponds to shower bath 6, where the perfume oil composition 1B is replaced by the same amount of the perfume oil composition 4.
Shower bath 31 corresponds to shower bath 7, where the perfume oil composition 1B is replaced by the same amount of the perfume oil composition 4.
Shower bath 32 corresponds to shower bath 8, where the perfume oil composition 1B is replaced by the same amount of the perfume oil composition 4.
Shower bath 33 corresponds to shower bath 1, where the perfume oil composition 1B is replaced by the same amount of the perfume oil composition 5.
Shower bath 34 corresponds to shower bath 2, where the perfume oil composition 1B is replaced by the same amount of the perfume oil composition 5.
Shower bath 35 corresponds to shower bath 3, where the perfume oil composition 1B is replaced by the same amount of the perfume oil composition 5.
Shower bath 36 corresponds to shower bath 4, where the perfume oil composition 1B is replaced by the same amount of the perfume oil composition 5.
Shower bath 37 corresponds to shower bath 5, where the perfume oil composition 1B is replaced by the same amount of the perfume oil composition 5.
Shower bath 38 corresponds to shower bath 6, where the perfume oil composition 1B is replaced by the same amount of the perfume oil composition 5.
Shower bath 39 corresponds to shower bath 7, where the perfume oil composition 1B is replaced by the same amount of the perfume oil composition 5.
Shower bath 40 corresponds to shower bath 8, where the perfume oil composition 1B is replaced by the same amount of the perfume oil composition 5.
Shower bath 41 corresponds to shower bath 1, where the perfume oil composition 1B is replaced by the same amount of the perfume oil composition 6.
Shower bath 42 corresponds to shower bath 2, where the perfume oil composition 1B is replaced by the same amount of the perfume oil composition 6.
Shower bath 43 corresponds to shower bath 3, where the perfume oil composition 1B is replaced by the same amount of the perfume oil composition 6.
Shower bath 44 corresponds to shower bath 4, where the perfume oil composition 1B is replaced by the same amount of the perfume oil composition 6.
Shower bath 45 corresponds to shower bath 5, where the perfume oil composition 1B is replaced by the same amount of the perfume oil composition 6.
Shower bath 46 corresponds to shower bath 6, where the perfume oil composition 1B is replaced by the same amount of the perfume oil composition 6.
Shower bath 47 corresponds to shower bath 7, where the perfume oil composition 1B is replaced by the same amount of the perfume oil composition 6.
Shower bath 48 corresponds to shower bath 8, where the perfume oil composition 1B is replaced by the same amount of the perfume oil composition 6.
Shower bath 49 corresponds to shower bath 1, where the perfume oil composition 1B is replaced by the same amount of the perfume oil composition 7.
Shower bath 50 corresponds to shower bath 2, where the perfume oil composition 1B is replaced by the same amount of the perfume oil composition 7.
Shower bath 51 corresponds to shower bath 3, where the perfume oil composition 1B is replaced by the same amount of the perfume oil composition 7.
Shower bath 52 corresponds to shower bath 4, where the perfume oil composition 1B is replaced by the same amount of the perfume oil composition 7.
Shower bath 53 corresponds to shower bath 5, where the perfume oil composition 1B is replaced by the same amount of the perfume oil composition 7.
Shower bath 54 corresponds to shower bath 6, where the perfume oil composition 1B is replaced by the same amount of the perfume oil composition 7.
Shower bath 55 corresponds to shower bath 7, where the perfume oil composition 1B is replaced by the same amount of the perfume oil composition 7.
Shower bath 56 corresponds to shower bath 8, where the perfume oil composition 1B is replaced by the same amount of the perfume oil composition 7.
Shower bath 57 corresponds to shower bath 1, where the perfume oil composition 1B is replaced by the same amount of the perfume oil composition 8.
Shower bath 58 corresponds to shower bath 2, where the perfume oil composition 1B is replaced by the same amount of the perfume oil composition 8.
Shower bath 59 corresponds to shower bath 3, where the perfume oil composition 1B is replaced by the same amount of the perfume oil composition 8.
Shower bath 60 corresponds to shower bath 4, where the perfume oil composition 1B is replaced by the same amount of the perfume oil composition 8.
Shower bath 61 corresponds to shower bath 5, where the perfume oil composition 1B is replaced by the same amount of the perfume oil composition 8.
Shower bath 62 corresponds to shower bath 6, where the perfume oil composition 1B is replaced by the same amount of the perfume oil composition 8.
Shower bath 63 corresponds to shower bath 7, where the perfume oil composition 1B is replaced by the same amount of the perfume oil composition 8.
Shower bath 64 corresponds to shower bath 8, where the perfume oil composition 1B is replaced by the same amount of the perfume oil composition 8.
Shower bath 65 corresponds to shower bath 1, where the perfume oil composition 1B is replaced by the same amount of the perfume oil composition 9.
Shower bath 66 corresponds to shower bath 2, where the perfume oil composition 1B is replaced by the same amount of the perfume oil composition 9.
Shower bath 67 corresponds to shower bath 3, where the perfume oil composition 1B is replaced by the same amount of the perfume oil composition 9.
Shower bath 68 corresponds to shower bath 4, where the perfume oil composition 1B is replaced by the same amount of the perfume oil composition 9.
Shower bath 69 corresponds to shower bath 5, where the perfume oil composition 1B is replaced by the same amount of the perfume oil composition 9.
Shower bath 70 corresponds to shower bath 6, where the perfume oil composition 1B is replaced by the same amount of the perfume oil composition 9.
Shower bath 71 corresponds to shower bath 7, where the perfume oil composition 1B is replaced by the same amount of the perfume oil composition 9.
Shower bath 72 corresponds to shower bath 8, where the perfume oil composition 1B is replaced by the same amount of the perfume oil composition 9.
Shower bath 73 corresponds to shower bath 1, where the perfume oil composition 1B is replaced by the same amount of the perfume oil composition 10.
Shower bath 74 corresponds to shower bath 2, where the perfume oil composition 1B is replaced by the same amount of the perfume oil composition 10.
Shower bath 75 corresponds to shower bath 3, where the perfume oil composition 1B is replaced by the same amount of the perfume oil composition 10.
Shower bath 76 corresponds to shower bath 4, where the perfume oil composition 1B is replaced by the same amount of the perfume oil composition 10.
Shower bath 77 corresponds to shower bath 5, where the perfume oil composition 1B is replaced by the same amount of the perfume oil composition 10.
Shower bath 78 corresponds to shower bath 6, where the perfume oil composition 1B is replaced by the same amount of the perfume oil composition 10.
Shower bath 79 corresponds to shower bath 7, where the perfume oil composition 1B is replaced by the same amount of the perfume oil composition 10.
Shower bath 80 corresponds to shower bath 8, where the perfume oil composition 1B is replaced by the same amount of the perfume oil composition 10.
Shower bath 81 corresponds to shower bath 1, where the perfume oil composition 1B is replaced by the same amount of the perfume oil composition 11.
Shower bath 82 corresponds to shower bath 2, where the perfume oil composition 1B is replaced by the same amount of the perfume oil composition 11.
Shower bath 83 corresponds to shower bath 3, where the perfume oil composition 1B is replaced by the same amount of the perfume oil composition 11.
Shower bath 84 corresponds to shower bath 4, where the perfume oil composition 1B is replaced by the same amount of the perfume oil composition 11.
Shower bath 85 corresponds to shower bath 5, where the perfume oil composition 1B is replaced by the same amount of the perfume oil composition 11.
Shower bath 86 corresponds to shower bath 6, where the perfume oil composition 1B is replaced by the same amount of the perfume oil composition 11.
Shower bath 87 corresponds to shower bath 7, where the perfume oil composition 1B is replaced by the same amount of the perfume oil composition 11.
Shower bath 88 corresponds to shower bath 8, where the perfume oil composition 1B is replaced by the same amount of the perfume oil composition 11.
Shower bath 89 corresponds to shower bath 1, where the perfume oil composition 1B is replaced by the same amount of the perfume oil composition 12.
Shower bath 90 corresponds to shower bath 2, where the perfume oil composition 1B is replaced by the same amount of the perfume oil composition 12.
Shower bath 91 corresponds to shower bath 3, where the perfume oil composition 1B is replaced by the same amount of the perfume oil composition 12.
Shower bath 92 corresponds to shower bath 4, where the perfume oil composition 1B is replaced by the same amount of the perfume oil composition 12.
Shower bath 93 corresponds to shower bath 5, where the perfume oil composition 1B is replaced by the same amount of the perfume oil composition 12.
Shower bath 94 corresponds to shower bath 6, where the perfume oil composition 1B is replaced by the same amount of the perfume oil composition 12.
Shower bath 95 corresponds to shower bath 7, where the perfume oil composition 1B is replaced by the same amount of the perfume oil composition 12.
Shower bath 96 corresponds to shower bath 8, where the perfume oil composition 1B is replaced by the same amount of the perfume oil composition 12.
Shower bath 97 corresponds to shower bath 1, where the perfume oil composition 1B is replaced by the same amount of the perfume oil composition 13.
Shower bath 98 corresponds to shower bath 2, where the perfume oil composition 1B is replaced by the same amount of the perfume oil composition 13.
Shower bath 99 corresponds to shower bath 3, where the perfume oil composition 1B is replaced by the same amount of the perfume oil composition 13.
Shower bath 100 corresponds to shower bath 4, where the perfume oil composition 1B is replaced by the same amount of the perfume oil composition 13.
Shower bath 101 corresponds to shower bath 5, where the perfume oil composition 1B is replaced by the same amount of the perfume oil composition 13.
Shower bath 102 corresponds to shower bath 6, where the perfume oil composition 1B is replaced by the same amount of the perfume oil composition 13.
Shower bath 103 corresponds to shower bath 7, where the perfume oil composition 1B is replaced by the same amount of the perfume oil composition 13.
Shower bath 104 corresponds to shower bath 8, where the perfume oil composition 1B is replaced by the same amount of the perfume oil composition 13.
Shower bath 105 corresponds to shower bath 1, where the perfume oil composition 1B is replaced by the same amount of the perfume oil composition 14.
Shower bath 106 corresponds to shower bath 2, where the perfume oil composition 1B is replaced by the same amount of the perfume oil composition 14.
Shower bath 107 corresponds to shower bath 3, where the perfume oil composition 1B is replaced by the same amount of the perfume oil composition 14.
Shower bath 108 corresponds to shower bath 4, where the perfume oil composition 1B is replaced by the same amount of the perfume oil composition 14.
Shower bath 109 corresponds to shower bath 5, where the perfume oil composition 1B is replaced by the same amount of the perfume oil composition 14.
Shower bath 110 corresponds to shower bath 6, where the perfume oil composition 1B is replaced by the same amount of the perfume oil composition 14.
Shower bath 111 corresponds to shower bath 7, where the perfume oil composition 1B is replaced by the same amount of the perfume oil composition 14.
Shower bath 112 corresponds to shower bath 8, where the perfume oil composition 1B is replaced by the same amount of the perfume oil composition 14.
Shower bath 113 corresponds to shower bath 1, where the perfume oil composition 1B is replaced by the same amount of the perfume oil composition 15.
Shower bath 114 corresponds to shower bath 2, where the perfume oil composition 1B is replaced by the same amount of the perfume oil composition 15.
Shower bath 115 corresponds to shower bath 3, where the perfume oil composition 1B is replaced by the same amount of the perfume oil composition 15.
Shower bath 116 corresponds to shower bath 4, where the perfume oil composition 1B is replaced by the same amount of the perfume oil composition 15.
Shower bath 117 corresponds to shower bath 5, where the perfume oil composition 1B is replaced by the same amount of the perfume oil composition 15.
Shower bath 118 corresponds to shower bath 6, where the perfume oil composition 1B is replaced by the same amount of the perfume oil composition 15.
Shower bath 119 corresponds to shower bath 7, where the perfume oil composition 1B is replaced by the same amount of the perfume oil composition 15.
Shower bath 120 corresponds to shower bath 8, where the perfume oil composition 1B is replaced by the same amount of the perfume oil composition 15.
Shower bath 121 corresponds to shower bath 1, where the perfume oil composition 1B is replaced by the same amount of the perfume oil composition 16.
Shower bath 122 corresponds to shower bath 2, where the perfume oil composition 1B is replaced by the same amount of the perfume oil composition 16.
Shower bath 123 corresponds to shower bath 3, where the perfume oil composition 1B is replaced by the same amount of the perfume oil composition 16.
Shower bath 124 corresponds to shower bath 4, where the perfume oil composition 1B is replaced by the same amount of the perfume oil composition 16.
Shower bath 125 corresponds to shower bath 5, where the perfume oil composition 1B is replaced by the same amount of the perfume oil composition 16.
Shower bath 126 corresponds to shower bath 6, where the perfume oil composition 1B is replaced by the same amount of the perfume oil composition 16.
Shower bath 127 corresponds to shower bath 7, where the perfume oil composition 1B is replaced by the same amount of the perfume oil composition 16.
Shower bath 128 corresponds to shower bath 8, where the perfume oil composition 1B is replaced by the same amount of the perfume oil composition 16.
Shower bath 129 corresponds to shower bath 1, where the perfume oil composition 1B is replaced by the same amount of the perfume oil composition 17.
Shower bath 130 corresponds to shower bath 2, where the perfume oil composition 1B is replaced by the same amount of the perfume oil composition 17.
Shower bath 131 corresponds to shower bath 3, where the perfume oil composition 1B is replaced by the same amount of the perfume oil composition 17.
Shower bath 132 corresponds to shower bath 4, where the perfume oil composition 1B is replaced by the same amount of the perfume oil composition 17.
Shower bath 133 corresponds to shower bath 5, where the perfume oil composition 1B is replaced by the same amount of the perfume oil composition 17.
Shower bath 134 corresponds to shower bath 6, where the perfume oil composition 1B is replaced by the same amount of the perfume oil composition 17.
Shower bath 135 corresponds to shower bath 7, where the perfume oil composition 1B is replaced by the same amount of the perfume oil composition 17.
Shower bath 136 corresponds to shower bath 8, where the perfume oil composition 1B is replaced by the same amount of the perfume oil composition 17.
Shower bath 137 corresponds to shower bath 1, where the perfume oil composition 1B is replaced by the same amount of the perfume oil composition 18.
Shower bath 138 corresponds to shower bath 2, where the perfume oil composition 1B is replaced by the same amount of the perfume oil composition 18.
Shower bath 139 corresponds to shower bath 3, where the perfume oil composition 1B is replaced by the same amount of the perfume oil composition 18.
Shower bath 140 corresponds to shower bath 4, where the perfume oil composition 1B is replaced by the same amount of the perfume oil composition 18.
Shower bath 141 corresponds to shower bath 5, where the perfume oil composition 1B is replaced by the same amount of the perfume oil composition 18.
Shower bath 142 corresponds to shower bath 6, where the perfume oil composition 1B is replaced by the same amount of the perfume oil composition 18.
Shower bath 143 corresponds to shower bath 7, where the perfume oil composition 1B is replaced by the same amount of the perfume oil composition 18.
Shower bath 144 corresponds to shower bath 8, where the perfume oil composition 1B is replaced by the same amount of the perfume oil composition 18.

**Table 20: Hydro-alcoholic AP/Deo pump spray**

| **Component** | **INCI** | **amount %** |
|---|---|---|
| Ethanol, cosm. | Alcohol | 40.0 |
| water, demin. | Aqua | 37.5 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1.5 |
| Chlorhydrol (50% solut.) | Aluminium Chlorohydrate | 8.0 |
| Perfume oil composition 1B | | 1.0 |

Deo pump spray according to table 20, where the perfume oil composition 1B is replaced by the same amount of the perfume oil composition 2B.
Deo pump spray according to table 20, where the perfume oil composition 1B is replaced by the same amount of the perfume oil composition 3.
Deo pump spray according to table 20, where the perfume oil composition 1B is replaced by the same amount of the perfume oil composition 4.
Deo pump spray according to table 20, where the perfume oil composition 1B is replaced by the same amount of the perfume oil composition 5.
Deo pump spray according to table 20, where the perfume oil composition 1B is replaced by the same amount of the perfume oil composition 6.
Deo pump spray according to table 20, where the perfume oil composition 1B is replaced by the same amount of the perfume oil composition 7.
Deo pump spray according to table 20, where the perfume oil composition 1B is replaced by the same amount of the perfume oil composition 8.
Deo pump spray according to table 20, where the perfume oil composition 1B is replaced by the same amount of the perfume oil composition 9.
Deo pump spray according to table 20, where the perfume oil composition 1B is replaced by the same amount of the perfume oil composition 10.
Deo pump spray according to table 20, where the perfume oil composition 1B is replaced by the same amount of the perfume oil composition 11.
Deo pump spray according to table 20, where the perfume oil composition 1B is replaced by the same amount of the perfume oil composition 12.
Deo pump spray according to table 20, where the perfume oil composition 1B is replaced by the same amount of the perfume oil composition 13.
Deo pump spray according to table 20, where the perfume oil composition 1B is replaced by the same amount of the perfume oil composition 14.
Deo pump spray according to table 20, where the perfume oil composition 1B is replaced by the same amount of the perfume oil composition 15.
Deo pump spray according to table 20, where the perfume oil composition 1B is replaced by the same amount of the perfume oil composition 16.
Deo pump spray according to table 20, where the perfume oil composition 1B is replaced by the same amount of the perfume oil composition 17.
Deo pump spray according to table 20, where the perfume oil composition 1B is replaced by the same amount of the perfume oil composition 18.

**Table 21: Aerosol 1**

| **Component** | **INCI** | **amount %** |
|---|---|---|
| Ethanol, cosm. | Alcohol | 80.0 |
| water, demin. | Aqua | 16.7 |
| Propylene glycol | Propylene Glycol | 1.0 |
| Eumulgin® HRE 60 | PEG-60 Hydrogenated Castor Oil | 1.5 |
| alpha-Bisabolol | Bisabolol | 0.05 |
| Triclosan | Triclosan | 0.25 |
| Perfume oil composition 1B | | 1.0 |

Aerosol 2 corresponds to aerosol 1, where the perfume oil composition 1B is replaced by the same amount of the perfume oil composition 2B.
Aerosol 3 corresponds to aerosol 1, where the perfume oil composition 1B is replaced by the same amount of the perfume oil composition 3.
Aerosol 4 corresponds to aerosol 1, where the perfume oil composition 1B is replaced by the same amount of the perfume oil composition 4.
Aerosol 5 corresponds to aerosol 1, where the perfume oil composition 1B is replaced by the same amount of the perfume oil composition 5.
Aerosol 6 corresponds to aerosol 1, where the perfume oil composition 1B is replaced by the same amount of the perfume oil composition 6.
Aerosol 7 corresponds to aerosol 1, where the perfume oil composition 1B is replaced by the same amount of the perfume oil composition 7.
Aerosol 8 corresponds to aerosol 1, where the perfume oil composition 1B is replaced by the same amount of the perfume oil composition 8.
Aerosol 9 corresponds to aerosol 1, where the perfume oil composition 1B is replaced by the same amount of the perfume oil composition 9.
Aerosol 10 corresponds to aerosol 1, where the perfume oil composition 1B is replaced by the same amount of the perfume oil composition 10.
Aerosol 11 corresponds to aerosol 1, where the perfume oil composition 1B is replaced by the same amount of the perfume oil composition 11.
Aerosol 12 corresponds to aerosol 1, where the perfume oil composition 1B is replaced by the same amount of the perfume oil composition 12.
Aerosol 13 corresponds to aerosol 1, where the perfume oil composition 1B is replaced by the same amount of the perfume oil composition 13.
Aerosol 14 corresponds to aerosol 1, where the perfume oil composition 1B is replaced by the same amount of the perfume oil composition 14.
Aerosol 15 corresponds to aerosol 1, where the perfume oil composition 1B is replaced by the same amount of the perfume oil composition 15.
Aerosol 16 corresponds to aerosol 1, where the perfume oil composition 1B is replaced by the same amount of the perfume oil composition 16.
Aerosol 17 corresponds to aerosol 1, where the perfume oil composition 1B is replaced by the same amount of the perfume oil composition 17.
Aerosol 18 corresponds to aerosol 1, where the perfume oil composition 1B is replaced by the same amount of the perfume oil composition 18.

**Table 22: Aqueous/alcoholic AP/Deo roll-on**

| **Component** | **INCI** | **amount %** |
|---|---|---|
| Ethanol, cosm. | Alcohol | 40.0 |
| water, demin. | Aqua | 37.0 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1.5 |
| Chlorhydrol (50% solut.) | Aluminium Chlorohydarte | 8.0 |
| Klucel M | Hydroxypropylcellulose | 0.5 |
| Perfume oil composition 1B | | 1.0 |

Aqueous/alcoholic AP/Deo roll-on according to table 22, where the perfume oil composition 1 B is replaced by the same amount of the perfume oil composition 2B. Aqueous/alcoholic AP/Deo roll-on according to table 22, where the perfume oil composition 1 B is replaced by the same amount of the perfume oil composition 3. Aqueous/alcoholic AP/Deo roll-on according to table 22, where the perfume oil composition 1 B is replaced by the same amount of the perfume oil composition 4. Aqueous/alcoholic AP/Deo roll-on according to table 22, where the perfume oil composition 1 B is replaced by the same amount of the perfume oil composition 5. Aqueous/alcoholic AP/Deo roll-on according to table 22, where the perfume oil composition 1B is replaced by the same amount of the perfume oil composition 6. Aqueous/alcoholic AP/Deo roll-on according to table 22, where the perfume oil composition 1B is replaced by the same amount of the perfume oil composition 7. Aqueous/alcoholic AP/Deo roll-on according to table 22, where the perfume oil composition 1B is replaced by the same amount of the perfume oil composition 8. Aqueous/alcoholic AP/Deo roll-on according to table 22, where the perfume oil composition 1B is replaced by the same amount of the perfume oil composition 9. Aqueous/alcoholic AP/Deo roll-on according to table 22, where the perfume oil composition 1B is replaced by the same amount of the perfume oil composition 10. Aqueous/alcoholic AP/Deo roll-on according to table 22, where the perfume oil composition 1B is replaced by the same amount of the perfume oil composition 11. Aqueous/alcoholic AP/Deo roll-on according to table 22, where the perfume oil composition 1B is replaced by the same amount of the perfume oil composition 12. Aqueous/alcoholic AP/Deo roll-on according to table 22, where the perfume oil composition 1B is replaced by the same amount of the perfume oil composition 13. Aqueous/alcoholic AP/Deo roll-on according to table 22, where the perfume oil composition 1B is replaced by the same amount of the perfume oil composition 14. Aqueous/alcoholic AP/Deo roll-on according to table 22, where the perfume oil composition 1B is replaced by the same amount of the perfume oil composition 15. Aqueous/alcoholic AP/Deo roll-on according to table 22, where the perfume oil composition 1B is replaced by the same amount of the perfume oil composition 16. Aqueous/alcoholic AP/Deo roll-on according to table 22, where the perfume oil composition 1B is replaced by the same amount of the perfume oil composition 17. Aqueous/alcoholic AP/Deo roll-on according to table 22, where the perfume oil composition 1B is replaced by the same amount of the perfume oil composition 18.

**Table 23: Styling Gel Type "Out of Bed"**

| **Phase** | **Component** | **INCI** | **%** | **Function** |
|---|---|---|---|---|
| I | Alcool 96% (Prolabo) | Alcohol 96% | 12.00 | Solvent |
| | Luviskol VA 64 (BASF) | PVP/VA | 4.50 | Styling agent |
| | Polyox WSR-301 | PEG-90M | 0.25 | Styling agent |
| | Methocel E4M Premium EP (DOW) | Hydroxypropyl Methylcellulose | 0.60 | Thickener |
| | DEHYQUART® L 80 | Dicocoylethyl Hydroxy-ethylmonium Methosulfate (and) Propylene Glycol | 0.60 | Active agent |
| II. | Eumulgin® HRE 60 | Hardened castor oil with approx. 60 mol EO | 1.00 | Solubilizer |
| | CETIOL® OE | Dicaprylyl Ether | 1.50 | Emollient |
| III. | Water, de-ionized | Aqua | a.d. | |
| IV. | HISPAGEL® 200 | Glycerin (and) Glyceryl Polyacrylate | 36.70 | Thickener |
| V. | Perfume oil composition 1B | | 0.02 | |
| >> | pH-value | | 6.8 | |
| >> | Viscosity (mPas) Brookfield RVT 23°C spindle 5, 10rpm | | 220000 mPas | |

Styling gel according to table 23, where the perfume oil composition 1B is replaced by the same amount of the perfume oil composition 2B.
Styling gel according to table 23,, where the perfume oil composition 1B is replaced by the same amount of the perfume oil composition 3.
Styling gel according to table 23, where the perfume oil composition 1B is replaced by the same amount of the perfume oil composition 4.
Styling gel according to table 23, where the perfume oil composition 1B is replaced by the same amount of the perfume oil composition 5.
Styling gel according to table 23, where the perfume oil composition 1B is replaced by the same amount of the perfume oil composition 6.
Styling gel according to table 23, where the perfume oil composition 1B is replaced by the same amount of the perfume oil composition 7.
Styling gel according to table 23, where the perfume oil composition 1B is replaced by the same amount of the perfume oil composition 8.
Styling gel according to table 23, where the perfume oil composition 1B is replaced by the same amount of the perfume oil composition 9.
Styling gel according to table 23, where the perfume oil composition 1B is replaced by the same amount of the perfume oil composition 10.
Styling gel according to table 23, where the perfume oil composition 1B is replaced by the same amount of the perfume oil composition 11.
Styling gel according to table 23, where the perfume oil composition 1B is replaced by the same amount of the perfume oil composition 12.
Styling gel according to table 23, where the perfume oil composition 1B is replaced by the same amount of the perfume oil composition 13.
Styling gel according to table 23, where the perfume oil composition 1B is replaced by the same amount of the perfume oil composition 14.
Styling gel according to table 23, where the perfume oil composition 1B is replaced by the same amount of the perfume oil composition 15.
Styling gel according to table 23, where the perfume oil composition 1B is replaced by the same amount of the perfume oil composition 16.
Styling gel according to table 23, where the perfume oil composition 1B is replaced by the same amount of the perfume oil composition 17.
Styling gel according to table 23, where the perfume oil composition 1B is replaced by the same amount of the perfume oil composition 18.

**Table 24: Shaving Foam**

| **Phase** | **Component** | **INCI** | **%** | **Function** |
|---|---|---|---|---|
| | CUTINA® FS 45 | Stearic Acid (and) Palmitic Acid | 7.6 | Surfactant (foam consistency) |
| | PLANTACARE® 1200 UP | Lauryl Glucoside | 6.0 | Surfactant (inhibitory effect) |
| | MONOMULS® 90-O 18 | Glyceryl Oleate | 1.2 | W/O emulsifier (moisturizing) |
| | EUMULGIN® O20 S | Oleth-20 | 1.2 | O/W-emulsifier (stability) |
| | CETIOL® CC | Dicaprylyl Carbonate | 2.0 | Emollient (low viscosity) |
| | DEHYQUART® SP | Quaternium-52 | 0.5 | Surfactant (inhibitor) |
| | TEA (99%) | | 4.0 | Neutralizer (for Cutina FS 45) |
| | Glycerin | Glycerin | 3.0 | Humectant |
| | Propylene Glycol | Propylene Glycol | 3.0 | Humectant (low viscosiy) |
| | Emulgin® HRE 60 | Hardened castor oil with approx. 60 mol EO | 2.0 | Solubilizer |
| | D-Panthenol | Panthenol | 0.2 | Care additive |
| | Phenonip | Phenoxyethanol (and) Parabens | 0.5 | Preservative |
| | Perfume oil composition 1B | | 0.3 | Fragrance |
| | Distilled or Deionized Water | Aqua | ad 100 | |

Shaving foam according to table 24, where the perfume oil composition 1B is replaced by the same amount of the perfume oil composition 2B.
Shaving foam according to table 24, where the perfume oil composition 1B is replaced by the same amount of the perfume oil composition 3.
Shaving foam according to table 24, where the perfume oil composition 1B is replaced by the same amount of the perfume oil composition 4.
Shaving foam according to table 24, where the perfume oil composition 1B is replaced by the same amount of the perfume oil composition 5.
Shaving foam according to table 24, where the perfume oil composition 1B is replaced by the same amount of the perfume oil composition 6.
Shaving foam according to table 24, where the perfume oil composition 1B is replaced by the same amount of the perfume oil composition 7.
Shaving foam according to table 24, where the perfume oil composition 1B is replaced by the same amount of the perfume oil composition 8.
Shaving foam according to table 24, where the perfume oil composition 1B is replaced by the same amount of the perfume oil composition 9.
Shaving foam according to table 24, where the perfume oil composition 1B is replaced by the same amount of the perfume oil composition 10.
Shaving foam according to table 24, where the perfume oil composition 1B is replaced by the same amount of the perfume oil composition 11.
Shaving foam according to table 24, where the perfume oil composition 1B is replaced by the same amount of the perfume oil composition 12.
Shaving foam according to table 24, where the perfume oil composition 1B is replaced by the same amount of the perfume oil composition 13.
Shaving foam according to table 24, where the perfume oil composition 1B is replaced by the same amount of the perfume oil composition 14.
Shaving foam according to table 24, where the perfume oil composition 1B is replaced by the same amount of the perfume oil composition 15.
Shaving foam according to table 24, where the perfume oil composition 1B is replaced by the same amount of the perfume oil composition 16.
Shaving foam according to table 24, where the perfume oil composition 1B is replaced by the same amount of the perfume oil composition 17.
Shaving foam according to table 24, where the perfume oil composition 1B is replaced by the same amount of the perfume oil composition 18.

**Table 25: Sensitive skin Baby shampoo**

| **Ingredients** | | **INCI / Chemistry** | **%** | **Function** |
|---|---|---|---|---|
| I. | PLANTAPON® LGC SORB | Sodium Lauryl Glucose Carboxylate (and) Lauryl Glucoside | 28.00 | Surfactant, cleaning |
| | ARLYPON® TT | PEG/PPG-120/10 Trimethylolpropane Trioleate (and) Laureth-2 | 2.00 | Thickener |
| | DEHYTON® MC | Sodium Cocoamphoacetate | 6.00 | Surfactant |
| | EUMULGIN® SML 20 | Polysorbate 20 | 3.00 | Solubilizer |
| | LAMESOFT® PO 65 | Coco-Glucoside (and) Glyceryl Oleate | 2.20 | Lipid layer enhancer |
| | MELHYDRAN™ LS 4420 | Honey Extract | 1.00 | Active ingredient |
| | Perfume oil composition 1B | Perfume | 0.20 | Perfume |
| | Dermosoft 1388 (Dr. Straetmans) | Water (and) Glycerin (and) Sodium Levulinate (and) Sodium Anisate | 4.00 | Active ingredient |
| | Water, demin. | Aqua | 53.60 | |

Sensitive skin baby shampoo according to table 25, where the perfume oil composition 1B is replaced by the same amount of the perfume oil composition 2B.
Sensitive skin baby shampoo according to table 25, where the perfume oil composition 1B is replaced by the same amount of the perfume oil composition 3.
Sensitive skin baby shampoo according to table 25, where the perfume oil composition 1B is replaced by the same amount of the perfume oil composition 4.
Sensitive skin baby shampoo according to table 25, where the perfume oil composition 1B is replaced by the same amount of the perfume oil composition 5.
Sensitive skin baby shampoo according to table 25, where the perfume oil composition 1 B is replaced by the same amount of the perfume oil composition 6.
Sensitive skin baby shampoo according to table 25, where the perfume oil composition 1 B is replaced by the same amount of the perfume oil composition 7.
Sensitive skin baby shampoo according to table 25, where the perfume oil composition 1 B is replaced by the same amount of the perfume oil composition 8.
Sensitive skin baby shampoo according to table 25, where the perfume oil composition 1B is replaced by the same amount of the perfume oil composition 9.
Sensitive skin baby shampoo according to table 25, where the perfume oil composition 1B is replaced by the same amount of the perfume oil composition 10.
Sensitive skin baby shampoo according to table 25, where the perfume oil composition 1B is replaced by the same amount of the perfume oil composition 11.
Sensitive skin baby shampoo according to table 25, where the perfume oil composition 1B is replaced by the same amount of the perfume oil composition 12.
Sensitive skin baby shampoo according to table 25, where the perfume oil composition 1B is replaced by the same amount of the perfume oil composition 13.
Sensitive skin baby shampoo according to table 25, where the perfume oil composition 1B is replaced by the same amount of the perfume oil composition 14.
Sensitive skin baby shampoo according to table 25, where the perfume oil composition 1B is replaced by the same amount of the perfume oil composition 15.
Sensitive skin baby shampoo according to table 25, where the perfume oil composition 1B is replaced by the same amount of the perfume oil composition 16.
Sensitive skin baby shampoo according to table 25, where the perfume oil composition 1B is replaced by the same amount of the perfume oil composition 17.
Sensitive skin baby shampoo according to table 25, where the perfume oil composition 1B is replaced by the same amount of the perfume oil composition 18.

**Table 26: Body wash for Sensitive Skin**

| **Ingredients** | | **INCI / Chemistry** | **%** | **Function** |
|---|---|---|---|---|
| I. | Water, demin. | Aqua | 70.90 | |
| | TEXAPON® N 70 | Sodium Laureth Sulfate | 13.00 | Surfactant |
| | PLANTACARE® 818 UP | Coco-Glucoside | 3.00 | Surfactant |
| | LAMESOFT® PO 65 | Coco-Glucoside (and) Glyceryl Oleate | 5.00 | Active, skin conditioning |
| | Perfume oil composition 1B | Fragrance | 0.15 | Perfume |
| | Sodium Benzoate | Sodium Benzoate | 0.55 | Preservative |
| II. | DEHYQUART® CC7 BZ | Polyquaternium-7 | 2.00 | Active, hair conditioning |
| III. | MELHYDRAN™ LS 4420 | Honey Extract | 1.00 | Active, moisturizing |
| | ARLYPON® TT | PEG/PPG-120/10 Trimethylolpropane Trioleate (and) Laureth-2 | 0.50 | Thickener |
| | DEHYTON® PK 45 | Cocamidopropyl Betaine | 3.80 | Surfactant |
| IV. | Sodium Chloride | Sodium Chloride | 0.10 | Agent, thickening |

Body wash for sensitive skin according to table 26, where the perfume oil composition 1B is replaced by the same amount of the perfume oil composition 2B.
Body wash for sensitive skin according to table 26, where the perfume oil composition 1B is replaced by the same amount of the perfume oil composition 3.
Body wash for sensitive skin according to table 26, where the perfume oil composition 1B is replaced by the same amount of the perfume oil composition 4.
Body wash for sensitive skin according to table 26, where the perfume oil composition 1B is replaced by the same amount of the perfume oil composition 5.
Body wash for sensitive skin according to table 26, where the perfume oil composition 1B is replaced by the same amount of the perfume oil composition 6.
Body wash for sensitive skin according to table 26, where the perfume oil composition 1B is replaced by the same amount of the perfume oil composition 7.
Body wash for sensitive skin according to table 26, where the perfume oil composition 1B is replaced by the same amount of the perfume oil composition 8.
Body wash for sensitive skin according to table 26, where the perfume oil composition 1B is replaced by the same amount of the perfume oil composition 9.
Body wash for sensitive skin according to table 26, where the perfume oil composition 1B is replaced by the same amount of the perfume oil composition 10.
Body wash for sensitive skin according to table 26, where the perfume oil composition 1B is replaced by the same amount of the perfume oil composition 11.
Body wash for sensitive skin according to table 26, where the perfume oil composition 1B is replaced by the same amount of the perfume oil composition 12.
Body wash for sensitive skin according to table 26, where the perfume oil composition 1B is replaced by the same amount of the perfume oil composition 13.
Body wash for sensitive skin according to table 26, where the perfume oil composition 1B is replaced by the same amount of the perfume oil composition 14.
Body wash for sensitive skin according to table 26, where the perfume oil composition 1B is replaced by the same amount of the perfume oil composition 15.
Body wash for sensitive skin according to table 26, where the perfume oil composition 1B is replaced by the same amount of the perfume oil composition 16.
Body wash for sensitive skin according to table 26, where the perfume oil composition 1B is replaced by the same amount of the perfume oil composition 17.
Body wash for sensitive skin according to table 26, where the perfume oil composition 1B is replaced by the same amount of the perfume oil composition 18.

**Table 27: Gloss Enhancing Shampoo for Sensitive Scalp**

| **Ingredients** | | **INCI / Chemistry** | **%** | **Function** |
|---|---|---|---|---|
| I. | Water, demin. | Aqua | 71.60 | |
| | Luviquat Ultra Care (BASF) | Polyquaternium-44 | 1.50 | Active, hair conditioning |
| II. | Sodium Benzoate | Sodium Benzoate | 0.55 | Preservative |
| III. | TEXAPON® N 70 | Sodium Laureth Sulfate | 14.30 | Surfactant |
| | DEHYTON® PK 45 | Cocamidopropyl Betaine | 5.40 | Surfactant |
| | MELHYDRAN™ LS 4420 | Honey Extract | 1.00 | Active, moisturizing |
| | Perfume oil composition 1B | Fragrance | 0.15 | Perfume |
| IV. | DEHYDOL® LS 2 DEO N | Laureth-2 | 1.00 | Thickener |
| V. | LAMESOFT® CARE | PEG-4 Distearyl Ether (and) Sodium Laureth sulfate (and) Distearyl Ether (and) Dicaprylyl Ether | 3.00 | Active, hair conditioning |
| VI. | Sodium Chloride | Sodium Chloride | 1.50 | Agent, thickening |

Gloss enhancing shampoo for sensitive scalp according to table 27, where the perfume oil composition 1 B is replaced by the same amount of the perfume oil composition 2B. Gloss enhancing shampoo for sensitive scalp according to table 27, where the perfume oil composition 1 B is replaced by the same amount of the perfume oil composition 3. Gloss enhancing shampoo for sensitive scalp according to table 27, where the perfume oil composition 1B is replaced by the same amount of the perfume oil composition 4. Gloss enhancing shampoo for sensitive scalp according to table 27, where the perfume oil composition 1 B is replaced by the same amount of the perfume oil composition 5. Gloss enhancing shampoo for sensitive scalp according to table 27, where the perfume oil composition 1B is replaced by the same amount of the perfume oil composition 6. Gloss enhancing shampoo for sensitive scalp according to table 27, where the perfume oil composition 1 B is replaced by the same amount of the perfume oil composition 7. Gloss enhancing shampoo for sensitive scalp according to table 27, where the perfume oil composition 1B is replaced by the same amount of the perfume oil composition 8. Gloss enhancing shampoo for sensitive scalp according to table 27, where the perfume oil composition 1B is replaced by the same amount of the perfume oil composition 9. Gloss enhancing shampoo for sensitive scalp according to table 27, where the perfume oil composition 1B is replaced by the same amount of the perfume oil composition 10. Gloss enhancing shampoo for sensitive scalp according to table 27, where the perfume oil composition 1B is replaced by the same amount of the perfume oil composition 11. Gloss enhancing shampoo for sensitive scalp according to table 27, where the perfume oil composition 1B is replaced by the same amount of the perfume oil composition 12. Gloss enhancing shampoo for sensitive scalp according to table 27, where the perfume oil composition 1B is replaced by the same amount of the perfume oil composition 13. Gloss enhancing shampoo for sensitive scalp according to table 27, where the perfume oil composition 1B is replaced by the same amount of the perfume oil composition 14. Gloss enhancing shampoo for sensitive scalp according to table 27, where the perfume oil composition 1B is replaced by the same amount of the perfume oil composition 15. Gloss enhancing shampoo for sensitive scalp according to table 27, where the perfume oil composition 1B is replaced by the same amount of the perfume oil composition 16. Gloss enhancing shampoo for sensitive scalp according to table 27, where the perfume oil composition 1B is replaced by the same amount of the perfume oil composition 17. Gloss enhancing shampoo for sensitive scalp according to table 27, where the perfume oil composition 1B is replaced by the same amount of the perfume oil composition 18.

**Table 28: Deo Stick**

| **Ingredients** | | **INCI / Chemistry** | **%** | **Function** |
|---|---|---|---|---|
| I. | LANETTE® 18 DEO | Stearyl Alcohol | 18.50 | Consistency factor |
| | CUTINA® HR POWDER | Hydrogenated Castor Oil | 4.50 | Consistency factor |
| | CUTINA® CP | Cetyl Palmitate | 25.00 | Emollient |
| | CETIOL® MM | Myristyl Myristate | 10.00 | Emollient |
| | CETIOL® RLF | Caprylyl Caprylate/Caprate | 5.00 | Emollient |
| | MYRITOL® 312 | Caprylic/Capric Triglyceride | 29.30 | Emollient |
| | HYDAGEN® C.A.T. | Triethyl Citrate | 5.00 | Active, antiperspirant |
| II. | COSMEDIA® SILC | Silica | 2.00 | Skin feel modifier |
| | PHYTOSOOTHE™ LS 9766 | Brassica Campestris (Rapeseed) Sterols (and) Cetearyl Alcohol | 0.50 | Active ingredient |
| III. | Perfume oil composition 1B | Fragrance | 0.20 | Perfume |

Deo stick according to table 28, where the perfume oil composition 1B is replaced by the same amount of the perfume oil composition 2B.
Deo stick according to table 28, where the perfume oil composition 1B is replaced by the same amount of the perfume oil composition 3.
Deo stick according to table 28, where the perfume oil composition 1B is replaced by the same amount of the perfume oil composition 4.
Deo stick according to table 28, where the perfume oil composition 1B is replaced by the same amount of the perfume oil composition 5.
Deo stick according to table 28, where the perfume oil composition 1B is replaced by the same amount of the perfume oil composition 6.
Deo stick according to table 28, where the perfume oil composition 1B is replaced by the same amount of the perfume oil composition 7.
Deo stick according to table 28, where the perfume oil composition 1B is replaced by the same amount of the perfume oil composition 8.
Deo stick according to table 28, where the perfume oil composition 1B is replaced by the same amount of the perfume oil composition 9.
Deo stick according to table 28, where the perfume oil composition 1B is replaced by the same amount of the perfume oil composition 10.
Deo stick according to table 28, where the perfume oil composition 1B is replaced by the same amount of the perfume oil composition 11.
Deo stick according to table 28, where the perfume oil composition 1B is replaced by the same amount of the perfume oil composition 12.
Deo stick according to table 28, where the perfume oil composition 1B is replaced by the same amount of the perfume oil composition 13.
Deo stick according to table 28, where the perfume oil composition 1B is replaced by the same amount of the perfume oil composition 14.
Deo stick according to table 28, where the perfume oil composition 1B is replaced by the same amount of the perfume oil composition 15.
Deo stick according to table 28, where the perfume oil composition 1B is replaced by the same amount of the perfume oil composition 16.
Deo stick according to table 28, where the perfume oil composition 1B is replaced by the same amount of the perfume oil composition 17.
Deo stick according to table 28, where the perfume oil composition 1B is replaced by the same amount of the perfume oil composition 18.

**Table 29: Baby Wipe**

| **Ingredients** | | **INCI / Chemistry** | **%** | **Function** |
|---|---|---|---|---|
| I. | Water, demin. | Water | 95.25 | |
| | Sodium Benzoate | Sodium Benzoate | 0.50 | Preservative |
| II. | EMULGADE® CPE | Vegetable Oil (and) Glycerin (and) Lauryl Glucoside (and) Polyglyceryl-2-Dipolyhydroxystearate (and) Glyceryl Oleate | 4.00 | Emulsion base (O/W) |
| | LIPOFRUCTYL™ ARGAN LS 9779 | Argania Spinosa Kernel Oil | 0.10 | Active ingredient |
| | Perfume oil composition 1B | Fragrance | 0.10 | Perfume |
| III. | Citric Acid (50 %) | Citric Acid | 0.15 | Agent, pH adjusting |

Baby wipe according to table 29, where the perfume oil composition 1B is replaced by the same amount of the perfume oil composition 2B.
Baby wipe according to table 29, where the perfume oil composition 1B is replaced by the same amount of the perfume oil composition 3.
Baby wipe according to table 29, where the perfume oil composition 1B is replaced by the same amount of the perfume oil composition 4.
Baby wipe according to table 29, where the perfume oil composition 1B is replaced by the same amount of the perfume oil composition 5.
Baby wipe according to table 29, where the perfume oil composition 1B is replaced by the same amount of the perfume oil composition 6.
Baby wipe according to table 29, where the perfume oil composition 1B is replaced by the same amount of the perfume oil composition 7.
Baby wipe according to table 29, where the perfume oil composition 1B is replaced by the same amount of the perfume oil composition 8.
Baby wipe according to table 29, where the perfume oil composition 1B is replaced by the same amount of the perfume oil composition 9.
Baby wipe according to table 29, where the perfume oil composition 1B is replaced by the same amount of the perfume oil composition 10.
Baby wipe according to table 29, where the perfume oil composition 1B is replaced by the same amount of the perfume oil composition 11.
Baby wipe according to table 29, where the perfume oil composition 1B is replaced by the same amount of the perfume oil composition 12.
Baby wipe according to table 29, where the perfume oil composition 1 B is replaced by the same amount of the perfume oil composition 13.
Baby wipe according to table 29, where the perfume oil composition 1 B is replaced by the same amount of the perfume oil composition 14.
Baby wipe according to table 29, where the perfume oil composition 1 B is replaced by the same amount of the perfume oil composition 15.
Baby wipe according to table 29, where the perfume oil composition 1 B is replaced by the same amount of the perfume oil composition 16.
Baby wipe according to table 29, where the perfume oil composition 1 B is replaced by the same amount of the perfume oil composition 17.
Baby wipe according to table 29, where the perfume oil composition 1 B is replaced by the same amount of the perfume oil composition 18.

**Table 30: After shave balm**

| **Ingredients** | | **INCI** | **%** | **Function** |
|---|---|---|---|---|
| I | Myritol® 331 | Cocoglycerides | 2.50 | Emollient |
| | Cetiol® Sensoft | Propylheptyl Caprylate | 5.00 | Emollient |
| | Cetiol® C5 | Coco-Caprylate | 2.50 | Emollient |
| | Cosmedia® Triple C | Polyquaternium-37 (and) Dicaprylyl Carbonate (and) Lauryl Glucoside | 1.50 | Cationic Polymer |
| II | Deionized Water | Aqua | 84.80 | |
| | Glycerine | Glycerin | 1.00 | Humectant |
| III | Anasensyl™ LS 9322 | Mannitol (and) Ammonium Glycyrrhizate (and) Caffeine (and) Zinc Gluconate (and) Aesculus Hippocastanum Extract | 1.00 | Soothing Active |
| IV | Perfume oil composition 1B | Fragrance | 0.10 | Perfume |
| V | Euxyl PE 9010 (Schülke) | Phenoxyethanol (and) Ethylhexylglycerin Ethylhexylglycerin | 1.00 | Preservative |
| | Sensiva SC 50 (Schülke) | | 0.50 | Preservative |
| VI | NaOH (Aq. Sol. 25%) | Sodium Hydroxide | 0.10 | Neutralizing Agent |

After shave balm according to table 30, where the perfume oil composition 1B is replaced by the same amount of the perfume oil composition 2B.
After shave balm according to table 30, where the perfume oil composition 1B is replaced by the same amount of the perfume oil composition 3.
After shave balm according to table 30, where the perfume oil composition 1B is replaced by the same amount of the perfume oil composition 4.
After shave balm according to table 30, where the perfume oil composition 1B is replaced by the same amount of the perfume oil composition 5.
After shave balm according to table 30, where the perfume oil composition 1B is replaced by the same amount of the perfume oil composition 6.
After shave balm according to table 30, where the perfume oil composition 1B is replaced by the same amount of the perfume oil composition 7.
After shave balm according to table 30, where the perfume oil composition 1B is replaced by the same amount of the perfume oil composition 8.
After shave balm according to table 30, where the perfume oil composition 1B is replaced by the same amount of the perfume oil composition 9.
After shave balm according to table 30, where the perfume oil composition 1B is replaced by the same amount of the perfume oil composition 10.
After shave balm according to table 30, where the perfume oil composition 1B is replaced by the same amount of the perfume oil composition 11.
After shave balm according to table 30, where the perfume oil composition 1B is replaced by the same amount of the perfume oil composition 12.
After shave balm according to table 30, where the perfume oil composition 1B is replaced by the same amount of the perfume oil composition 13.
After shave balm according to table 30, where the perfume oil composition 1B is replaced by the same amount of the perfume oil composition 14.
After shave balm according to table 30, where the perfume oil composition 1B is replaced by the same amount of the perfume oil composition 15.
After shave balm according to table 30, where the perfume oil composition 1B is replaced by the same amount of the perfume oil composition 16.
After shave balm according to table 30, where the perfume oil composition 1B is replaced by the same amount of the perfume oil composition 17.
After shave balm according to table 30, where the perfume oil composition 1B is replaced by the same amount of the perfume oil composition 18.

**Table 31: Face Gel**

| **Ingredients** | | **INCI** | **%** | **Function** |
|---|---|---|---|---|
| I | Deionized Water | Aqua | 71.65 | |
| | Glycerine | Glycerin | 3.00 | Humectant |
| | EDETA B Powder (BASF) | Tetrasodium EDTA | 0.05 | Complexing Agent |
| II | Cosmedia® SP | Sodium Polyacrylate | 1.20 | Emulsifying Polymer |
| III | Cegesoft® PFO | Passiflora Incarnata Oil | 2.00 | Emollient |
| | Uvinul MC 80 (BASF) | Ethylhexyl Methoxycinnamate | 2.00 | UVB Filter |
| | KF 96, 100 cs (Shin Etsu) | Dimethicone | 2.00 | Emollient |
| IV | Cetiol® CC | Dicaprylyl Carbonate | 5.00 | Emollient |
| | Cetiol® C5 | Coco-Caprylate | 4.00 | Emollient |
| | Uvinul A Plus Granular (BASF) | Diethylamino Hydroxybenzoyl Hexyl Benzoate | 0.50 | UVA Filter |
| | Cegesoft® SBE | Butyrospermum Parkii | 1.50 | Emollient |
| | Eumulgin® SG | Sodium Stearoyl Glutamate | 0.10 | O/W Emulsifier |
| V | Deionized Water | Aqua | 5.00 | |
| | Skinasensyl™ PW LS 9852 | Mannitol (and) Sodium Citrate (and) Acetyl Tetrapeptide-15 | 0.30 | Soothing Active |
| VI | Covi-ox® T70C | Tocopherol | 0.10 | Antioxidant |
| | Perfume oil composition 1B | Fragrance | 0.10 | Perfume |
| VII | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face gel according to table 31, where the perfume oil composition 1B is replaced by the same amount of the perfume oil composition 2B.
Face gel according to table 31, where the perfume oil composition 1B is replaced by the same amount of the perfume oil composition 3.
Face gel according to table 31, where the perfume oil composition 1B is replaced by the same amount of the perfume oil composition 4.
Face gel according to table 31, where the perfume oil composition 1B is replaced by the same amount of the perfume oil composition 5.
Face gel according to table 31, where the perfume oil composition 1B is replaced by the same amount of the perfume oil composition 6.
Face gel according to table 31, where the perfume oil composition 1B is replaced by the same amount of the perfume oil composition 7.
Face gel according to table 31, where the perfume oil composition 1B is replaced by the same amount of the perfume oil composition 8.
Face gel according to table 31, where the perfume oil composition 1B is replaced by the same amount of the perfume oil composition 9.
Face gel according to table 31, where the perfume oil composition 1B is replaced by the same amount of the perfume oil composition 10.
Face gel according to table 31, where the perfume oil composition 1B is replaced by the same amount of the perfume oil composition 11.
Face gel according to table 31, where the perfume oil composition 1B is replaced by the same amount of the perfume oil composition 12.
Face gel according to table 31, where the perfume oil composition 1B is replaced by the same amount of the perfume oil composition 13.
Face gel according to table 31, where the perfume oil composition 1B is replaced by the same amount of the perfume oil composition 14.
Face gel according to table 31, where the perfume oil composition 1B is replaced by the same amount of the perfume oil composition 15.
Face gel according to table 31, where the perfume oil composition 1B is replaced by the same amount of the perfume oil composition 16.
Face gel according to table 31, where the perfume oil composition 1B is replaced by the same amount of the perfume oil composition 17.
Face gel according to table 31, where the perfume oil composition 1B is replaced by the same amount of the perfume oil composition 18.

**Table 32: Face Day Care Cream**

| **Ingredients** | | **INCI** | **%** | **Function** |
|---|---|---|---|---|
| I | Deionized Water | Aqua | 69.55 | |
| | Glycerine | Glycerin | 3.00 | Humectant |
| | EDETA B Powder (BASF) | Tetrasodium EDTA | 0.05 | Complexing Agent |
| II | Cosmedia® SP | Sodium Polyacrylate | 0.80 | Emulsifying Polymer |
| III | Emulgade® PL 68/50 | Cetearyl Glucoside (and) Cetearyl Alcohol | 2.00 | Self-Emulsifying Base |
| | Eumulgin® SG | Sodium Stearoyl Glutamate | 0.50 | O/W Emulsifier |
| | Monomuls® 90-0-18 | Glyceryl Oleate | 2.00 | W/O Emulsifier |
| | Cutina® GMS-V | Glyceryl Stearate | 2.00 | Consistency Giving Factor |
| | Cetiol® Sensoft | Propylheptyl Caprylate | 5.00 | Emollient |
| | Myritol® 331 | Cocoglycerides | 3.00 | Emollient |
| | Cegesoft® PFO | Passiflora Incarnata Oil | 3.00 | Emollient |
| | Lipodermol™ LS 8656 | Octyldodecanol (and) Lecithin (and) Arachidyl Propionate (and) Tocopheryl Acetate (and) Retinyl Palmitate (and) Ethyl Linoleate (and) Ethyl Linolenate (and) Ethyl Oleate | 1.00 | Anti-Ageing Active |
| | General® R | Brassica Campestris (Rapeseed) Sterols | 1.00 | Anti-inflammatory Active |
| IV | Sphingoceryl™ WS LS 9859 | Aqua (and) Glycerin (and) Helianthus Annuus Seed Extract (and) Decyl Glucoside | 1.00 | Moisturizing Active |
| | Deionized Water | Aqua | 4.00 | |
| | Perfume oil composition 1B | Fragrance | 0.10 | Perfume |
| V | Covi-ox® T70C | Tocopherol | 0.50 | Antioxidant |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face day care cream according to table 32, where the perfume oil composition 1B is replaced by the same amount of the perfume oil composition 2B.
Face day care cream according to table 32, where the perfume oil composition 1B is replaced by the same amount of the perfume oil composition 3.
Face day care cream according to table 32, where the perfume oil composition 1B is replaced by the same amount of the perfume oil composition 4.
Face day care cream according to table 32, where the perfume oil composition 1B is replaced by the same amount of the perfume oil composition 5.
Face day care cream according to table 32, where the perfume oil composition 1B is replaced by the same amount of the perfume oil composition 6.
Face day care cream according to table 32, where the perfume oil composition 1B is replaced by the same amount of the perfume oil composition 7.
Face day care cream according to table 32, where the perfume oil composition 1B is replaced by the same amount of the perfume oil composition 8.
Face day care cream according to table 32, where the perfume oil composition 1B is replaced by the same amount of the perfume oil composition 9.
Face day care cream according to table 32, where the perfume oil composition 1B is replaced by the same amount of the perfume oil composition 10.
Face day care cream according to table 32, where the perfume oil composition 1B is replaced by the same amount of the perfume oil composition 11.
Face day care cream according to table 32, where the perfume oil composition 1B is replaced by the same amount of the perfume oil composition 12.
Face day care cream according to table 32, where the perfume oil composition 1B is replaced by the same amount of the perfume oil composition 13.
Face day care cream according to table 32, where the perfume oil composition 1B is replaced by the same amount of the perfume oil composition 14.
Face day care cream according to table 32, where the perfume oil composition 1B is replaced by the same amount of the perfume oil composition 15.
Face day care cream according to table 32, where the perfume oil composition 1B is replaced by the same amount of the perfume oil composition 16.
Face day care cream according to table 32, where the perfume oil composition 1B is replaced by the same amount of the perfume oil composition 17.
Face day care cream according to table 32, where the perfume oil composition 1B is replaced by the same amount of the perfume oil composition 18.

**Table 33: Face Cleanser**

| **Ingredients** | | **INCI** | **%** | **Function** |
|---|---|---|---|---|
| I | Deionized Water | Aqua | 80.15 | |
| | EDETA B Powder (BASF) | Tetrasodium EDTA | 0.05 | Complexing Agent |
| | Eumulgin® SG | Sodium Stearoyl Glutamate | 0.30 | O/W Emulsifier |
| II | Cosmedia® SP | Sodium Polyacrylate | 1.20 | Emulsifier |
| III | Isopropylpalmitate | Isopropyl palmitate | 5.00 | Emollient |
| | Cetiol® CC | Dicaprylyl Carbonate | 3.00 | Emollient |
| | Cetiol® C5 | Coco-Caprylate | 7.00 | Emollient |
| IV | Plantacare® 818 UP | Coco Glucoside | 0.75 | Non Ionic Surfactant |
| V | Indinyl® CA LS 8998 | Water (and) Cassia Angustifolia Seed Polysaccharide | 0.50 | Smoothing and Moisturizing Active |
| | Perfume oil composition 1B | Fragrance | 0.10 | Perfume |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| VII | NaOH (Aq. Sol. 25%) | Sodium Hydroxide | 0.45 | Neutralizing Agent |

Face cleanser according to table 33, where the perfume oil composition 1B is replaced by the same amount of the perfume oil composition 2B.
Face cleanser according to table 33, where the perfume oil composition 1B is replaced by the same amount of the perfume oil composition 3.
Face cleanser according to table 33, where the perfume oil composition 1B is replaced by the same amount of the perfume oil composition 4.
Face cleanser according to table 33, where the perfume oil composition 1B is replaced by the same amount of the perfume oil composition 5.
Face cleanser according to table 33, where the perfume oil composition 1B is replaced by the same amount of the perfume oil composition 6.
Face cleanser according to table 33, where the perfume oil composition 1B is replaced by the same amount of the perfume oil composition 7.
Face cleanser according to table 33, where the perfume oil composition 1B is replaced by the same amount of the perfume oil composition 8.
Face cleanser according to table 33, where the perfume oil composition 1B is replaced by the same amount of the perfume oil composition 9.
Face cleanser according to table 33, where the perfume oil composition 1B is replaced by the same amount of the perfume oil composition 10.
Face cleanser according to table 33, where the perfume oil composition 1B is replaced by the same amount of the perfume oil composition 11.
Face cleanser according to table 33, where the perfume oil composition 1B is replaced by the same amount of the perfume oil composition 12.
Face cleanser according to table 33, where the perfume oil composition 1B is replaced by the same amount of the perfume oil composition 13.
Face cleanser according to table 33, where the perfume oil composition 1B is replaced by the same amount of the perfume oil composition 14.
Face cleanser according to table 33, where the perfume oil composition 1B is replaced by the same amount of the perfume oil composition 15.
Face cleanser according to table 33, where the perfume oil composition 1B is replaced by the same amount of the perfume oil composition 16.
Face cleanser according to table 33, where the perfume oil composition 1B is replaced by the same amount of the perfume oil composition 17.
Face cleanser according to table 33, where the perfume oil composition 1B is replaced by the same amount of the perfume oil composition 18.

**Table 34: Sun Care SPF50+, Sprayable Lotion**

| **Ingredients** | | **INCI / Chemistry** | **%** | **Function** |
|---|---|---|---|---|
| I. | DEHYMULS® PGPH | Polyglyceryl-2 Dipolyhydroxystearate | 4.00 | Emulsifier (W/O) |
| | CETIOL® B | Dibutyl Adipate | 8.00 | Emollient |
| | MYRITOL® 331 | Cocoglycerides | 5.00 | Emollient |
| | Tinosorb S (BASF) | Bis-Ethylhexyloxyphenol Methoxyphenyl Triazine | 3.00 | UV filter, UV-A + UV-B |
| | Uvinul T 150 (BASF) | Ethylhexyl Triazone | 2.50 | UV filter, UV-B |
| | Uvinul A Plus (BASF) | Diethylamino Hydroxybenzoyl Hexyl Benzoate | 10.00 | UV filter, UV-A |
| | Uvinul MC 80 (BASF) | Ethylhexyl Methoxycinnamate | 10.00 | UV filter, UV-B |
| | Sensiva SC 50 (Schülke) | Ethylhexylglycerin | 0.50 | Preservative |
| II. | Water, demin. | Water | 45.55 | |
| | Glycerin | Glycerin | 3.00 | Agent, humectant |
| | EDETA BD (BASF) | Disodium EDTA | 0.05 | Chelating agent |
| | Euxyl PE 9010 (Schülke) | Phenoxyethanol, Ethylhexylglycerin | 1.00 | Preservative |
| | Keltrol CG-T (CP Kelco) | Xanthan Gum | 0.10 | Agent, thickening |
| | Veegum Ultra (RT Vanderbilt, Inc.) | Magnesium Aluminum Silicate | 2.00 | Stabilizer |
| III. | PLANTAPON® LGC SORB | Sodium Lauryl Glucose Carboxylate (and) Lauryl Glucoside | 1.50 | Surfactant |
| IV. | Eusolex T 2000 (Merck) | Titanium Dioxide and Alumina and Simethicone | 3.5 | UV filter, UV-A + UV-B |
| V. | DN-AGE™ PW LS 9827 | Cassia Alata Leaf Extract (and) Maltodextrin | 0.10 | Active ingredient |
| | Perfume oil composition 1B | Fragrance | 0.20 | Perfume |

Sun Care SPF50+, sprayable lotion according to table 34, where the perfume oil composition 1B is replaced by the same amount of the perfume oil composition 2B.
Sun Care SPF50+, sprayable lotion according to table 34, where the perfume oil composition 1B is replaced by the same amount of the perfume oil composition 3.
Sun Care SPF50+, sprayable lotion according to table 34, where the perfume oil composition 1B is replaced by the same amount of the perfume oil composition 4.
Sun Care SPF50+, sprayable lotion according to table 34, where the perfume oil composition 1B is replaced by the same amount of the perfume oil composition 5.
Sun Care SPF50+, sprayable lotion according to table 34, where the perfume oil composition 1B is replaced by the same amount of the perfume oil composition 6.
Sun Care SPF50+, sprayable lotion according to table 34, where the perfume oil composition 1B is replaced by the same amount of the perfume oil composition 7.
Sun Care SPF50+, sprayable lotion according to table 34, where the perfume oil composition 1B is replaced by the same amount of the perfume oil composition 8.
Sun Care SPF50+, sprayable lotion according to table 34, where the perfume oil composition 1B is replaced by the same amount of the perfume oil composition 9.
Sun Care SPF50+, sprayable lotion according to table 34, where the perfume oil composition 1B is replaced by the same amount of the perfume oil composition 10.
Sun Care SPF50+, sprayable lotion according to table 34, where the perfume oil composition 1B is replaced by the same amount of the perfume oil composition 11.
Sun Care SPF50+, sprayable lotion according to table 34, where the perfume oil composition 1B is replaced by the same amount of the perfume oil composition 12.
Sun Care SPF50+, sprayable lotion according to table 34, where the perfume oil composition 1B is replaced by the same amount of the perfume oil composition 13.
Sun Care SPF50+, sprayable lotion according to table 34, where the perfume oil composition 1B is replaced by the same amount of the perfume oil composition 14.
Sun Care SPF50+, sprayable lotion according to table 34, where the perfume oil composition 1B is replaced by the same amount of the perfume oil composition 15.
Sun Care SPF50+, sprayable lotion according to table 34, where the perfume oil composition 1B is replaced by the same amount of the perfume oil composition 16.
Sun Care SPF50+, sprayable lotion according to table 34, where the perfume oil composition 1B is replaced by the same amount of the perfume oil composition 17.
Sun Care SPF50+, sprayable lotion according to table 34, where the perfume oil composition 1B is replaced by the same amount of the perfume oil composition 18.

**Table 35: Hand dish cleaner, regular**

| **Hand dish cleaner, regular** | | |
|---|---|---|
| **Component** | **INCI** | **Amount %** |
| Lutensit ® A-LBN 50 | Benzenesulfonic acid, C10-13-alkyl derivs., sodium salts | 25 |
| Lutensol ® GD 70 | Alkyl polyglucoside | 3 |
| Lutensol ® A 7 N | Fatty alcohol ethoxylate | 2 |
| Perfume oil composition 1B | Fragrance | 0.7 |
| Dyes | Dye | 0.05 |
| Water, de ionized | Aqua | ad 100 |

Hand dish cleaner according to table 35, where the perfume oil composition 1B is replaced by the same amount of the perfume oil composition 2B.
Hand dish cleaner according to table 35, where the perfume oil composition 1B is replaced by the same amount of the perfume oil composition 3.
Hand dish cleaner according to table 35, where the perfume oil composition 1B is replaced by the same amount of the perfume oil composition 4.
Hand dish cleaner according to table 35, where the perfume oil composition 1B is replaced by the same amount of the perfume oil composition 5.
Hand dish cleaner according to table 35, where the perfume oil composition 1B is replaced by the same amount of the perfume oil composition 6.
Hand dish cleaner according to table 35, where the perfume oil composition 1B is replaced by the same amount of the perfume oil composition 7.
Hand dish cleaner according to table 35, where the perfume oil composition 1B is replaced by the same amount of the perfume oil composition 8.
Hand dish cleaner according to table 35, where the perfume oil composition 1B is replaced by the same amount of the perfume oil composition 9.
Hand dish cleaner according to table 35, where the perfume oil composition 1B is replaced by the same amount of the perfume oil composition 10.
Hand dish cleaner according to table 35, where the perfume oil composition 1B is replaced by the same amount of the perfume oil composition 11.
Hand dish cleaner according to table 35, where the perfume oil composition 1B is replaced by the same amount of the perfume oil composition 12.
Hand dish cleaner according to table 35, where the perfume oil composition 1B is replaced by the same amount of the perfume oil composition 13.
Hand dish cleaner according to table 35, where the perfume oil composition 1B is replaced by the same amount of the perfume oil composition 14.
Hand dish cleaner according to table 35, where the perfume oil composition 1B is replaced by the same amount of the perfume oil composition 15.
Hand dish cleaner according to table 35, where the perfume oil composition 1B is replaced by the same amount of the perfume oil composition 16.
Hand dish cleaner according to table 35, where the perfume oil composition 1B is replaced by the same amount of the perfume oil composition 17.
Hand dish cleaner according to table 35, where the perfume oil composition 1B is replaced by the same amount of the perfume oil composition 18.

**Table 36: Body lotion**

| **Ingredients** | | **INCI** | **%** | **Function** |
|---|---|---|---|---|
| I | Eumulgin® SG | Sodium Stearoyl Glutamate | 0.40 | O/W Emulsifier |
| | Cutina® GMS-V | Glyceryl Stearate | 1.50 | Consistency Giving Factor |
| | Cetiol® SB 45 | Butyrospermum Parkii (Shea) Butter | 3.00 | Emollient |
| | Cetiol® C5 | Coco-Caprylate | 2.00 | Emollient |
| | Irwinol™ LS 9890 | Octyldodecanol (and) Irvingia Gabonensis (and) Hydrogenated Coco-Glycerides | 1.00 | Moisturizing Active |
| II | Water, demin. | Aqua | 86.77 | |
| | Glycerine | Glycerin | 3.00 | Humectant |
| | EDETA B Powder (BASF) | Tetrasodium EDTA | 0.05 | Complexing Agent |
| III | Rheocare™ C Plus | Carbomer | 0.35 | Thickener |
| IV | NaOH (Aq sol. 25%) | Sodium Hydroxide | 0.10 | Neutralizing Agent |
| V | Covi-ox® T70C | Tocopherol | 0.05 | Antioxidant |
| | Perfume oil composition 1B | Fragrance | 0.10 | Perfume |
| VI | Euxyl PE 9010 (Schülke) | Phenoxyethanol (and) Ethylhexylglycerin | 1.00 | Preservative |
| | Sensiva SC50 (Schülke) | Ethylhexylglycerin | 0.50 | Preservative |
| VII | NaOH (Aq sol. 25%) | Sodium Hydroxide | 0.18 | Neutralizing Agent |

Body lotion according to table 36, where the perfume oil composition 1B is replaced by the same amount of the perfume oil composition 2B.
Body lotion according to table 36, where the perfume oil composition 1B is replaced by the same amount of the perfume oil composition 3.
Body lotion according to table 36, where the perfume oil composition 1B is replaced by the same amount of the perfume oil composition 4.
Body lotion according to table 36, where the perfume oil composition 1B is replaced by the same amount of the perfume oil composition 5.
Body lotion according to table 36, where the perfume oil composition 1B is replaced by the same amount of the perfume oil composition 6.
Body lotion according to table 36, where the perfume oil composition 1B is replaced by the same amount of the perfume oil composition 7.
Body lotion according to table 36, where the perfume oil composition 1B is replaced by the same amount of the perfume oil composition 8.
Body lotion according to table 36, where the perfume oil composition 1B is replaced by the same amount of the perfume oil composition 9.
Body lotion according to table 36, where the perfume oil composition 1B is replaced by the same amount of the perfume oil composition 10.
Body lotion according to table 36, where the perfume oil composition 1B is replaced by the same amount of the perfume oil composition 11.
Body lotion according to table 36, where the perfume oil composition 1B is replaced by the same amount of the perfume oil composition 12.
Body lotion according to table 36, where the perfume oil composition 1B is replaced by the same amount of the perfume oil composition 13.
Body lotion according to table 36, where the perfume oil composition 1B is replaced by the same amount of the perfume oil composition 14.
Body lotion according to table 36, where the perfume oil composition 1B is replaced by the same amount of the perfume oil composition 15.
Body lotion according to table 36, where the perfume oil composition 1B is replaced by the same amount of the perfume oil composition 16.
Body lotion according to table 36, where the perfume oil composition 1B is replaced by the same amount of the perfume oil composition 17.
Body lotion according to table 36, where the perfume oil composition 1B is replaced by the same amount of the perfume oil composition 18.

**Table 35: Hand dish cleaner, concentrate:**

| **Hand dish cleaner, concentrate** | | |
|---|---|---|
| **Component** | **INCI** | **amount %** |
| Lutensit ® A-LBN 50 | Benzenesulfonic acid, C10-13-alkyl derivs., sodium salts | 40 |
| Lutensit ® AS 2230 | Alkylether sulphate | 25 |
| Lutensol ® GD 70 | Alkyl polyglucoside | 7 |
| Lutensol ® A 7 N | Fatty alcohol ethoxylate | 4 |
| Perfume oil composition 1B | Perfume | 1 |
| Dyes | Dye | 0.05 |
| Water, de ionized | Aqua | ad 100 |

Hand dish cleaner according to table 37, where the perfume oil composition 1B is replaced by the same amount of the perfume oil composition 2B.
Hand dish cleaner according to table 37, where the perfume oil composition 1B is replaced by the same amount of the perfume oil composition 3.
Hand dish cleaner according to table 37, where the perfume oil composition 1B is replaced by the same amount of the perfume oil composition 4.
Hand dish cleaner according to table 37, where the perfume oil composition 1B is replaced by the same amount of the perfume oil composition 5.
Hand dish cleaner according to table 37, where the perfume oil composition 1B is replaced by the same amount of the perfume oil composition 6.
Hand dish cleaner according to table 37, where the perfume oil composition 1B is replaced by the same amount of the perfume oil composition 7.
Hand dish cleaner according to table 37, where the perfume oil composition 1B is replaced by the same amount of the perfume oil composition 8.
Hand dish cleaner according to table 37, where the perfume oil composition 1B is replaced by the same amount of the perfume oil composition 9.
Hand dish cleaner according to table 37, where the perfume oil composition 1B is replaced by the same amount of the perfume oil composition 10.
Hand dish cleaner according to table 37, where the perfume oil composition 1B is replaced by the same amount of the perfume oil composition 11.
Hand dish cleaner according to table 37, where the perfume oil composition 1B is replaced by the same amount of the perfume oil composition 12.
Hand dish cleaner according to table 37, where the perfume oil composition 1B is replaced by the same amount of the perfume oil composition 13.
Hand dish cleaner according to table 37, where the perfume oil composition 1B is replaced by the same amount of the perfume oil composition 14.
Hand dish cleaner according to table 37, where the perfume oil composition 1B is replaced by the same amount of the perfume oil composition 15.
Hand dish cleaner according to table 37, where the perfume oil composition 1B is replaced by the same amount of the perfume oil composition 16.
Hand dish cleaner according to table 37, where the perfume oil composition 1B is replaced by the same amount of the perfume oil composition 17.
Hand dish cleaner according to table 37, where the perfume oil composition 1B is replaced by the same amount of the perfume oil composition 18.

**Table 38: Sanitary cleaner, concentrate**

| **Sanitary cleaner, concentrate** | | |
|---|---|---|
| **Component** | **INCI** | **amount %** |
| Lutensol ® TO 8 | isotridecanolethoxylate | 5 |
| Phosphoric acid | Phosphoric acid | 20 |
| Korantin ® PM | 2-propyn-1-ol, ethoxylated | 3 |
| Citric acid | Citric acid | 3 |
| Perfume oil composition 1B | Fragrance | 1 |
| Water, deionized | Aqua | ad 100 |

Sanitary cleaner according to table 38, where the perfume oil composition 1B is replaced by the same amount of the perfume oil composition 2B.
Sanitary cleaner according to table 38, where the perfume oil composition 1B is replaced by the same amount of the perfume oil composition 3.
Sanitary cleaner according to table 38, where the perfume oil composition 1B is replaced by the same amount of the perfume oil composition 4.
Sanitary cleaner according to table 38, where the perfume oil composition 1B is replaced by the same amount of the perfume oil composition 5.
Sanitary cleaner according to table 38, where the perfume oil composition 1B is replaced by the same amount of the perfume oil composition 6.
Sanitary cleaner according to table 38, where the perfume oil composition 1B is replaced by the same amount of the perfume oil composition 7.
Sanitary cleaner according to table 38, where the perfume oil composition 1B is replaced by the same amount of the perfume oil composition 8.
Sanitary cleaner according to table 38, where the perfume oil composition 1B is replaced by the same amount of the perfume oil composition 9.
Sanitary cleaner according to table 38, where the perfume oil composition 1B is replaced by the same amount of the perfume oil composition 10.
Sanitary cleaner according to table 38, where the perfume oil composition 1B is replaced by the same amount of the perfume oil composition 11.
Sanitary cleaner according to table 38, where the perfume oil composition 1B is replaced by the same amount of the perfume oil composition 12.
Sanitary cleaner according to table 38, where the perfume oil composition 1B is replaced by the same amount of the perfume oil composition 13.
Sanitary cleaner according to table 38, where the perfume oil composition 1B is replaced by the same amount of the perfume oil composition 14.
Sanitary cleaner according to table 38, where the perfume oil composition 1B is replaced by the same amount of the perfume oil composition 15.
Sanitary cleaner according to table 38, where the perfume oil composition 1B is replaced by the same amount of the perfume oil composition 16.
Sanitary cleaner according to table 38, where the perfume oil composition 1B is replaced by the same amount of the perfume oil composition 17.
Sanitary cleaner according to table 38, where the perfume oil composition 1B is replaced by the same amount of the perfume oil composition 18.

**Table 39: All purpose cleaner**

| **All purpose cleaner** | | |
|---|---|---|
| **Component** | **INCI** | **amount %** |
| Lutensit ® A LBA | Benzenesulfonic acid, 4-C10-13-sec-alkyl derivs | 1 |
| Ammonia | Ammonia | 0.2 |
| Lutensol ® CS 6250 | Hexan-1-ol, ethoxylated | 3 |
| Trilon ® A | trisodium nitrilotriacetate | 1 |
| Citric acid | Citric acid | 0.65 |
| Ethanol | Ethanol | 5 |
| Perfume oil composition 1B | Perfume | 0.5 |
| Water, de ionized | Aqua | ad 100 |

All purpose cleaner according to table 39, where the perfume oil composition 1B is replaced by the same amount of the perfume oil composition 2B.
All purpose cleaner according to table 39, where the perfume oil composition 1B is replaced by the same amount of the perfume oil composition 3.
All purpose cleaner according to table 39, where the perfume oil composition 1B is replaced by the same amount of the perfume oil composition 4.
All purpose cleaner according to table 39, where the perfume oil composition 1B is replaced by the same amount of the perfume oil composition 5.
All purpose cleaner according to table 39, where the perfume oil composition 1B is replaced by the same amount of the perfume oil composition 6.
All purpose cleaner according to table 39, where the perfume oil composition 1B is replaced by the same amount of the perfume oil composition 7.
All purpose cleaner according to table 39, where the perfume oil composition 1B is replaced by the same amount of the perfume oil composition 8.
All purpose cleaner according to table 39, where the perfume oil composition 1B is replaced by the same amount of the perfume oil composition 9.
All purpose cleaner according to table 39, where the perfume oil composition 1B is replaced by the same amount of the perfume oil composition 10.
All purpose cleaner according to table 39, where the perfume oil composition 1B is replaced by the same amount of the perfume oil composition 11.
All purpose cleaner according to table 39, where the perfume oil composition 1B is replaced by the same amount of the perfume oil composition 12.
All purpose cleaner according to table 39, where the perfume oil composition 1B is replaced by the same amount of the perfume oil composition 13.
All purpose cleaner according to table 39, where the perfume oil composition 1B is replaced by the same amount of the perfume oil composition 14.
All purpose cleaner according to table 39, where the perfume oil composition 1B is replaced by the same amount of the perfume oil composition 15.
All purpose cleaner according to table 39, where the perfume oil composition 1B is replaced by the same amount of the perfume oil composition 16.
All purpose cleaner according to table 39, where the perfume oil composition 1B is replaced by the same amount of the perfume oil composition 17.
All purpose cleaner according to table 39, where the perfume oil composition 1B is replaced by the same amount of the perfume oil composition 18.

**Table 40: Anti bacterial fabric softener**

| **Anti-bacterial fabric softener** | | |
|---|---|---|
| **Component** | **INCI** | **amount %** |
| Tinosan ® HP 100 | Hydroxydichlordiphenyl ether | 0.3 |
| Dehyquat AU-46 | Esterquat | 4 |
| Lutensol ® AO 7 | Alcohols, C₁₃₋₁₅, ethoxylated | 0.5 |
| Perfume oil composition 1B | Fragrance | 0.5 |
| Water, deionized | Aqua | ad 100 |

Anti bacterial fabric softener according to table 40, where the perfume oil composition 1B is replaced by the same amount of the perfume oil composition 2B.
Anti bacterial fabric softener according to table 40, where the perfume oil composition 1B is replaced by the same amount of the perfume oil composition 3.
Anti bacterial fabric softener according to table 40, where the perfume oil composition 1B is replaced by the same amount of the perfume oil composition 4.
Anti bacterial fabric softener according to table 40, where the perfume oil composition 1B is replaced by the same amount of the perfume oil composition 5.
Anti bacterial fabric softener according to table 40, where the perfume oil composition 1B is replaced by the same amount of the perfume oil composition 6.
Anti bacterial fabric softener according to table 40, where the perfume oil composition 1B is replaced by the same amount of the perfume oil composition 7.
Anti bacterial fabric softener according to table 40, where the perfume oil composition 1B is replaced by the same amount of the perfume oil composition 8.
Anti bacterial fabric softener according to table 40, where the perfume oil composition 1B is replaced by the same amount of the perfume oil composition 9.
Anti bacterial fabric softener according to table 40, where the perfume oil composition 1B is replaced by the same amount of the perfume oil composition 10.
Anti bacterial fabric softener according to table 40, where the perfume oil composition 1B is replaced by the same amount of the perfume oil composition 11.
Anti bacterial fabric softener according to table 40, where the perfume oil composition 1B is replaced by the same amount of the perfume oil composition 12.
Anti bacterial fabric softener according to table 40, where the perfume oil composition 1B is replaced by the same amount of the perfume oil composition 13.
Anti bacterial fabric softener according to table 40, where the perfume oil composition 1B is replaced by the same amount of the perfume oil composition 14.
Anti bacterial fabric softener according to table 40, where the perfume oil composition 1B is replaced by the same amount of the perfume oil composition 15.
Anti bacterial fabric softener according to table 40, where the perfume oil composition 1B is replaced by the same amount of the perfume oil composition 16.
Anti bacterial fabric softener according to table 40, where the perfume oil composition 1B is replaced by the same amount of the perfume oil composition 17.
Anti bacterial fabric softener according to table 40, where the perfume oil composition 1B is replaced by the same amount of the perfume oil composition 18.

## Claims

1. The use of a compound of the general formula (I) 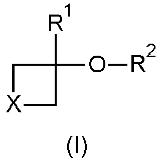 wherein
X is linear C₁-C₄-alkylene, which is unsubstituted or substituted by 1, 2, 3 or 4 radicals independently of each other selected from C₁-C₄-alkyl,
R¹ is ethyl or ethenyl, and
R² is C₁₋C₄-alkyl or C₁-C₅-alkanoyl,
or of a mixture of two or more compounds of the general formula (I),
or of a stereoisomer thereof or of a mixture of two or more stereoisomers thereof, as an aroma chemical.

2. The use according to claim 1, where X is selected from linear C₁-C₃-alkylene, which is unsubstituted or substituted by 1 or 2 radicals selected from methyl.

3. The use according to claim 1, where X is unsubstituted linear C₁-C₄-alkylene.

4. The use according to any of the preceding claims, where X is unsubstituted 1,2-ethanediyl or 1,3-propanediyl.

5. The use according to any of the preceding claims, where R¹ is ethyl.

6. The use according to any of the preceding claims, where R² is C₁-C₄-alkyl.

7. The use according to claim 6, where R² is methyl or ethyl, in particular ethyl.

8. The use according to any of claims 1 to 5, where R² is C₁-C₅-alkanoyl, preferably C₂-C₅-alkanoyl, in particular acetyl or propanoyl.

9. The use of a compound of formula (I) or a mixture of two or more compounds of formula (I) or a stereoisomer thereof or a mixture of two or more stereoisomers thereof, according to any of claims 1 to 8, for modifying the scent character of a fragranced composition.

10. The use according to any of the preceding claims, in a composition selected from the group consisting of perfume compositions, body care compositions, hygiene articles, cleaning compositions, textile detergent compositions, compositions for scent dispensers, foods, food supplements, pharmaceutical compositions and crop protection compositions.

11. Aroma chemical composition comprising
- a compound of formula (I) or a mixture of two or more compounds of the general formula (I) or a stereoisomer thereof or a mixture of two or more stereoisomers thereof, according to any of claims 1 to 8, and
- at least one further aroma chemical and/or a non-aroma chemical carrier, where the non-aroma chemical carrier is in particular selected from the group consisting of surfactants, oil components and solvents.

12. The composition according to claim 11, selected from the group consisting of perfume compositions, body care compositions, hygiene articles, cleaning compositions, textile detergent compositions, compositions for scent dispensers, foods, food supplements, pharmaceutical compositions and crop protection compositions.

13. A method of preparing a fragranced composition, comprising incorporating a compound of formula (I) or a mixture of two or more compounds of the general formula (I) or a stereoisomer thereof or a mixture of two or more stereoisomers thereof, according to any of claims 1 to 8, into said composition.

14. A compound of the general formula (I.a) or a stereoisomer thereof 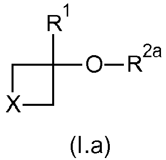 wherein
X is 1,2-ethanediyl or 1,3-propanediyl,
R¹ is ethyl or ethenyl, and
R^{2a} is C₂-C₄-alkyl, in particular ethyl,
or a compound of the general formula (I.b) or a stereoisomer thereof 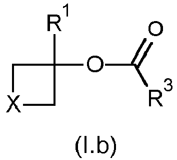 wherein
X is 1,2-ethanediyl or 1,3-propanediyl,
R¹ is ethyl or ethenyl, and
R³ is C₂-C₄-alkyl, in particular ethyl.

15. The compound according to claim 14, selected from the group consisting of 1-ethoxy-1-ethyl-cyclohexane,
1-ethoxy-1-ethyl-cyclopentane,
1-ethoxy-1-vinyl-cyclohexane and
(1-vinylcyclohexyl) propanoate.
